# EUROPEAN PATENT APPLICATION

(11) **EP 1 452 594 A1**
(43) Date of publication of application: **01.09.2004**
(21) Application number: 02781768.3
(22) Date of filing: 14.11.2002
(51) Int. Cl.: C12N 15/09, C12N 15/12, C12N 5/06, C07K 14/47, C12Q 1/02, G01N 33/50, G01N 33/15, A61K 45/00, A61K 38/00, A61P 25/00, A61P 25/28, A61P 25/16, A61P 17/00, A61P 43/00

(54) **INDUCER FOR DIFFERENTIATION OF EMBRYO STEM CELLS INTO ECTODERMAL CELLS, METHOD OF OBTAINING THE SAME AND USE THEREOF**

(30) Priority: 15.11.2001 JP 2001350724
(71) Applicant: KYOWA HAKKO KOGYO CO., LTD., Chiyoda-ku, Tokyo 100-8185 (JP); Sasai, Yoshiki, Kyoto-shi, Kyoto 606-8507 (JP); Iwata, Hiroo, Kyoto-shi, Kyoto 606-8507 (JP)
(72) Inventor: SASAI, Yoshiki, Ricken, Kobe Institute, Chuo-ku, Kobe-shi, Kyogo 650-0047 (JP); IWATA, Hiroo, Kyoto Uni., Inst. for F. M. S., Kyoto-shi, Kyoto 606-8507 (JP); MURAKAMI, Yoshinobu, Kyoto Uni., Inst. for F. M.S., Kyoto-shi, Kyoto 606-8507 (JP); SATOH, Mitsuo, Kyowa Hakko Kogyo Co., Ltd., Machida-shi, Tokyo 194-8533 (JP); KOBORI, Masato, Kyowa Hakko Kogyo Co., Ltd., Machida-shi, Tokyo 194-8533 (JP); YANO, Keiichi, Kyowa Hakko Kogyo Co., Ltd., Machida-shi, Tokyo 194-8533 (JP)
(74) Representative: VOSSIUS & PARTNER
(86) International application number: PCT/JP2002/011894
(87) International publication number: WO 2003/042384

(57) **Abstract**

A method for obtaining a solution having activity to induce differentiation of an embryonic stem cell into an ectodermal cell or ectoderm-derived cell, which comprises culturing a stromal cell in a culture comprising a polyanionic compound and recovering the culture; a solution having activity to induce differentiation of an embryonic stem cell into an ectodermal cell or ectoderm-derived cell, which is obtainable by the method; and an agent for inducing differentiation of an embryonic stem cell into an ectodermal cell or ectoderm-derived cell.

SEQUENCE LISTING

<110> KYOWA HAKKO KOGYO CO., LTD.

<110> YOSHIKI SASAI

<110> HIROO IWATA

<120> AGENT FOR INDUCING EMBRYONIC STEM CELL TO ECTODERMAL CELL, METHOD FOR
OBTAINING THE SAME, AND USE OF THE SAME

<130> K 1827 EP

<140> JP 2001-350724
<141> 2001-11-15

<160> 10

<210> 1
<211> 23
<212> DNA
<213> Artificial Sequence
<223> Description of Artificial Sequence: Synthetic DNA

<400> 1
tgaagagagc ggacaaggag atc                                                23

<210> 2
<211> 24
<212> DNA
<213> Artificial Sequence
<223> Description of Artificial Sequence: Synthetic DNA

<400> 2
tctggagtta agaaatcgga gctg                                               24

<210> 3
<211> 19
<212> DNA
<213> Artificial Sequence
<223> Description of Artificial Sequence: Synthetic DNA

<400> 3
aggacggctc tctgaagaa                                                     19

<210> 4
<211> 20
<212> DNA
<213> Artificial Sequence
<223> Description of Artificial Sequence: Synthetic DNA

<400> 4
ttgaccgagt tgaaggcgaa                                                    20

<210> 5
<211> 22
<212> DNA
<213> Artificial Sequence
<223> Description of Artificial Sequence: Synthetic DNA

<400> 5
gaccacagtc catgccatca ct                                                22

<210> 6
<211> 13
<212> DNA
<213> Artificial Sequence
<223> Description of Artificial Sequence: Synthetic DNA

<400> 6
tccaccaccc tgttgctgta g                                                 21

<210> 7
<211> 314
<212> PRT
<213> Mus musculus

<400> 7
Met Gly Val Gly Arg Ser Ala Arg Gly Arg Gly Gly Ala Ala Ser Gly
1               5                   10                  15

Val Leu Leu Ala Leu Ala Ala Ala Leu Leu Ala Ala Gly Ser Ala Ser
            20                  25                  30

Glu Tyr Asp Tyr Val Ser Phe Gln Ser Asp Ile Gly Ser Tyr Gln Ser
        35                  40                  45

Gly Arg Phe Tyr Thr Lys Pro Pro Gln Cys Val Asp Ile Pro Val Asp
    50                  55                  60

Leu Arg Leu Cys His Asn Val Gly Tyr Lys Lys Met Val Leu Pro Asn
65                  70                  75                  80

Leu Leu Glu His Glu Thr Met Ala Glu Val Lys Gln Gln Ala Ser Ser
                85                  90                  95

Trp Val Pro Leu Leu Asn Lys Asn Cys His Met Gly Thr Gln Val Phe
            100                 105                 110

Leu Cys Ser Leu Phe Ala Pro Val Cys Leu Asp Arg Pro Ile Tyr Pro
        115                 120                 125

Cys Arg Trp Leu Cys Glu Ala Val Arg Asp Ser Cys Glu Pro Val Met
    130                 135                 140

Gln Phe Phe Gly Phe Tyr Trp Pro Glu Met Leu Lys Cys Asp Lys Phe
145                 150                 155                 160

Pro Glu Gly Asp Val Cys Ile Ala Met Thr Pro Pro Asn Thr Thr Glu
                165                 170                 175

Ala Ser Lys Pro Gln Gly Thr Thr Val Cys Pro Pro Cys Asp Asn Glu
            180                 185                 190

Leu Lys Ser Glu Ala Ile Ile Glu His Leu Cys Ala Ser Glu Phe Ala
        195                 200                 205

Leu Arg Met Lys Ile Lys Glu Val Lys Lys Glu Asn Gly Asp Lys Lys
    210                 215                 220

Ile Val Pro Lys Lys Lys Lys Pro Leu Lys Leu Gly Pro Ile Lys Lys
225                 230                 235                 240

Lys Glu Leu Lys Ala Leu Val Leu Phe Leu Lys Asn Gly Ala Asp Cys
                245                 250                 255

Pro Cys His Gln Leu Asp Asn Leu Ser His Asn Phe Leu Ile Met Gly
            260                 265                 270

Arg Lys Val Lys Ser Gln Tyr Leu Leu Thr Ala Ile His Lys Trp Asp
        275                 280                 285

Lys Lys Asn Lys Glu Phe Lys Asn Phe Met Lys Arg Met Lys Asn His
    290                 295                 300

Glu Cys Pro Thr Phe Gln Ser Val Phe Lys
305                 310

<210> 8
<211> 313
<212> PRT
<213> Homo sapiens

<400> 8
Met Gly Ile Gly Arg Ser Glu Gly Gly Arg Arg Gly Ala Leu Gly Val
  1               5                  10                  15

Leu Leu Ala Leu Gly Ala Ala Leu Leu Ala Val Gly Ser Ala Ser Glu
             20                  25                  30

Tyr Asp Tyr Val Ser Phe Gln Ser Asp Ile Gly Pro Tyr Gln Ser Gly
         35                  40                  45

Arg Phe Tyr Thr Lys Pro Pro Gln Cys Val Asp Ile Pro Ala Asp Leu
     50                  55                  60

Arg Leu Cys His Asn Val Gly Tyr Lys Lys Met Val Leu Pro Asn Leu
 65                  70                  75                  80

Leu Glu His Glu Thr Met Ala Glu Val Lys Gln Gln Ala Ser Ser Trp
                 85                  90                  95

Val Pro Leu Leu Asn Lys Asn Cys His Ala Gly Thr Gln Val Phe Leu
            100                 105                 110

Cys Ser Leu Phe Ala Pro Val Cys Leu Asp Arg Pro Ile Tyr Pro Cys
        115                 120                 125

Arg Trp Leu Cys Glu Ala Val Arg Asp Ser Cys Glu Pro Val Met Gln
    130                 135                 140

Phe Phe Gly Phe Tyr Trp Pro Glu Met Leu Lys Cys Asp Lys Phe Pro
145                 150                 155                 160

Glu Gly Asp Val Cys Ile Ala Met Thr Pro Pro Asn Ala Thr Glu Ala
                165                 170                 175

Ser Lys Pro Gln Gly Thr Thr Val Cys Pro Pro Cys Asp Asn Glu Leu
            180                 185                 190

Lys Ser Glu Ala Ile Ile Glu His Leu Cys Ala Ser Glu Phe Ala Leu
        195                 200                 205

Arg Met Lys Ile Lys Glu Val Lys Lys Glu Asn Gly Asp Lys Lys Ile
    210                 215                 220

Val Pro Lys Lys Lys Lys Pro Leu Lys Leu Gly Pro Ile Lys Lys Lys
225                 230                 235                 240

Asp Leu Lys Lys Leu Val Leu Tyr Leu Lys Asn Gly Ala Asp Cys Pro
                245                 250                 255

Cys His Gln Leu Asp Asn Leu Ser His His Phe Leu Ile Met Gly Arg
            260                 265                 270

Lys Val Lys Ser Gln Tyr Leu Leu Thr Ala Ile His Lys Trp Asp Lys
        275                 280                 285

Lys Asn Lys Glu Phe Lys Asn Phe Met Lys Lys Met Lys Asn His Glu
    290                 295                 300

Cys Pro Thr Phe Gln Ser Val Phe Lys
305                 310

<210> 9
<211> 945
<212> DNA
<213> Mus musculus

<400> 9
atg ggc gtc ggg cgc agc gcg cgg ggt cgc ggc ggg gcc gcc tcg gga      48
Met Gly Val Gly Arg Ser Ala Arg Gly Arg Gly Gly Ala Ala Ser Gly
  1               5                  10                  15

gtg ctg ctg gcg ttg gcc gcc gct ctg ctg gcc tcg ggt tcg gcc agc      96
Val Leu Leu Ala Leu Ala Ala Ala Leu Leu Ala Ser Gly Ser Ala Ser
             20                  25                  30

gag tac gac tac gtg agc ttc cag tcc gac atc ggc tcg tat cag agc     144
Glu Tyr Asp Tyr Val Ser Phe Gln Ser Asp Ile Gly Ser Tyr Gln Ser
         35                  40                  45

ggg cgc ttc tac acc aag ccc ccg cag tgc gtg gac atc ccg gtg gac     192
Gly Arg Phe Tyr Thr Lys Pro Pro Gln Cys Val Asp Ile Pro Val Asp
     50                  55                  60

ctg agg ctg tgc cac aac gtg ggc tac aag aag atg gtg ctg ccc aac     240
Leu Arg Leu Cys His Asn Val Gly Tyr Lys Lys Met Val Leu Pro Asn
 65                  70                  75                  80

ctg ctg gag cac gag acc atg gca gag gtg aag cag cag gcc agc agc     288
Leu Leu Glu His Glu Thr Met Ala Glu Val Lys Gln Gln Ala Ser Ser
                 85                  90                  95

tgg gtg ccg ctg ctc aac aag aac tgc cac atg ggc acc cag gtc ttc     336
Trp Val Pro Leu Leu Asn Lys Asn Cys His Met Gly Thr Gln Val Phe
            100                 105                 110

ctc tgt tcg ctc ttc gcg ccc gtc tgt ctg gac cgg ccc atc tac ccg     384
Leu Cys Ser Leu Phe Ala Pro Val Cys Leu Asp Arg Pro Ile Tyr Pro
        115                 120                 125

tgt cgc tgg ctc tgc gag gcc gtg cgc gac tcg tgc gag ccg gtc atg     432
Cys Arg Trp Leu Cys Glu Ala Val Arg Asp Ser Cys Glu Pro Val Met
    130                 135                 140

cag ttc ttc ggc ttc tac tgg ccc gag atg ctc aaa tgt gac aag ttc     480
Gln Phe Phe Gly Phe Tyr Trp Pro Glu Met Leu Lys Cys Asp Lys Phe

145                 150                 155                 160

ccc gag ggc gac gtc tgc atc gcc atg acc ccg ccc aat acc acg gaa     528
Pro Glu Gly Asp Val Cys Ile Ala Met Thr Pro Pro Asn Thr Thr Glu
                165                 170                 175

gcc tct aag ccc caa ggt aca acc gtg tgt cct cca tgc gac aac gag     576
Ala Ser Lys Pro Gln Gly Thr Thr Val Cys Pro Pro Cys Asp Asn Glu
            180                 185                 190

ttg aag tca gag gcc atc att gaa cat ctc tgt gca agc gag ttt gca     624
Leu Lys Ser Glu Ala Ile Ile Glu His Leu Cys Ala Ser Glu Phe Ala
        195                 200                 205

ctg agg atg aaa atc aaa gaa gtg aag aag gaa aac ggt gac aag aag     672
Leu Arg Met Lys Ile Lys Glu Val Lys Lys Glu Asn Gly Asp Lys Lys
    210                 215                 220

att gtc ccc aag aag aag aaa ccc ttg aag ctg ggg ccc atc aag aag     720
Ile Val Pro Lys Lys Lys Lys Pro Leu Lys Leu Gly Pro Ile Lys Lys
225                 230                 235                 240

aag gag ctg aag cgg ctt gtg ctg ttc ctg aag aac ggt gcc gac tgt     768
Lys Glu Leu Lys Arg Leu Val Leu Phe Leu Lys Asn Gly Ala Asp Cys
                245                 250                 255

ccc tgc cac cag ctg gac aac ctc agc cac aac ttt ctc atc atg ggc     816
Pro Cys His Gln Leu Asp Asn Leu Ser His Asn Phe Leu Ile Met Gly
            260                 265                 270

cgc aag gtg aag agc cag tac ctg ctg aca gcc att cac aag tgg gac     864
Arg Lys Val Lys Ser Gln Tyr Leu Leu Thr Ala Ile His Lys Trp Asp
        275                 280                 285

aag aaa aac aag gag ttc aaa aac ttc atg aag aga atg aaa aac cac     912
Lys Lys Asn Lys Glu Phe Lys Asn Phe Met Lys Arg Met Lys Asn His
    290                 295                 300

gag tgt ccc acc ttc cag tct gtt ttt aag tga                         945
Glu Cys Pro Thr Phe Gln Ser Val Phe Lys
305                 310


<210> 10
<211> 942
<212> DNA
<213> Homo sapiens

<400> 10
atg ggc atc ggg cgc agc gag ggg ggc cgc cgc ggg gcc ctg ggc gtg     48
Met Gly Ile Gly Arg Ser Glu Gly Gly Arg Arg Gly Ala Leu Gly Val
1               5                   10                  15

ctg ctg gcg ctg ggc gcg gcg ctt ctg gcc gtg ggc tcg gcc agc gag     96
Leu Leu Ala Leu Gly Ala Ala Leu Leu Ala Val Gly Ser Ala Ser Glu
            20                  25                  30

tac gac tac gtg agc ttc cag tcg gac atc ggc ccg tac cag agc ggg     144
Tyr Asp Tyr Val Ser Phe Gln Ser Asp Ile Gly Pro Tyr Gln Ser Gly
        35                  40                  45

cgc ttc tac acc aag cca cct cag tgc gtg gac atc ccc gcg gac ctg     192
Arg Phe Tyr Thr Lys Pro Pro Gln Cys Val Asp Ile Pro Ala Asp Leu
    50                  55                  60

cgg ctg tgc cac aac gtg ggc tac aag aag atg gtg ctg ccc aac ctg 240
Arg Leu Cys His Asn Val Gly Tyr Lys Lys Met Val Leu Pro Asn Leu
65                  70                  75                  80

ctg gag cac gag acc atg gcg gag gtg aag cag cag gcc agc agc tgg 288
Leu Glu His Glu Thr Met Ala Glu Val Lys Gln Gln Ala Ser Ser Trp
                85                  90                  95

gtg ccc ctg ctc aac aag aac tgc cac gcc ggg acc cag gtc ttc ctc 336
Val Pro Leu Leu Asn Lys Asn Cys His Ala Gly Thr Gln Val Phe Leu
            100                 105                 110

tgc tcg ctc ttc gcg ccc gtc tgc ctg gac cgg ccc atc tac ccg tgt 384
Cys Ser Leu Phe Ala Pro Val Cys Leu Asp Arg Pro Ile Tyr Pro Cys
        115                 120                 125

cgc tgg ctc tgc gag gcc gtg cgc gac tcg tgc gag ccg gtc atg cag 432
Arg Trp Leu Cys Glu Ala Val Arg Asp Ser Cys Glu Pro Val Met Gln
    130                 135                 140

ttc ttc ggc ttc tac tgg ccc gag atg ctt aag tgt gac aag ttc ccg 480
Phe Phe Gly Phe Tyr Trp Pro Glu Met Leu Lys Cys Asp Lys Phe Pro
145                 150                 155                 160

gag ggg gac gtc tgc atc gcc atg acg ccg ccc aat gcc acc gaa gcc 528
Glu Gly Asp Val Cys Ile Ala Met Thr Pro Pro Asn Ala Thr Glu Ala
                165                 170                 175

tcc aag ccc caa ggc aca acg gtg tgt cct ccc tgt gac aac gag ttg 576
Ser Lys Pro Gln Gly Thr Thr Val Cys Pro Pro Cys Asp Asn Glu Leu
            180                 185                 190

aaa tct gag gcc atc att gaa cat ctc tgt gcc agc gag ttt gca ctg 624
Lys Ser Glu Ala Ile Ile Glu His Leu Cys Ala Ser Glu Phe Ala Leu
        195                 200                 205

agg atg aaa ata aaa gaa gtg aaa aaa gaa aat ggc gac aag aag att 672
Arg Met Lys Ile Lys Glu Val Lys Lys Glu Asn Gly Asp Lys Lys Ile
    210                 215                 220

gtc ccc aag aag aag aag ccc ctg aag ttg ggg ccc atc aag aag aag 720
Val Pro Lys Lys Lys Lys Pro Leu Lys Leu Gly Pro Ile Lys Lys Lys
225                 230                 235                 240

gac ctg aag aag ctt gtg ctg tac ctg aag aat ggg gct gac tgt ccc 768
Asp Leu Lys Lys Leu Val Leu Tyr Leu Lys Asn Gly Ala Asp Cys Pro
                245                 250                 255

tgc cac cag ctg gac aac ctc agc cac cac ttc ctc atc atg ggc cgc 816
Cys His Gln Leu Asp Asn Leu Ser His His Phe Leu Ile Met Gly Arg
            260                 265                 270

aag gtg aag agc cag tac ttg ctg acg gcc atc cac aag tgg gac aag 864
Lys Val Lys Ser Gln Tyr Leu Leu Thr Ala Ile His Lys Trp Asp Lys
        275                 280                 285

aaa aac aag gag ttc aaa aac ttc atg aag aaa atg aaa aac cat gag 912
Lys Asn Lys Glu Phe Lys Asn Phe Met Lys Lys Met Lys Asn His Glu
    290                 295                 300

tgc ccc acc ttt cag tcc gtg ttt aag tga 942
Cys Pro Thr Phe Gln Ser Val Phe Lys
305                 310

## Description

### TECHNICAL FIELD

The present invention relates to a method for obtaining a factor which induces differentiation of an embryonic stem cell into a functional cell. More particularly, the present invention relates to an agent or factor which induces differentiation of an embryonic stem cell into an ectodermal cell or ectoderm-derived cell useful for cell medical treatment, to a method for obtaining it, and to use thereof. Furthermore, the present invention relates to a cell differentiated from an embryonic stem cell by using the factor and to use thereof.

### BACKGROUND ART

In general, an embryonic stem cell means a cell which can be cultured *in vitro* and can also differentiate into all cells including germ cells when injected into the vacuole of an embryo before implantation, such as blastocyst, of other individual, and is called an embryonic stem cell or an ES cell.

Relationship between the generation of the initial stage embryo and the embryonic stem cell is described below by using mouse as an example.

While moving from the oviduct to the uterus, a mouse fertilized egg repeats its division into 2 cells, 4 cells and 8 cells, generates compaction in which adhesion among cells is increased when it becomes the 16-cell stage, and reaches the stage called morula where borders among cells become unclear. In addition, 3.5 days after fertilization, a space called blastcoel is formed inside the embryo and becomes blastocyst. The blastocyst of this stage comprises the outer trophectogerm layer and inner cell mass (ICM). The blastocyst is implanted onto the uterus wall spending 4.5 to 5.5 days after fertilization. At the stage of implantation, surface cells facing the blastcoel in the inner cell mass are differentiated into primitive endoderm cells. A part of these cells separates from the embryo itself, migrates into inside of the trophectoderm layer and becomes parietal endoderm cells to form Reichert's membrane by secreting an extracellular matrix.

On the other hand, the primitive endodermal cells around the embryonic part form a cell layer called visceral endoderm. These parietal and visceral endoderms then become a supporting tissue for protecting the fetus itself and exchanging nourishment and waste matter between it and the mother body. Cells of the inner cell mass, which form the fetus body in the future, proliferate and form a cell layer called primitive ectoderm. The primitive ectoderm is also called embryonic ectoderm or epiblast. Since the embryo after implantation grows into a cylindrical form as a whole, the embryo after 5.5 to 7.5 days of implantation is called egg cylinder. In half of the base side of the egg cylinder to the uterus, an extraembryonic tissue which forms the placenta in the future is formed by differentiating from the trophectoderm. After 6.5 days of fertilization, a groove called primitive streak appears on the primitive ectoderm layer, and, in this part, the primitive ectoderm enters into a space between the primitive ectoderm layer and the visceral endoderm layer by changing to a mesenchymal cell-like form and migrates from the primitive streak toward all directions to form embryonic mesoderm. In this cell layer, cells which become the definitive endoderm of the fetus body in the future are also contained.

Thus, it is known that 3 germ layers of not only ectoderm but also mesoderm and endoderm of the fetus are produced from the primitive ectoderm, and that all tissues of the fetus are derived from the primitive ectoderm. Also, it has been found that cells of the nervous system and the epidermal system are formed from ectoderms, and the ectoderm destined to differentiate into nervous system cells is called neuroectoderm (neural ectoderm), and the ectoderm destined to differentiate into epidermal system cells is called non-neuroectoderm.

Among the cell lineage in the embryo generation process described above, individual blastomere staring from fertilized egg to morula, cells of the inner cell mass in the blastocyst and cells constituting the primitive ectoderm layer have a totipotency and have properties as undifferentiated embryonic stem cells. When a primitive ectoderm starts its differentiation into each germ layer, most of its cells lose the totipotency, but a part of them is left as a primordial germ cell which takes part in transmitting genes to the next generation. When the primitive ectoderm is differentiated into each germ layer, the primordial germ cell migrates in the rear together with the embryonic mesoderm layer invaginating from the primitive streak and appears in a specific region of the extraembryonic mesoderm at the base of allantois. The primordial germ cell then migrates toward the gonad primordium and forms an ovum or a spermatozoon according to the sexual differentiation of gonad.

The embryonic stem cell can be established by culturing the inner cell mass-constituting undifferentiated stem cell existing in the inside of blastocyst and frequently repeating dissociation and subculturing of the cell mass. It is known that the cell can repeat proliferation and subculture almost unlimitedly while maintaining its normal karyotype and has a pluripotency of differentiating into every type of cells just as the same as the inner cell mass.

When an embryonic stem cell is injected into the blastocyst of other individual, it is mixed with the cell of inner cell mass of the host embryo and forms a chimeric individual by contributing to the formation of embryo and fetus. In an extreme case, an individual fetus body mostly composed of the only embryonic stem cell injected can be produced. Among chimeric individuals, an individual in which the injected embryonic stem cell has contributed to the formation of a primordial germ cell which will produce an egg or a sperm in the future is called germ line chimera, and since an individual derived from the injected embryonic stem cell can be obtained by crossing the germ line chimera, it has been confirmed that the embryonic stem cell has a totipotency of differentiating into all cells (*Manipulating the Mouse Embryo, A Laboratory Manual,* Second Edition, Cold Spring Harbor Laboratory Press (1994) (hereinafter referred to as "*Manipulating the Mouse Embryo*, *A Laboratory Manual*"); *Gene Targeting, A Practical Approach,* IRL Press at Oxford University Press (1993) (hereinafter referred to as "*Gene Targeting*"); *Biomanual Series 8, Gene Targeting, Production of Mutation Mouse Using ES Cell,* Yodo-sha (1995) (hereinafter referred to as "*Production of Mutation Mouse Using ES Cell*")).

When the inner cell mass of blastocyst is cultured like the usual primary culture, it directly differentiates into a fibroblast-like cell in most cases. In order to culture it while maintaining undifferentiated conditions, it is necessary in general to use a primary fibroblast cell produced from the fetus or STO cell derived from an SIHM mouse as a feeder cell (*Gene Targeting, Production of Mutation Mouse Using ES Cell*). By keeping an appropriate cell density on the feeder cell and repeating dissociation and subculture of the cell mass while frequently exchanging the culture medium, it becomes possible to maintain the conditions while keeping properties of the undifferentiated stem cell *(Manipulating the Mouse Embryo, A Laboratory Manual*).
As a factor for maintaining undifferentiated conditions of an embryonic stem cell, LIF (leukemia inhibitory factor) has been identified (A.G. Smith and M.L. Hooper, *Dev. Biol*., 121, 1 (1987); A.G. *Smith et al*., *Nature*, 336, 688 (1988); P.D. Rathjen *et al*., *Genes Dev.,* 4, 2308 (1990)), and it has been reported that an embryonic stem cell having a totipotency can be isolated and cultured without using a feeder cell when LIF is added to the culture medium (J. Nichols *et al*., *Development,* 110, 1341 (1990); S. *Pease et al*., *Dev. Biol.,* 141, 344 (1990)). Also, it has been shown that the addition of a family molecule of interleukin 6 sharing a subunit gp130 of LIF receptor as the common receptor is effective, instead of adding LIF itself to the culture medium (D.P. Gearing and G. Bruce, *New Biol*., 4, 61 (1992); J.I. Conover *et al*., *Development,* 119, 559 (1993); C. Piquet-Pellorce *et al*., *Exp*. *Cell Res.,* 213, 340 (1994); D. Pennica *et al*., *J*. *Biol*. *Chem.,* 270, 10915 (1995)).

In addition, since it has been reported that an embryonic stem cell capable of contributing to the formation of a germ line cell by maintaining undifferentiated conditions of the embryonic cell was established by jointly using interleukin 6 capable of directly activating gp130 and a soluble interleukin 6 receptor (K. Yoshida *et al*., *Mech*. *Dev*., 45, 163 (1994); J. Nichols *et al*., *Exp*. *Cell Res.,* 215, 237 (1994); Japanese Published Unexamined Patent Application No. 51060/95, It has been found that intracellular signal transduction from gp130 is playing an important role in maintaining the pluripotency and undifferentiation of the embryonic stem cell. This is supported also by a fact that normal generation of initial stage embryo is observed in a deficiency mouse whose LIF gene and LIF receptor gene were destroyed by using gene targeting techniques (C.L. *Stewaet et al*., *Nature,* 359, 76 (1992); J.L. Escary *et al*., *Nature,* 363, 361 (1993); M. Li *et al*., *Nature*, 378, 724 (1995); C.B. Ware *et al*., *Development,* 121, 1283 (1995)), but fetal death occurs during a period from the fetal age of 12.5 days to birth in a mouse whose gp130 gene was destroyed (K. Yoshida *et al*., *Proc. Natl*. *Acad*. *Sci. USA,* 93, 407 (1996)).

Since the first establishment of an embryonic stem cell in mice (M.J. Evans *et al*, *Nature*, 292, 154 (1981); G.R. Martin, *Proc. Natl*. *Acad Sci. USA,* 78, 7634 (1981)), methods for establishing efficient embryonic stem cells such as methods for establishing embryonic stem cells in non-mice (U.S. Patent 5,453,357; U.S. Patent 5,670,372) have been studied, and embryonic stem cells have so far been established in rat (P.M. Iannaccone *et al*., *Dev. Biol*., 163, 288 (1994)), in domestic fowl (B. Pain *et al*., *Development,* 122, 2339 (1996); U.S. Patent 5,340,740; U.S. Patent 5,656,479)), in pig (M.B. Wheeler, *Reprod Fertil. Dev*., 6, 563 (1994); H. Shim *et al*., *Biol*. *Reprod.,* 57, 1089 (1997)), in monkey (J.A. *Thomson et al*., *Proc. Natl*. *Acad Sci. USA*, 92, 7844 (1996)) and in human (J.A. Thomson *et al*., *Science,* 283, 1145 (1998); M.J. Shamblott *et al*., *Proc*. *Natl*. *Acad Sci. USA,* 95, 13726 (1998)).

It is known that a teratoma in which various tissues are mixed is formed when an embryonic stem cell is transplanted, e.g., under the skin of an animal of the same line of the embryonic stem cell *(Manipulating the Mouse Embryo, A Laboratory Manual*).

Also, it has been reported that, in *in vitro* culturing, various cells such as endodermal cells, ectodermal cells, mesodermal cells, blood cells, endothelial cells, cartilage cells, skeletal muscle cells, smooth muscle cells, heart muscle cells, glial cells, nerve cells, epithelial cells, melanocytes and keratinocytes can be formed by inducing differentiation through the formation of a cell mass called embryoid body (hereinafter referred to as "EB") in which embryonic stem cells are once aggregated to form a pseudo-embryonic state (P.D. *Rathjen et al*., *Reprod Fertil*. *Dev.,* 10, 31 (1998)). However, in the differentiation induction by this culturing method, spontaneous differentiation is generated by the formation of cell aggregation mass and, as a result, appearance of the intended cell is observed. Accordingly, it does not result in the efficient induction of a specified cell group and appearance of a variety of tissue cells is simultaneously observed.

Various attempts have been made for methods for efficiently inducing differentiation of nervous system cells from the embryonic stem cell. It has been reported that expression of a transcription factor Pax3 and neurofilament important for the differentiation of nervous system cells is significantly increased when culturing of the stem cell after formation of EB is continued in a medium supplemented with NGF (nerve growth factor) on a glass dish coated with poly-L-lysine or laminin (G. Yamada *et al*., *Biochem*. *Biophys. Res. Commun.,* 199, 552 (1994)). Based on the information that differentiation of an EC cell which will be described later into nervous system is accelerated by retinoic acid treatment (E.M.V. Jones-Villeneuve *et al*., *J*. *Cell Biol.,* 94, 253 (1982); G. Bain *et al*., *BioEssays*, 16, 323 (1994)), its effect on embryonic stem cells has also been examined, and it has been reported that a neuron-like cell which generates action potential by developing axons appears at a high ratio of about 40%, when EB is cultured for 4 days in the presence of retinoic acid and then treated with trypsin to carry out monolayer culturing, and that expression of class III tubulin, neurofilament M subunit, GAP-43 (growth-associated protein-43) as a substrate of nerve-specific calmodulin binding kinase C, MAP-2 (microtubule-associated protein-2), γ-aminobutyric acid (hereinafter referred to as "GABA") receptor, NMDA (N-methyl-D-aspartate) receptor and synapsin is observed in this cell at a protein level, and expression of neurofilament L subunit, glutamic acid receptor, tyrosine hydroxylase, a transcription factor Brn-3, GFAP (glial fibrillary acidic protein) and a GABA synthesizing enzyme GAD (glutamic acid decarboxylase) is observed at a mRNA level (G. *Bain et al*., *Dev. Biol*., 168, 342 (1995); F.A. Michael *et al*., *J*. *Neurosci*., 16, 1056 (1996)).

Since it is known that Bm-3 is expressed in central nervous system (X. He *et al*., *Nature,* 340, 35 (1989)), and GAP-43 is expressed in nerve axon (L.I. Benowitz and A. Routtenberg, *Trends Neurosci.,* 20, 84 (1997)), MAP-2 is expressed in nerve dendrite (L.I. Binder *et al*., *Ann*. *NY Acad*. *Sci.,* 76, 145 (1986)), GFAP is expressed in glial cell (A. Bignami *et al*., *Brain Res.,* 43, 429 (1972)), GABA receptor and GAD are expressed in inhibitory nerve (Y. Chang and D.I. Gottlieb, *J. Neurosci*., 8, 2123 (1988)) and glutamic acid receptor and NMDA receptor are expressed in excitatory nerve, it is shown that signals of differentiation into various nervous system cells are simultaneously transmitted when the differentiation is induced by using retinoic acid.

Also, it has been reported that differentiating induction to nervous cells was not observed when retinoic acid was simply allowed to react directly with embryonic stem cells without mediating the interaction of cells by EB formation (H.G. *Slager et al*., *Dev*. *Gen.,* 14, 212 (1993)). It has been reported that, when 10⁻⁷ mol/l retinoic acid was allowed to react with monolayer-cultured embryonic stem cells, expression of GAP-43 was observed in about 50% of the cells 3 days thereafter, and expression of neurofilament-165 (S.H. Yen and K.L. Fields, *J*. *Cell Biol*., 88, 115 (1981)) in less than 5% of the cells 4 to 5 days thereafter, both at protein level, but most of the GAP-43 positive cells showed an endodermal cell-like form (W.G. van Inzen *et al*., *Biochim. Biophys. Acta.,* 1312, 21 (1996)). It has been reported that a part of the GAP-43 positive cells show a glial cell-like morphology and about half thereof are neurofilament-165 positive cells, but both of the GAP-43 and neurofilament-165 have lower staining degree by antibody staining than the nervous cells induced by retinoic acid treatment after EB formation (W.G. van Inzen *et al*., *Biochim. Biophys. Acta.,* 1312, 21 (1996)). Thus, it has been confirmed that the interaction among cells by EB formation is necessary for the efficient differentiation induction of nervous system cells.

In addition, it has been reported that, when action potential of the cells having glial cell-like morphology was measured according to a patch clamp method, generation of the potential by 5-HT (5-hydroxytryptamin)-, GABA-, kainic acid-, glutamic acid-, dopamine- or carbachol-stimulation was observed in about half of the examined cells, but generation of action potential by carbachol-stimulation was not observed in the neuron-like cells induced by retinoic acid treatment after EB formation, used as a control, instead, generation of action potential by noradrenaline-stimulation was observed, thus showing that the interaction among cells by EB formation is also important for the determination of the direction of differentiation of nerve cells (W.G. van Inzen *et al*., *Biochim. Biophys. Acta.,* 1312, 21 (1996)). It is known that the cell layer on the EB surface differentiates into a primitive endoderm-like form in the EB formation by cell aggregation and it is considered that the differentiation is induced by a certain interaction between the cell layer and inner undifferentiated cells, but its factor has not specifically been identified (P.D. Rathjen *et al*., *Reprod Fertil*. *Dev.,* 10, 31 (1998)).

Generally, formation ofEB from an embryonic stem cell is carried out by a method in which embryonic stem cells grown in a medium containing LIF and 10 to 20% fetal calf serum, while keeping them under undifferentiated conditions, are loosened by trypsin-EDTA treatment or the like and then cultured by using an LIF-free medium containing from 10 to 20% of fetal calf serum on a plastic dish which is not coated in order to avoid their adhesion to the culture dish. It is experimentally known that formation of EB is influenced by each lot of the serum contained in the medium and it is suggested that a certain factor in serum have an influence on the formation of EB of an embryonic stem cell, but since such a factor has not been identified yet, it is difficult to efficiently form EB by supporting differentiation and proliferation of embryonic stem cell under serum-free culture conditions.

Also, it has been found that, when EB formed in a medium supplemented with retinoic acid is cultured in a dish for tissue culture, a nestin-positive precursor cell common for neuron and glial cells firstly appears, and then cells differentiated into GABAergic nerve cells, cholinergic nerve cells, GFAP positive astrocytes and 04 positive (M. Schachner *et al*., *Dev. Biol*., 83, 328 (1981)) oligodendrocytes appear (A. Fraichard *et al*., *J*. *Cell Sci.,* 108, 3181 (1995)).

Differentiation of neuron and glial cells from nestin-positive common precursor cells in the living body has been suggested by a labeling test using retrovirus (U. Lendahl *et al*., *Cell,* 60, 585 (1990); J. Price *et al*., *Development Supplement,* 2, 23 (1991); J. *Price et al*., *Brain Pathol*., 2, 23 (1992)), and then confirmed by the isolation of a precursor cell existing in the brain of the adult body as a nervous system stem cell (S.J. Morrison *et al*., *Cell,* 88, 287 (1997); R.D.G. McKay, *Science,* 276, 66 (1997)).

However, when retinoic acid is used for the differentiation induction of an embryonic stem cell, it is used at a markedly higher concentration (10 to 500 times) than the physiologically existing concentration. Since the use of retinoic acid at a concentration higher than the physiologically existing concentration is disliked from the toxicity point of view, it is difficult to use the obtained cell in medical treatment such as transplantation. Accordingly, attempts have been made to induce an embryonic stem cell into a nervous system cell under conditions more close to the physiological conditions without using retinoic acid.

Under such circumstances, recently, a method has been developed for efficiently inducing differentiation of an embryonic stem cell into a nerve system cell without forming EB and using retinoic acid (H. Kawasaki *et al*., *Neuron,* 28, 31 (2000)). According to this method, it is possible to induce differentiation of an embryonic stem cell into an ectodermal cell and an ectoderm-derived nerve system cell or epidermis system cell, by simply co-culturing the former cell with a certain kind of stromal cell under serum-free culture. It is considered that the stromal cell acts upon embryonic cell and thereby accelerates its differentiation into the ectoderm system, and this activity is named SDIA (stromal cell-derived inducing activity). It has been reported that the SDIA activity is present in stromal cells such as mouse embryo fibroblast (hereinafter also referred to as "MEF cell"), a mouse embryo-derived NIH/3T3 cell (J.L. Jainchill *et al*., *J*. *Virol*., 4, 549 (1969)), macrophage colony-stimulating factor (hereinafter also referred to as "M-CSF"), a deficient mouse skull-derived OP9 cell (T. Nakano *et al*., *Science,* 272, 722 (1996)), and a mouse skull-derived MC3T3-G2/PA6 cell (H. Kodama *et al*., *J. Cell. Physiol.,* 112, 89 (1982)), but is not present in a female cocker spaniel kidney-derived MDCK cell (C.R. *Gaush et al*., *Proc*. *Soc. Exp*. *Biol*. *Med.,* 122, 931 (1996); D.S. Misfeldt *et al*., *Proc. Natl*. *Acad*. *Sci. USA*, 73, 1212 (1976), a rat fibroblast 3Y1 (S. Sandineyer *et al*., *Cancer Res.,* 41, 830 (1981)), an African green monkey *(Cercopithecus aetiopus*) kidney-derived COS cell (Y. Gluzman, *Cell,* 23, 175 (1981) and the like. Also, it has been shown that even when a stromal cell having SDIA activity is treated with 4% paraformaldehyde, the SDIA activity remains on the surface of cell membrane. On the other hand, the SDIA activity has not been observed in the culture supernatants of these stromal cells. In addition, it has been reported that there is no SDIA-like activity to induce differentiation of embryonic stem cell into ectoderm system cells in already known molecules concerned in differentiation, such as basic fibroblast growth factor (hereinafter also referred to as "bFGF"), fibroblast growth factor 8 (hereinafter also referred to as "FGF-8"), sonic hedgehog (hereinafter also referred to as "shh"), hepatocyte growth factor (hereinafter also referred to as "HGF"), epidermal growth factor (hereinafter also referred to as "EGF"), platelet-derived growth factor (hereinafter also referred to as "PDGF"), LIF, Wnt, interleukin 1 (hereinafter also referred to as "IL-1"), interleukin I 1 (hereinafter also referred to as "IL-11") and glial cell-line derived neurotrophic factor (hereinafter also referred to as "GDNF") (H. *Kawasaki et al*., *Neuron,* 28, 31 (2000)).

Its actual conditions including whether or not the SDIA activity is derived from a single factor have not been found, and there are no reports also on a method for efficiently recovering the SDIA activity from a stromal cell and a method for inducing differentiation of an embryonic cell in a cell-free system which does not use a stromal cell, employing the recovered SDIA activity.

The polypeptides having the amino acid sequences represented by SEQ ID NOs:7 and 8 are proteins reported as mouse- and human-derived secreted frizzled-related protein (SFRP) 1 (*Proc. Natl*. *Acad*. *Sci. USA*, 94, 2859 (1997), and *Proc. Natl*. *Acad. Sci. USA*, 94, 6770 (1997). Since the first report on SFRP by Hoang *et al*. (*J*. *Biol*. *Chem*., 271, 26131 (1996)), at least five genes and proteins of SFRPs 1 to 5 have so far been identified. These SFRPs are also called by other names as cited below (*BioEssays*, 24, 811 (2002)).
· SFRP 1: Frz A, FRP-1, SARP 2, sFRP-1
· SFRP 2: SDF-5, SARP 1, sFRP-2
· SFRP 3: Frz b-1, Frz B, Flitz, Frezzled, sFRP-3
· SFRP 4: DDC-4, sFRP-4, frp AP, frp HE, Frz B-2
· SFRP 5: SARP 3, hFRP-1b, Frz b-1b

It is known that the protein of each of the SFRPs 1 to 5 controls a network of various types of signal transduction induced by Wnt, by undergoing mutual interaction with Wnt or the receptor of Wnt via a cysteine rich domain (CRD) containing 10 cysteine residues. Wnt is a secretory glycoprotein which comprises 350 to 400 amino acids and is engaged in the development of the central nerve system, determination of body axis at the early stage of embryogenesis, formation of visceral organs, determination of the proliferation and differentiation of cells and the like. To date, at least 19 kinds or more of Wnt molecules have been identified in the human genome. Included in the receptors of Wnt is a seven times transmembrane type glycoprotein named Frizzled. To date, 10 kinds or more of the Frizzled membrane receptor have been identified. This receptor has the CRD in the N-terminus moiety positioning in the extracellular region, and this domain is considered to be important for its binding with Wnt. Expression and function of molecules such as Wnt, receptors of Wnt and SFRP at steps such as development, proliferation and differentiation have been studied by using certain biotic species such as mouse and *Xenopus*, but their binding specificity and the like have not been sufficiently found yet.

As to the SFRP, it has been reported so far that SFRP 1 and SFRP 3 play an important role in the cartilage formation (WO 01/19855 A2, and *Int*. *J*. *Dev*. *Biol*., 43, 495 (1999)), and it has been suggested that SFRP 3 relates to induction of differentiation into the endoderm, heart and nerve (US 20020128439). In this connection, homology of the human-derived SFRP 3 with human-derived SFRP 1 in terms of their amino acid sequences is about 30%. On the other hand, regarding the SFRP, the activity to induce differentiation of embryonic stem cells into ectodermal cells or ectoderm-derived cells has not been reported yet.

The following has been reported. A nestin-positive and fatty acid binding protein (which is expressed in the brain)-positive (A. *Kurtz et al*., *Development,* 120, 2637 (1994)) nerve epithelial cell-like precursor cell (neuroepithelial precursor cell) is induced, when EB formed by 4 days of suspension culturing is adhered onto a tissue culture dish by 1 day of culturing and then cultured for 5 to 7 days in an ITSFn medium comprising insulin, transferrin, selenium chloride and fibronectin (A. Rizzino and C. Growley, *Proc. Natl*. *Acad Sci. USA*, 77, 457 (1980)), and the precursor cell grows keeping as the precursor cell when cultured in an mN3 serum-free medium comprising bFGF (basic fibroblast growth factor) and laminin, but it differentiates into a central nervous system cell and a glial cell when cultured in the medium from which bFGF is removed, and synaptogenesis of excitatory nervous system and inhibitory nervous system is observed when culturing is continued in a serum-supplemented medium (S. Okabe *et al*., *Mech. Dev*., 59, 89 (1996)).

A possibility for the nervous system cell induced *in vitro* in this manner to function normally in the living body has also been examined.

It has been observed that when the mouse epithelial cell-like precursor cell induced in the ITSFn medium is transplanted into the cerebral ventricle of a rat of 16 to 18 days of fetal age, the transplanted precursor cell migrates to be incorporated by the brain tissue and differentiates into a nerve cell, an astrocyte and an oligodendrocyte, but they cannot be distinguished from the host cell morphologically (O. Brustle *et al*., *Proc*. *Natl*. *Acad*. *Sci. USA*, 94, 14809 (1997)). However, formation of teratoma tissues which are not observed in the original tissue is observed in the transplanted region, such as formation of a neural tube-like structural body actively repeating cell division and a small cluster of alkaline phosphatase positive undifferentiated cells.

Formation of such teratoma tissues has also been observed in the transplantation of a nervous system precursor cell induced from embryonic stem cell by using retinoic acid (J. Dinsmore *et al*., *Cell Transplant*., 5, 131 (1996); T. Deacon *et al*., *Exp*. *Neurol.,* 149, 28 (1998)).

Thereafter, it has been reported that repair of myelin sheath was observed without forming teratoma, when a precursor cell of a glial cell was induced from embryonic stem cells and the glial precursor cell was transplanted into the brain or spinal cord of a rat congenitally lacking myelin sheath (O. Brustle et al., *Science,* 285, 754 (1999)). In this transplantation, a further differentiated glial cell precursor cell was induced from the above-mentioned nerve epithelial cell-like precursor cell induced in an ITSFn medium after the EB formation and used in the transplantation.

That is, it is shown that the cell differentiation-induced in this manner can be used in the transplantation, because differentiation into a glial precursor cell can be induced by culturing the induced nerve epithelial cell-like precursor cell for 5 days on a dish coated with polyornithine in a medium containing insulin, transferrin, progesterone, putrescine, selenium chloride, FGF2 (fibroblast growth factor 2) and laminin, pealing the cells with Hanks' buffer which does not contain calcium and magnesium, subculturing the cells at a cell density of 1/5 in a medium containing FGF2 and EGF (epidermal growth factor) and then, when the cells reached confluent, continuing the subculture at a cell density of 1/5 in a medium comprising FGF2 and PDGF-AA (platelet-derived growth factor-AA). It has been found that the cell differentiation-induced in this manner is a glial precursor cell, because it is A2B5-positive (M.C. Raff *et al*., *Nature*, 303, 390 (1983)) and its differentiation into an astrocyte and an oligodendrocyte is observed *in vitro* when cultured in a medium which does not comprise FGF2 and EGF.

Regarding cells having functions similar to the embryonic stem cell, their relationships with the embryonic stem cell are described below.

Various embryonal carcinoma cells (EC cells) have been established from a malignant teratoma (teratocarcinoma), as cell lines having a pluripotency like the case of an embryonic stem cell (M.J. Evans, *J*. *Embryol*. *Exp*. *Morph.,* 28, 163 (1972)).

These cells are considered to be cells having the properties of an embryonic stem cell as an undifferentiated stem cell, because they express a gene to be used as a marker of an embryonic stem cell (E.G. Bernstine *et al*., *Proc*. *Natl*. *Acad Sci. USA,* 70, 3899 (1973); S.B. Diwan and L.C. Steven, *J*. *Natl*. *Cancer Inst*., 57, 937 (1976); D. Solter and B.B. Knowles, *Proc. Natl*. *Acad Sci. USA*, 75, 5565 (1978); B.A. Hosler *et al*., *Mol. Cell. Biol*., 9, 5623 (1989); S.C. Pruitt, *Development*, 120, 37 (1994)), they are capable of differentiating into various cells *in vitro* (G.R. Martin and M.J. Evans, *Cell,* 6, 467 (1975); G.R. Martin and M.J. Evans, *Proc. Natl*. *Acad Sci*. *USA*, 72, 1441 (1975); M.W. McBurney, *J*. *Cell. Physiol*., 89, 441 (1976)), teratoma is formed from various tissues by their transplantation into congenic individuals (L.J. Kleinsmith and G.B. Pierce, *Cancer Res.,* 24, 797 (1964)), they form chimeric individuals by contributing to fetus formation when injected into a blastocyst (B. Mintz and K. Illmensee, *Proc*. *Natl*. *Acad Sci. USA,* 72, 3538 (1975); V.E. Papaioannou *et al*., *Nature,* 258, 70 (1975); M.J. *Dewey et al*., *Proc. Natl. Acad Sci. USA,* 74, 5564 (1977)) and, although it is extremely rare, an example is reported on an embryonal carcinoma cell line capable of producing a germ line chimera (T.A. Stewart and B. Mintz, *Proc. Natl. Acad Sci. USA*, 78, 7634 (1981)).

Also, it was shown that a cell line of a cell analogous to an embryonic stem cell appeared when bFGF was added in culturing a primordial germ cell, and was established as an EG cell (embryonic germ cell) (Y. Matsui *et al*., *Cell,* 70, 841 (1992); J.L. Resnic *et al*., *Nature,* 359, 550 (1992)). It has been found that this EG cell is capable of contributing to the formation of a germ line chimera (C.L. Stewart *et al*., *Devel*. *Biol*., 161, 626 (1994); P.A. Labosky *et al*., *Development*, 120, 3197 (1994)) and has the properties as the undifferentiated stem cell possessed by the embryonic stem cell. Since undifferentiated stem cells and germ cells have fairly common properties, it is considered that they can be mutually converted relatively easily by changes in the controlling conditions of proliferation and differentiation.

On the other hand, with the advance in developmental engineering, possibility of preparing an embryonic stem cell of individual human has been reported. Since the creation of a sheep, Dolly, as a somatic cell nucleus-derived clone individual for the first time in an mammal by Wilmut *et al*. in 1997 (I. Wilmut *et al*., *Nature,* 385, 810 (1997)), creation of a cloned calf using the nucleus of a fetal cell (J.B. Cibelli *et al*., *Science,* 280, 1256 (1998)), a cloned calf using the nucleus of a skin, muscle, ear capsule, oviduct or proligerous cumulus cell (A. Iritani, *Protein, Nucleic Acid and Enryme*, 44, 892 (1999)), a cloned goat (A. Baguisi *et al*., *Nature Biotechnology,* 17, 456 (1999)), a cloned mouse using the nucleus of proligerous cumulus cell (T. Wakayama *et al*., *Nature,* 394, 369 (1998)), a cloned mouse using a cell of male tail (T. Wakayama *et al*., *Nature Genetics,* 22, 127 (1999)) and a cloned mouse using the nucleus of embryonic stem cell (T. Wakayama *et al*., *Proc. Natl*. *Acad Sci. USA*, 96, 14984 (1999); W.M. Rideout III et al., *Nature Genetics,* 24, 109 (2000)) has been reported, thus showing a possibility of creating cloned individuals of mammals by introducing the nucleus of a somatic cell into enucleated oocytes. Since it is possible to prepare an embryonic stem cell of individual human by combining this nucleus transplantation technique with a technique for establishing the embryonic stem cell, a possibility of applying it to organ plantation as a cell medical treatment has been pointed out (R.P. Lanza *et al*., *Nature Medicine,* 5, 975 (1999)). Also, it has been pointed out that it is possible to carry out more effective gene therapy by applying gene manipulation to an embryonic stem cell and to modify histocompatibility antigens (P.D. Rathjen *et al*., *Reprod*. *Fertil*. *Dev.,* 10, 31 (1998)).

Next, effectiveness of the cell medical treatment in organ transplantation is described with examples.

Parkinson disease is a chronic progressive disease mainly caused by the degeneration of dopaminergic neurons of substantia nigra corpus striatum. A perlingual therapy mainly using L-DOPA (L-dihydroxyphenylalanine) has conventionally been carried out, but since it is necessary to carry out its internal use for a prolonged period of time, its effect gradually attenuates in many patients who then will suffer from side effects such as wearing off phenomenon, and dyskinesia. Accordingly, development of more effective therapeutic methods has been attempted, and a treatment for transplanting an abortion fetal brain to patients of Parkinson disease has been started. In the whole world, several hundred cases of abortion fetal brain transplantation treatment have so far been carried out. Recently, a double blindfold test on the transplantation of abortion fetal brain cells was carried out in the United States for 40 patients of Parkinson disease, and its usefulness was confirmed. In addition, a case has been reported in which the transplanted cell was fixed for 10 years or more and the transplanted cell formed a synapse with corpus striatum in some patients who underwent such an abortion fetal brain cell transplantation. Thus, it has been understood that the cell treatment for transplanting the brain of abortion fetus shows high efficiency for Parkinson disease, but a protest against the use of abortion fetuses is strong due to ethical problems. In addition, since close to 10 fetuses are practically required for the treatment of one patient, it meets with a great obstacle for its realistic application to the therapy. Accordingly, concern has been directed toward the development of a method for preparing a dopaminergic neuron in a large amount by a commonly acceptable method.

From the viewpoint of cell medical treatment, development of a method for inducing differentiation of a target functional cell selectively and efficiently from an undifferentiated stem cell which can be cultured while maintaining its pluripotency has been drawing attention, and various attempts have been made thereon. However, induction of a target functional cell under an artificially controlled physiological environment, such as culture conditions which do not use serum, retinoic acid or stromal cells, is desired, but such a method is not known. Particularly, a method for obtaining an ectoderm-derived cell, specifically a dopaminergic neuron having normal functions, by efficient differentiation induction from an undifferentiated stem cell is important and desired from the viewpoint of the medical treatment of patients of brain diseases including Parkinson disease, but such a method has not been developed yet.

### DISCLOSURE OF THE INVENTION

An object of the present invention is to provide a method for efficiently recovering the activity of stromal cells to induce differentiation of embryonic stem cells into ectodermal cells or ectoderm-derived cells (hereinafter also referred to as "SDIA activity").

Another object of the present invention is to provide a method for inducing differentiation of embryonic stem cells into functional cells which can be used in cell and organ transplantation medical treatment by using the recovered SDIA activity, and to an agent for inducing differentiation used in the method, to a cell prepared by the differentiation induction, and to a method for using thereof.

The present inventors have conducted intensive studies on conditions for recovering SDIA activity from a stromal cell and found the method as a result thereof. Thus, the present invention has been completed.

The present invention relates to the following (1) to (42).
(1) A method for obtaining a solution having activity to induce differentiation of an embryonic stem cell into an ectodermal cell or ectoderm-derived cell, which comprises culturing a stromal cell in a culture comprising a polyanionic compound, and recovering the culture.
(2) The method according to the above (1), wherein the polyanionic compound is a copolymer or homopolymer which has a negative charge in the culture.
(3) The method according to the above (2), wherein the copolymer which has a negative charge in the culture is a mucopolysaccharide.
(4) The method according to the above (3), wherein the mucopolysaccharide is a compound selected from the group consisting of the following (a), (b), (c), (d), (e), (f), (g), (h), (i) and (j):
   (a) chondroitin 4-sulfate;
   (b) chondroitin 5-sulfate;
   (c) chondroitin 6-sulfate;
   (d) dermatan sulfate;
   (e) heparan sulfate;
   (f) heparin;
   (g) keratan sulfate I;
   (h) keratan sulfate II;
   (i) hyaluronic acid; and
   (j) chondroitin.
(5) The method according to the above (2), wherein the homopolymer which has a negative charge in the culture is a compound selected from the group consisting of the following (a), (b), (c), (d), (e), (f), (g), (h), (i), (j) and (k):
   (a) dextran sulfate;
   (b) carboxymethyldextran;
   (c) sulfated polyvinyl;
   (d) polyvinyl sulfite;
   (e) sulfonated polystyrene;
   (f) polyacrylic acid;
   (g) carboxymethylcellulose;
   (h) cellulose sulfate;
   (i) polyglutamic acid;
   (j) polymaleic acid; and
   (k) polymethacrylic acid.
(6) The method according to any one of the above (1) to (5), wherein the culture is a basal medium or a balanced salt solution used for cell culturing.
(7) The method according to any one of the above (I) to (6), wherein the stromal cell is recognized by a monoclonal antibody produced by a hybridoma FERM BP-7573.
(8) The method according to any one of the above (1) to (6), wherein the stromal cell is selected from the group consisting of the following (a), (b), (c), (d), (e), (f) and (g):
   (a) a fetal primary culture fibroblast;
   (b) an SIHM mouse-derived STO cell;
   (c) a mouse fetus-derived NIH/3T3 cell;
   (d) a macrophage colony-stimulating factor (M-CSF) deficient mouse calvaria-derived OP9 cell;
   (e) a mouse calvaria-derived MC3T3-G2/PA6 cell;
   (f) an embryonic stem cell-derived stromal cell; and
   (g) a bone marrow mesenchymal stem cell-derived stromal cell.
(9) A solution having activity to induce differentiation of an embryonic stem cell into an ectodermal cell or ectoderm-derived cell, which is obtainable by the method according to any one of the above (1) to (8).
(10) An agent for inducing differentiation of an embryonic stem cell into an ectodermal cell or ectoderm-derived cell, which comprises, as an active ingredient, the solution according to the above (9).
(11) A factor which induces differentiation of an embryonic stem cell into an ectodermal cell or ectoderm-derived cell contained in the solution according to the above (9).
(12) An agent for inducing differentiation of an embryonic stem cell into an ectodermal cell or ectoderm-derived cell, which comprises, as an active ingredient, a polypeptide consisting of the amino acid sequence represented by SEQ ID NO:7.
(13) An agent for inducing differentiation of an embryonic stem cell into an ectodermal cell or ectoderm-derived cell, which comprises, as an active ingredient, a polypeptide consisting of an amino acid sequence in which one or more amino acid residue(s) is/are deleted, substituted, inserted and/or added in the amino acid sequence represented by SEQ ID NO:7.
(14) An agent for inducing differentiation of an embryonic stem cell into an ectodermal cell or ectoderm-derived cell, which comprises, as an active ingredient, a polypeptide consisting of an amino acid sequence having a homology of 60% or more with the amino acid sequence represented by SEQ ID NO:7.
(15) An agent for inducing differentiation of an embryonic stem cell into an ectodermal cell or ectoderm-derived cell, which comprises, as an active ingredient, a recombinant vector comprising a DNA encoding the amino acid sequence represented by SEQ ID NO:7.
(16) An agent for inducing differentiation of an embryonic stem cell into an ectodermal cell or ectoderm-derived cell, which comprises, as an active ingredient, a recombinant vector comprising a DNA having the nucleotide sequence represented by SEQ ID NO:9.
(17) An agent for inducing differentiation of an embryonic stem cell into an ectodermal cell or ectoderm-derived cell, which comprises, as an active ingredient, a transformant obtainable by introducing a recombinant vector comprising a DNA encoding the amino acid sequence represented by SEQ ID NO:7 into a stromal cell.
(18) An agent for inducing differentiation of an embryonic stem cell into an ectodermal cell or ectoderm-derived cell, which comprises, as an active ingredient, a transformant obtained by introducing a recombinant vector comprising a DNA having the nucleotide sequence represented by SEQ ID NO:9 into a stromal cell.
(19) An agent for inducing differentiation of an embryonic stem cell into an ectodermal cell or ectoderm-derived cell, which comprises, as an active ingredient, a polypeptide consisting of the amino acid sequence represented by SEQ ID NO:8.
(20) An agent for inducing differentiation of an embryonic stem cell into an ectodermal cell or ectoderm-derived cell, which comprises, as an active ingredient, a polypeptide consisting of an amino acid sequence in which one or more amino acid residue(s) is/are deleted, substituted, inserted and/or added in the amino acid sequence represented by SEQ ID NO:8.
(21) An agent for inducing differentiation of an embryonic stem cell into an ectodermal cell or ectoderm-derived cell, which comprises, as an active ingredient, a polypeptide consisting of an amino acid sequence having a homology of 60% or more with the amino acid sequence represented by SEQ ID NO:8.
(22) An agent for inducing differentiation of an embryonic stem cell into an ectodermal cell or ectoderm-derived cell, which comprises, as an active ingredient, a recombinant vector comprising a DNA encoding the amino acid sequence represented by SEQ ID NO:8.
(23) An agent for inducing differentiation of an embryonic stem cell into an ectodermal cell or ectoderm-derived cell, which comprises, as an active ingredient, a recombinant vector comprising a DNA having the nucleotide sequence represented by SEQ ID NO:10.
(24) An agent for inducing differentiation of an embryonic stem cell into an ectodermal cell or ectoderm-derived cell, which comprises, as an active ingredient, a transformant obtained by introducing a recombinant vector comprising a DNA encoding the amino acid sequence represented by SEQ ID NO:7 into a stromal cell.
(25) An agent for inducing differentiation of an embryonic stem cell into an ectodermal cell or ectoderm-derived cell, which comprises, as an active ingredient, a transformant obtained by introducing a recombinant vector comprising a DNA having the nucleotide sequence represented by SEQ ID NO: 10 into a stromal cell.
(26) An agent for inducing differentiation of an embryonic stem cell into an ectodermal cell or ectoderm-derived cell, which comprises, as an active ingredient, an Wnt antagonist.
(27) A method for inducing differentiation of an embryonic stem cell into an ectodermal cell or ectoderm-derived cell, which comprises a step of culturing the embryonic stem cell under non-aggregation conditions by using the solution according to the above (9) or the agent for inducing differentiation according to any one of the above (12) to (26).
(28) The method according to the above (27), wherein a culture vessel immobilized with the solution according to the above (9) or the agent for inducing differentiation according to any one of the above (12) to (26) is used.
(29) The method according to any one of the above (1) to (8), (27) and (28), wherein the ectodermal cell is a cell capable of differentiating into a nervous system cell or an epidermal system cell.
(30) The method according to any one of the above (1) to (8), (27) and (28), wherein the ectoderm-derived cell is a nervous system cell or an epidermal system cell.
(31) The method according to the above (29) or (30), wherein the epidermal system cell is an epidermal cell.
(32) The method according to the above (29) or (30), wherein the nervous system cell is a cell selected from the group consisting of the following (a), (b), (c), (d) and (e):
   (a) a neural stem cell;
   (b) a nerve cell;
   (c) a cell of neural tube;
   (d) a cell of neural crest; and
   (e) a retinal pigment cell.
(33) The method according to the above (32), wherein the neural stem cell is a neural stem cell expressing nestin.
(34) The method according to the above (32), wherein the nerve cell is a nerve cell selected from the group consisting of the following (a), (b), (c) and (d):
   (a) a dopaminergic neuron;
   (b) an acetylcholinergic neuron;
   (c) a γ-aminobutyratergic neuron; and
   (d) a serotonergic neuron.
(35) The method according to the above (34), wherein the acetylcholinergic neuron is a motor nerve cell expressing islet 1.
(36) The method according to the above (32), wherein the cell of neural tube is a cell selected from the group consisting of the following (a), (b), (c) and (d):
   (a) a cell of neural tube before determination of dorso-ventral axis, which is capable of differentiating into a cell positioned at the ventral side by reacting with sonic hedgehog as a ventral factor of neural tube and of differentiating into a cell positioned at the dorsal side by reacting with bone morphogenetic protein 4 as a dorsal factor of neural tube;
   (b) a cell of the neural tube ventral side, expressing HNF-3β (hepatocyte nuclear factor-3β) positioned on the basal plate of the most ventral side of neural tube;
   (c) a cell of the neural tube ventral side, expressing a marker Nkx2.2 existing secondary to the HNF-3β (hepatocyte nuclear factor-3β) from the ventral side of neural tube; and
   (d) a cell of the neural tube dorsal side, expressing Pax-7.
(37) The method according to the above (32), wherein the cell of neural crest is a cell expressing AP-2 (activator protein 2).
(38) The method according to any one of the above (27) to (37), wherein said culturing is carried out in the presence of bone morphogenetic protein 4.
(39) The method according to any one of the above (27) to (38), wherein said culturing is carried out in the presence of sonic hedgehog.
(40) The method according to any one of the above (27) to (39), wherein the non-aggregation conditions are conditions not mediating an embryoid body.
(41) The method according to any one of the above (27) to (40), which comprises a step of culturing under serum-free culture conditions.
(42) The method according to any one of the above (27) to (41), wherein said culturing is carried out in the absence of retinoic acid.
(43) The method according to any one of the above (27) to (42), wherein the embryonic stem cell is selected from the group consisting of the following (a), (b) and (c):
   (a) an embryonic stem cell established by culturing an early embryo before implantation;
   (b) an embryonic stem cell established by culturing an early embryo produced by nuclear transplantation of the nucleus of a somatic cell; and
   (c) an embryonic stem cell in which a gene on the chromosome of the embryonic stem cell of (a) or (b) is modified by using a genetic engineering technique.
(44) The method according to any one of the above (1) to (8) and (27) to (43), wherein the embryonic stem cell is differentiated into an ectodermal cell or an ectoderm-derived cell at an efficiency of 5% or more.
(45) The method according to any one of the above (27) to (44), which does not substantially accompany differentiation induction of a mesodermal system cell.
(46) An ectodermal cell or an ectoderm-derived cell, which is induced by using the method of any one of the above (27) to (45).
(47) A method for increasing purity of a cell which is differentiation-induced from an embryonic stem cell, which comprises a step of culturing the cell according to the above (46) in a medium comprising an antitumor agent.
(48) The method according to the above (47), wherein the antitumor agent is selected from the group consisting of mitomycin C, 5-fluorouracil, adriamycin, methotrexate and ara-C.
(49) A cell which is obtainable by using the method according to the above (46) or (47).
(50) A medicament comprising the cell according to the above (46) or (49).
(51) A medicament which comprises, as an active ingredient, at least one selected from the group consisting of the following (a) to (o):
   (a) a polypeptide consisting of the amino acid sequence represented by SEQ ID NO:7;
   (b) a polypeptide consisting of an amino acid sequence in which one or more amino acid residue(s) is/are deleted, substituted, inserted and/or added in the amino acid sequence represented by SEQ ID NO:7;
   (c) a polypeptide consisting of an amino acid sequence having a homology of 60% or more with the amino acid sequence represented by SEQ ID NO:7;
   (d) a recombinant vector comprising a DNA encoding the amino acid sequence represented by SEQ ID NO:7;
   (e) a recombinant vector comprising a DNA having the nucleotide sequence represented by SEQ ID NO:9;
   (f) a transformant obtainable by introducing a recombinant vector comprising a DNA encoding the amino acid sequence represented by SEQ ID NO:7 into a stromal cell;
   (g) a transformant obtained by introducing a recombinant vector comprising a DNA having the nucleotide sequence represented by SEQ ID NO:9 into a stromal cell;
   (h) a polypeptide consisting of the amino acid sequence represented by SEQ ID NO:8;
   (i) a polypeptide consisting of an amino acid sequence in which one or more amino acid residue(s) is/are deleted, substituted, inserted and/or added in the amino acid sequence represented by SEQ ID NO:8;
   (j) a polypeptide consisting of an amino acid sequence having a homology of 60% or more with the amino acid sequence represented by SEQ ID NO:8;
   (k) a recombinant vector comprising a DNA encoding the amino acid sequence represented by SEQ ID NO:8;
   (l) a recombinant vector comprising a DNA having the nucleotide sequence represented by SEQ ID NO:10;
   (m) a transformant obtained by introducing a recombinant vector comprising a DNA encoding the amino acid sequence represented by SEQ ID NO:7 into a stromal cell;
   (n) a transformant obtained by introducing a recombinant vector comprising a DNA having the nucleotide sequence represented by SEQ ID NO:10 into a stromal cell; and
   (o) an agent for inducing differentiation of an embryonic stem cell into an ectodermal cell or ectoderm-derived cell, which comprises, as an active ingredient, an Wnt antagonist.
(52) The medicament according to the above (50) or (51), which is a medicament for diagnosing, preventing and/or treating diseases caused by the disorder of an ectoderm-derived cell.
(53) The medicament according to the above (52), wherein the diseases caused by the disorder of an ectoderm-derived cell are diseases caused by the disorder of a nervous system cell or an epidermal system cell.
(54) The medicament according to the above (53),
   wherein the diseases caused by the disorder of a nervous system cell are Alzheimer disease, Huntington chorea, Parkinson disease, ischemic cerebral disease, epilepsy, brain injury, vertebral injury, motor neuron disease, neurodegeneration disease, pigmentary retinal dystrophy, cochlear hearing loss, multiple sclerosis, amyotrophic lateral sclerosis or diseases due to a neurotoxin damage; and
   the diseases caused by the disorder of an epidermal system cell are burn, wound, healing of wound, compression gangrene or psoriasis.
(55) A method for evaluating a substance relating to the regulation in a differentiation step from an embryonic stem cell into an ectodermal cell or an ectoderm-derived cell, which comprises:
   carrying out the method according to any one of the above (27) to (45) in the presence of a substance to be tested and the method in the absence of the substance to be tested; and
   comparing the differentiation step from an embryonic stem cell into an ectodermal cell or an ectoderm-derived cell in the presence of the substance to be tested with that in the absence of the substance to be tested.
(56) A method for screening a substance relating to the regulation in a differentiation step from an embryonic stem cell into an ectodermal cell or an ectoderm-derived cell, which comprises:
   carrying out the method according to any one of the above (27) to (45) in the presence of a substance to be tested and the method in the absence of the substance to be tested; and
   comparing the differentiation step from an embryonic stem cell into an ectodermal cell or an ectoderm-derived cell in the presence of a substance to be tested with that in the absence of the substance to be tested.
(57) A method for evaluating a substance relating to the regulation of the function of an ectodermal cell or an ectoderm-derived cell, which comprises:
   culturing the cell according to the above (46) in the presence of a substance to be tested and the cell in the absence of the substance to be tested; and
   comparing the function of an ectodermal cell or an ectoderm-derived cell in the presence of the substance to be tested with that in the absence of the substance to be tested.
(58) A method for screening a substance relating to the regulation of the function of an ectodermal cell or an ectoderm-derived cell, which comprises:
   culturing the cell according to the above (46) in the presence of a substance to be tested and that in the absence of the substance to be tested; and
   comparing the function of the ectodermal cell or the ectoderm-derived cell in the presence of the substance to be tested with that in the absence of the substance to be tested.

The embodiments and methods for carrying out the present invention are described below in detail.

### 1. Method for inducing differentiation of the present invention

### (1) Animals to be applied

Examples of the animals used in the present invention include vertebral animals, particularly warm-blooded animals, and more particularly mammals such as mouse, rat, guinea pig, hamster, rabbit, cat, dog, sheep, pig, cattle, goat, monkey, and human.

### (2) Embryonic stem cell

The embryonic stem cell include those cells which can be *cultured in vitro* and have a pluripotency capable of differentiating into all cells constituting the living body. Examples include (a) an embryonic stem cell of, e.g., a mammal established by culturing an early embryo before implantation, and specific examples include an ES cell established from an early embryo-constituting inner cell mass, an EG cell established from a primordial germ cell, a cell isolated from a cell group (e.g., primitive ectoderm) having a pluripotency of an early embryo before implantation and a cell obtained by culturing such a cell. Since it is known that an EC cell established from a malignant teratoma shows properties similar to those of the ES cell, it is included in the embryonic stem cell of, e.g., a mammal established by culturing an early embryo before implantation in a broad sense.

The embryonic stem cell according to the present invention includes the embryonic stem cell of the above (a), (b) an embryonic stem cell established by culturing an early embryo produced by nuclear transplantation of the nucleus of a somatic cell and (c) an embryonic stem cell in which a gene on the chromosome of the embryonic stem cell of (a) or (b) is modified by using a genetic engineering technique.

### (3) Ectodermal cell and ectoderm-derived cell

The embryonic stem cell described above can be differentiation-induced into an ectodermal cell or an ectoderm-derived cell by culturing the embryonic stem cell under non-aggregation conditions using the method for inducing differentiation of the present invention.

According to the present invention, the ectodermal cell include germ layer cells comprising a cell capable of differentiating into a nervous system cell or an epidermal system cell. Examples include a fetal ectodermal cell differentiated from a primitive ectoderm.

According to the present invention, the ectoderm-derived cell includes functional cells which are differentiated from an ectodermal cell and constitute the living body. Specific examples include nervous system cells and epidermal system cells. That is, an ectodermal cell can be induced into a nervous system cell or an epidermal system cell by the method of the present invention.

### (a) Nervous system cell

Examples of the nervous system cell include a neural stem cell, a nerve cell, a cell of neural tube, a cell of neural crest and the like.

### (i) Nerve cell

The nerve cell means a cell which functions to receive a stimulus from other nerve cells or stimulus receptor cells and transmit the stimulus to other nerve cells, muscle or glandular cells.

The nerve cell is classified based on the difference in the neurotransmitter produced by the nerve cell, specifically, based on, e.g., each neurotransmitter and synthase of the neurotransmitter. The neurotransmitter includes both of peptide and non-peptide substances. The non-peptide neurotransmitter includes dopamine, noradrenaline, adrenaline, serotonin, acetylcholine, γ-aminobutyric acid and glutamic acid. Dopamine, noradrenaline and adrenaline are called catecholamine.

Examples of nerve cells classified by these neurotransmitters include dopaminergic neurons, acetylcholinergic neurons, γ-aminobutyratergic neurons, serotonergic neurons, noradrenalinergic neurons, adrenalinergic neurons, glutamatergic neurons and the like. The dopaminergic neurons, noradrenalinergic neurons and adrenalinergic neurons are generally referred to as catecholaminergic neurons.

The catecholaminergic neurons express tyrosine hydroxylase in common, and the noradrenalinergic neurons and the adrenalinergic neurons express dopamine-β-hydroxylase in common. Also, phenylethanolamine N-methyltransferase is specifically expressed in the noradrenalinergic neurons, tryptophan hydroxylase is specifically expressed in the serotonergic neurons, choline acetyltransferase is specifically expressed in the acetylcholinergic neurons and glutamate decarboxylase is specifically expressed in the γ-aminobutyratergic neurons. Accordingly, a method for recognizing a nerve cell includes an identification method using antibodies which recognizes the above enzymes, a method for detecting expression of mRNA coding for the above enzyme, and the like.

Examples of the peptide neurotransmitter include adrenocorticotropic hormone (corticotropin (ACTH)), ααγ,β-lipotropin, α-melanin cell stimulating hormone (MSH), α-endorphin, β-endorphin, γ-endorphin, methionine enkephalin (Met-enkephalin), leucine enkephalin (Leu-enkephalin), α-neoendorphin, β-neoendorphin, dynorphin A, dynorphin B, leumorphin, vasopressin, neurophysin, oxytocin, neurophysin I, substance P, neurokinin A, neuropeptide K, neuropeptide-γ, neurokinin B, bombesin, gastrin-releasing peptide, secretin, motilin, glucagon, vasoactive intestinal peptide, growth hormone-releasing factor, insulin, insulin-like growth factors, somatostatin, gastrin, cholecystokinin, neuropeptide Y, pancreatic polypeptide, peptide YY, corticotropin-releasing factor, calcitonin, calcitonin gene-related peptide, angiotensin, bradykinin, thyrotropin-releasing hormone, neurotensin, galanin and luteinizing hormone-releasing hormone. Nerve cells capable of producing these peptide neurotransmitters can be identified by staining using an antibody which recognizes a neurotransmitte or a neurotransmitter precursor peptide, or by detecting expression of mRNA coding for the neurotransmitter or neurotransmitter precursor peptide.

Also, the motor neuron transmits information to skeletal muscle by secreting acetylcholine from its nerve ending and is classified into the acetylcholinergic neurons. Examples of a marker protein of the motor nerve cell include islet 1 (O. Karlsson *et al*., *Nature,* 344, 879 (1990)).

The method for inducing differentiation of the present invention is suitably used for inducing differentiation into nerve cells, preferably dopaminergic neurons, acetylcholinergic neurons, γ-aminobutyratergic neurons and serotonergic neurons.

Particularly, the dopaminergic neuron induced from an embryonic stem cell by the method of the present invention is characterized as a cell which expresses tyrosine hydroxylase whose expression is observed in the catecholaminergic neurons in common but which does not express dopamine-β-hydroxylase whose expression is observed in the noradrenalinergic neurons and adrenalinergic neurons in common, as described above, and is capable of improving symptoms of nerve degeneration diseases such as Parkinson disease by its transplantation.

### (ii) Neural stem cell

The neural stem cell is defined as a cell which is capable of differentiating into neuron, astrocyte and oligodendrocyte and also has the self-replicating ability. The neural stem cell does not have the pluripotency of embryonic stem cell to differentiate into all cells but functions to supply a nerve cell, an astrocyte and an oligodendrocyte in the brain.

Accordingly, examples of a method for confirming that the cell is the neural stem cell include a method in which the cell is practically transplanted into the brain and its differentiation ability is confirmed and a method in which differentiation induction of the neural stem cell into a nerve cell, an astrocyte and an oligodendrocyte is confirmed *in vitro* (*Mol*. *Cell. Neuro Science,* 8, 389 (1997); *Science,* 283, 534 (1999)).

Also, the neural stem cell having such a function can be stained with an anti-nestin antibody which recognizes a cytoskeletal protein nestin whose expression in a nerve precursor cell has been confirmed (R. Mckay, *Science,* 276, 66 (1997)). Accordingly, the neural stem cell can also be confirmed by staining it with the anti-nestin antibody.

### (iii) Cells of neural tube and neural crest

In the initial stage development in chordates, a primitive streak appears in the primitive ectoderm layer and neural induction starts. The neural induction means a stage in which an ectoderm positioned on the dorsal side of an early embryo receives a signal from an organizer region positioned in its adjacent or inner part and thereby differentiates into a neuroectoderm. The neuroectoderm formed by this neural induction becomes a neural plate independently from a non-neuroectoderm, namely an epidermal ectoderm, and then forms a neural tube by invaginating into the ventral side. The ectoderm portion positioned between the neural plate and epidermal ectoderm forms a neural crest during the invagination. All cell groups of the central nervous system are generated from one layer of the neuroepithelial tissues which constitute the neural tube. That is, the front part of the neural tube expands and forms a brain vesicle which becomes primordium of the brain, and the rear part differentiates into the spinal cord as the tube. The neural crest does not directly take part in the differentiation of central nerve itself, but the cells constituting neural crest migrate and differentiate into various tissues such as cerebral or spinal ganglion, sympathetic nerve and its ganglion, adrenal medulla and melanocyte.

The term "cell of neural tube" means a cell which constitutes a neural tube in the above generation process.

The term "cell of neural crest" means a cell which constitutes a neural crest in the above generation process.

The method for inducing differentiation of the present invention is suitably used for the differentiation induction into the cells of neural tube and neural crest.

The cell of neural tube induced from an embryonic stem cell by the method of the present invention includes a cell characterized as a cell of neural tube before the step in which the dorso-ventral axis is determined, which is capable of differentiating into a cell positioned at the ventral side by reacting with sonic hedgehog (hereinafter referred to as "shh") as a ventral factor of neural tube and of differentiating into a cell positioned at the dorsal side by reacting with bone morphogenetic protein 4 (hereinafter referred to as "BMP4") as a dorsal factor of neural tube. Also, a cell of the neural tube ventral side, expressing a marker HNF-3β (hepatocyte nuclear factor-3β, hereinafter referred to as "HNF-3 β") positioned on the basal plate of the most ventral side of neural tube, a cell of the neural tube ventral side, expressing a marker Nkx2.2 existing secondary to the HNF-3β from the ventral side of neural tube, and a cell of the neural tube dorsal side, expressing Pax-7, all of which are differentiated from the above cell, are also included as neural tube cells induced from an embryonic stem cell by the method of the present invention.

The cell of neural crest induced from an embryonic stem cell by the method of the present invention includes a cell which is characterized as a cell expressing AP-2 (activator protein 2, hereinafter referred to as "AP-2").

shh is a secretory factor relating to the morphogenesis in the early stage of development, such as formation of the dorso-ventral axis of neural tube and formation of the antero-posterior axis of limb bud (C. Chiang *et al*., *Nature*, 383, 407 (1996); M. Bitgood *et al*., *Curr*. *Biol*., 6, 298 (1996)).

BMP4 is a secretory factor relating to the morphogenesis in the early stage of development, which reacts as a dorsal factor such as formation of the dorso-ventral axis of neural tube and formation of the dorso-ventral axis of mesoderm (J.M. Graff *et al*., *Cell,* 79, 169 (1994); A. Suzuki *et al*., *Proc. Natl*. *Acad*. *Sci. USA*, 91, 10255 (1994)).

It is known that HNF-3β is expressed in the liver, small intestines, lungs and pancreas Langerhans' cell after birth and is also expressed at the developmental stage in the intestinal epithelium and liver primordium of in and after the fore-gut forming stage or, through the gastulation stage, in organizer regions such as dorsal lip part, pronotochordal plate, notochord and ventral central part of neural tube, and it is known that this is an important factor in controlling a signal for the body axis pattern formation of neural tube and metamere at the time of development (C. Vaisse *et al*., *Diabetes*, 46, 1364 (1997); M. Levinson-Dushnik *et al*., *Mol*. *Cell. Biol.,* 17, 3817 (1997)).

It is known that Nkx2.2 is a factor which is expressed in the ventral side of neural tube at the developmental stage, and plays an important role in the differentiation and function of these cells (L. Sussel *et al*., Development, 125, 2213 (1998); J. Briscoe *et al*., *Nature*,398, 622 (1999)).

Pax-7 is a factor which is expressed solely in the dorsal part of neural tube (B. Jostes *et al*., *Mech*. *Dev.,* 33, 27 (1990) and plays an important role in the differentiation formation of head neural crest-derived tissue and central nervous system (A. Mansouri *et al*., *Development*, 122, 831 (1996)).

AP-2 is a factor which is expressed in neural crest cell and head perception ganglion, spinal ganglion and facial mesenchyme as important tissues derived from the neural crest cell, in the mouse-derived embryo of 8.5 to 12.5 days of fetal age, and plays an important role in the differentiation and function of these cells (H. *Schorle et al*., *Nature*, 381, 235 (1996); J. Zhang *et al*., *Nature,* 381, 238 (1996)). As transcription factors other than AP2, Pax-3 and twist can be exemplified, which are expressed in the neural crest cell and take part in the head capsule atresia.

Since marker genes for these cells of neural tube and neural crest and factors which exert influences on the developmental polarity of these cells are known, a cell can be specified by detecting mRNA of the marker gene, detecting the expressed marker gene product itself or examining a response to the factor.

### (iv) Retinal pigment cell

Retina as a sensory nerve epithelial tissue on the innermost side of eyeball comprises a nervous layer and a pigment epithelium which are developed from eye primordium (eye capsule). The nervous layer is constituted by two kinds of visual cells (rod cell and pyramid), a group of nerve cells and glial cells which support them. The proximal region of visual cells is rich in acetylcholine esterase and forms a functional synapse with nerve cells. The energy of light captured by the visual pigment contained in the membrane structure of the outer segment of visual cell is converted in a photo-scientific manner and transmitted into the brain via visual cells, and thalamus cells are excited thereby to form visual sense. The pigment epithelium comprises pigment epithelial cells generally containing melanin granules in a large amount and forms a dark membrane closely adhered to the outer segment of visual cells via a large number of cytoplasmic processes. The pigment epithelial cell not only sends melanin granules into its cytoplasmic processes in response to the quantity of light, thereby adjusting the luminous energy to be given to the visual cells, but also is concerned in the periodical regeneration of the outer segment of visual cells by the phagocytic activity.

The retinal pigment cell is a cell positioning in the outermost layer of the pigment granule-containing retina in the eyeball structure, namely a pigment epithelial cell.

In the pigment epithelial cell, its marker gene and a factor which has influence upon the developmental direction of this cell are known, so that this cell can be specified by detecting mRNA of the marker gene, by detecting the expressed marker gene product itself or by examining its response to the factor.

### (b) Epidermal system cell

Examples of an epidermal system cell include an epidermal cell and the like.

The skin comprises an ectoderm-derived epithelial tissue epidermis and a mesoderm-derived connective tissue dermis, and the epidermal cell is defined as an epithelial cell which constitutes the epidermis. The epidermis basically comprises a keratinized stratified squamous epithelium comprising, from the dermis toward the outer surface, stratum basale, stratum spinosum, stratum granulosum, stratum lucidum and stratum comeum. The epidermal cell is classified by using morphology of the cell and expression mode of keratin filament as indexes. Since keratins 8 and 18 are expressed at the early stage of development, they are used as a marker of an epithelial cell at the early fetal period (R.G. Oshima *et al*., *Dev. Bio.,* 99, 447 (1983)). Keratin 19 is used as a marker of an epithelial cell in a fetus (P.C. Stasiak & E.B. Lane, *Nucleic Acids Res.,* 15, 10058 (1987)). Keratins 5 and 14 are used as a marker of an epithelial cell which constitutes the stratum basale of epidermis (E. Fuchs & H. Green, *Cell,* 19, 1033 (1980)). The epidermal cell during keratinization is called keratinocyte, and expression of keratins 5 and 14 decreases as the keratinization progresses but expression of keratins 1 and 10 increases instead (E. Fuchs & H. Green, *Cell,* 19, 1033 (1980); C. Bagutti *et al*., *Dev. Biol*., 179, 184 (1996)).

Epidermal system cells, particularly epidermal cells, can be identified by detecting with an antibody against each of these keratins or an antibody against E cadherin which is a marker of non-neuroectodermal cells or by detecting mRNA for these keratin proteins.

Differentiation induction into the epidermal cell of the stratum basale having high cell division ability can be suitably carried out by the method of the present invention.

### (4) Stromal cell

The stromal cell used in the present invention may be any stromal cell, so long as it has SDIA activity. Whether or not the stromal cell has SDIA activity can be examined by the report of Kawasaki *et al*. (H. Kawasaki, *et al*., *Neuron*, 28, 31 (2000)).

Examples include:
(a) a fetal primary culture fibroblast (*Manipulating the Mouse Embryo, A Laboratory Manual*; *Gene Targeting*; *Production of Mutation Mice Using ES Cell);*
(b) an SIHM mouse-derived STO cell (G. Martin, *Proc. Natl*. *Acad*. *Sci. USA,* 78, 7634 (1981); M.J. Evans *et al*., *Nature*, 292, 154 (1981));
(c) a mouse fetus-derived NIH/3T3 cell (J.L. Jainchill *et al*., *J*. *Virol.,* 4, 549 (1969));
(d) a macrophage colony-stimulating factor (M-CSF) deficient mouse calvaria-derived OP9 cell (T. Nakano *et al*., *Science,* 272, 722 (1996));
(e) a mouse calvaria-derived MC3T3-G2/PA6 cell (H. Kodama *et al*., *J*. *Cell. Physiol*., 112, 89 (1982));
(f) a stromal cell obtained by its differentiation induction from an embryonic stem cell which is already confirmed to have a pluripotency (*Manipulating the Mouse Embryo, A Laboratory Manual*); and
(g) a stromal cell obtained by its differentiation induction from a bone marrow-derived mesenchymal stem cell which is shown to have a differentiation potency into various stromal cells (*Science,* 284, 143 (1999)).

Among these, a stromal cell of (c), (d) or (e) is preferred, and a stromal cell of (e) is more preferred.

The stromal cell recognized by the monoclonal antibody produced by a hybridoma FERM BP-7573 obtained in Reference Example 15 (5) is also preferably used. The stromal cell recognized by the monoclonal antibody produced by a hybridoma FERM BP-7573 obtained in Reference Example 15 (5) is identified by immunological assay described in documents (e.g., *Monoclonal Antibodies: Principles and Applications,* Wiley-Liss, Inc., 1995; *Enzyme Immunoassay*, Third Edition, Igaku Shoin, 1987), for example, by an enzyme-antibody method using the antibody, a cell separation method using a flow cytometer or the like.

### (5) Polyanionic compound

The polyanionic compound used in the present invention may be any compound, so long as it is a compound in which one or at least two of unit compound molecules are bound by a polymerization or condensation reaction, and the constituting unit compound molecules are water-soluble compounds having a negative charge and a polymerization degree of 2 or more. Examples include a homopolymer in which its unit compound molecule is a single monomer and a copolymer in which it contains two or more monomers.

The term "having a negative charge" means that the polyanionic compound molecules have a negative charge as a whole in a solution in which the polyanionic compound is dissolved.

The copolymer includes mucopolysaccharides.

The mucopolysaccharide is a long chain polysaccharide comprising a disaccharide repeating unit of a hexosamine and a uronic acid, and one having sulfate group is called sulfated mucopolysaccharide, and the other one having no sulfate group is called non-sulfated mucopolysaccharide.

The sulfated mucopolysaccharide includes chondroitin 4-sulfate, chondroitin 5-sulfate, chondroitin 6-sulfate, dermatan sulfate, heparan sulfate, heparin, keratan sulfate I, keratan sulfate II and the like.

The non-sulfated mucopolysaccharide includes hyaluronic acid, chondroitin and the like.

The homopolymer includes dextran sulfate, carboxymethyldextran, sulfated polyvinyl, polyvinyl sulfite, sulfonated polystyrene, polyacrylic acid, carboxymethylcellulose, cellulose sulfate, polyglutamic acid, polymaleic acid, polymethacrylic acid and the like.

### (6) Basal medium and balanced salt solution

In the present invention, the basal medium and the balanced salt solution used for cell culturing may be any medium and any balanced salt solution, so long as they are a basal medium and a balanced salt solution generally used for culturing of animal cells.

As the basal medium, any medium available for the culturing of animal cells can be used. Examples include BME medium (*Proc. Soc. Exp*. *Biol*. *Med*., 89, 362 (1965)), BGJb medium (*Exp*. *Cell Res.,* 25, 41 (1961)), CMRL 1066 medium (*N*.*Y*. *Academy of Science*, 5, 303 (1957)), Glasgow MEM medium (*Virology*, 16, 147 (1962)), Improved MEM Zinc Option medium (*J*. *National Cancer Inst*., 49, 1705 (1972)), IMDM medium (*In Vitro,* 9, 6 (1970)), Medium 199 medium (*Proc*. *Soc. Exp*. *Biol*. *Med.,* 73, 1 (1950)), Eagle's MEM medium (*Science,* 130, 432 (1959)), Alpha MEM medium (*Nature New Biology,* 230, 310 (1971)), Dulbecco's MEM medium (*Virology*, 8, 396 (1959)), Ham's medium (*Exp*. *Cell Res.,* 29, 515 (1963); *Proc. Natl*. *Acad Sci. USA*, 53, 288 (1965)), RPMI 1640 medium (*J*. *A. M A*., 199, 519 (1967)), Fischer's medium (*Methods in Med Res.,* 10 (1964)), McCoy's medium (*Proc. Soc. Exp. Biol. Med.,* 100, 115 (1959)), William's E medium (*Exp*. *Cell Res.,* 69, 106 (1971); *Exp. Cell Res.,* 89, 139 (1974)), a mixed medium thereof and the like.

Also, any of the embryo culturing media described, e.g., in *Manipulating the Mouse Embryo, A Laboratory Manual* and *Methods in Enzymology*, volume 225, Guide to Techniques in Mouse Development, Academic Press (1993); *Production of Mutation Mice Using ES Cell,* such as M2 medium, M16 medium, Whitten medium and *in vitro* fertilization medium, can be used as a basal medium, so long as it can be used in embryo culturing.

As the balanced salt solution, any solution available for the culturing of animal cells can be used. Examples include Dulbecco's phosphate-buffered saline (D-PBS), phosphate-buffered saline (PBS), Hanks' balanced salt solutions (HBSS), Gey's balanced salt solutions (GBSS), Earle's balanced salt solutions (EBSS), and the like.

### (7) Culturing of embryonic stem cell under non-aggregation conditions

The method for inducing differentiation of the present invention includes a method which comprises a step for preparing embryonic stem cells in a single cell state and a step for culturing the embryonic stem cell under non-aggregation conditions using the solution having SDIA activity of the present invention.

Herein, the term "using the solution having SDIA activity" includes use of a medium comprising the agent for inducing differentiation of the present invention described in the following item 9(1) and use of a culture vessel immobilized with the agent for inducing differentiation of the present invention described in the following item l(11).

Culturing of an embryonic stem cell under non-aggregation conditions means that culturing is started under a single cell state effected by disengaging mutual adhesion of cells, followed by culturing continuously. The single cell state means a condition in which individual cells are separated without mutual adhesion of cells, e.g., with an enzyme digestion.

In culturing, the inoculated cells do not aggregate or form an embryoid body. In order to start culturing of an embryonic stem cell in a single cell state, followed by continuously culturing, the embryonic stem cells are inoculated at a cell density of lower than the cell density used for usual subculturing of embryonic stem cells and cultured. That is, the embryonic stem cell is treated, e.g., with an enzyme digestion, a cell suspension of single cell state is prepared by using a medium and then the cell suspension is cultured under such conditions that individual cells are present without mutual contact in the culturing system. Such culturing is fundamentally different from the idea of a conventional embryoid body-employed method for generating the differentiation induction by positively aggregating cells and thereby reproducing a pseudo-embryo state. In this case, the cell density of inoculating embryonic stem cell by which individual cells are present without mutual contact in the culturing system is preferably from several tens to several hundreds of cells/cm², more preferably from 30 to 300 cells/cm².

Examples of the method for obtaining single cell state embryonic stem cell include a known enzyme digestion method used in tissue cell culturing. Specifically, the embryonic stem cell is proliferated to a stage from several 10% to almost confluent by exchanging the medium on the preceding day, the medium is removed from the culture dish and then the cells are washed with an aqueous phosphate-buffered saline solution (hereinafter referred to as "PBS") several times, preferably 2 to 3 times. After washing, an appropriate enzyme digestion solution (e.g., PBS containing 1 mM EDTA and 0.25% trypsin) is added to the culture dish containing the embryonic stem cells, followed by culturing at 37°C for several tens of minutes, preferably from 5 to 20 minutes. After the enzyme reaction, the cells are suspended in a medium prepared in the following item 2 and centrifuged (e.g., at 4°C and 200xg for 5 minutes) and then the embryonic stem cells are again suspended in the medium to thereby recover the embryonic stem cells in the single cell state.

As the culturing method of the present invention for inducing differentiation of an ectodermal cell and an ectoderm-derived cell from an embryonic stem cell, any method can be used, so long as it is suitable for the differentiation induction of the embryonic stem cell to be used. Examples include a monolayer culturing method, a micro-carrier culturing method, a perfusion culturing method, a soft-agar culturing method and the like. Specific examples include a method in which a single cell state embryonic stem cell is cultured in a medium prepared in the following item 2, a method in which a single cell state embryonic stem cell is cocultured with a stromal cell produced in advance in the following item 4 in a culture vessel immobilized with the agent for inducing differentiation prepared in the following item 1(11) for several days under non-aggregation conditions, and the like.

The step of the present invention for culturing an embryonic stem cell under non-aggregation conditions is preferably carried out under serum-free culture conditions, but it is possible to carry out, after serum-free culturing, a step for culturing under serum-added culturing conditions (e.g., a step in which culturing is carried out at 37°C in a stream of several percents, preferably 5%, of carbon dioxide in a CO₂ incubator, in a medium prepared by adding preferably of several ten percents, more preferably from 5 to 20%, of a mammal serum to the basal medium described in the following item 2). Particularly, in differentiation induction in an epidermal system cell, the differentiation induction ratio can be further improved by including this step for culturing under serum-added culturing conditions.

By this method, the ectodermal cell or ectoderm-derived cell of the present invention can be obtained. By the method of the present invention, the embryonic stem cell is differentiation-induced into the ectodermal cell or ectoderm-derived cell, and 5% or more, preferably 15% or more, more preferably 40% or more, and most preferably 80% or more, of the embryonic stem cell subjected to the method for inducing differentiation of the present invention can be differentiation-induced into an ectodermal system cell (an ectodermal cell or ectoderm-derived cell).

Differentiation of an ectodermal cell or an ectoderm-derived cell into a nervous system cell can be induced by continuing culturing by a method including the above step while optionally exchanging the medium.

In order to induce differentiation of an ectodermal cell or an ectoderm-derived cell into an epidermal system cell, it is preferable to add BMP4 to a culturing system including the above step.

In order to induce differentiation of an ectodermal cell or an ectoderm-derived cell into a cell of neural tube or neural crest, the above step using a medium which does not contain BMP4 is carried out and then, when differentiation of the embryonic stem cell into a neuroectoderm is started (e.g., 1 to 14 days, preferably 2 to 8 days, and more preferably 4 to 6 days after starting of culturing), culturing in a medium containing shh or BMP4 is continuously carried out along with optional exchange of the medium.

### (8) Culturing in the presence of stromal cell

In the method for inducing differentiation of the present invention which comprises culturing the embryonic stem cell under non-aggregation conditions by using the solution having SDIA activity, the embryonic stem cell may be cultured in the presence of a stromal cell or the solution having SDIA activity from which a stromal cell is removed.

The stromal cell which can coexist includes the stromal cell in the above item 4.

In the method for inducing differentiation of the present invention, the ratio of stromal cell to embryonic stem cell in the culturing system may be any ratio, so long as the embryonic stem cell can be differentiation-induced into an ectodermal cell or an ectoderm-derived cell by the ratio. But it is from 10⁴ to 1 per 1 (the number of stromal cells per the number of embryonic stem cells), preferably from 10³ to 1 per 1, and more preferably from 10² to 10 per 1.

In this case, coculturing of an embryonic stem cell with a stromal cell includes coculturing in which the embryonic stem cell and stromal cell are physically contacted with each other and coculturing in which both cells are present in the same culturing system but cannot be contacted physically with each other due to their separation by a partition wall through which substances can pass.

Coculturing in which the embryonic stem cell and stromal cell are present in the same culturing system but cannot be contacted physically with each other due to their separation by a partition wall through which substances can pass includes coculturing in which both cells are separately cultured using a filter generally used in cell culture can be exemplified. The filter may have a pore size of preferably from 0.01 to several tens µm, more preferably from 0.02 to 12 µm. Specific examples of the filter include Membrane Culture Insert (manufactured by Iwaki Glass), Nunc TC Insert (manufactured by Nunc), COCULTURE Dish (manufactured by Greiner), Cell Culture Insert (manufactured by Falcon), Chemotaxis Chamber (manufactured by Neuro Probe Inc.) and the like. Either of the embryonic stem cell and the stromal cell can be cultured on the filter, but it is preferable to culture the stromal cell on the filter.

Specific examples of the method for inducing differentiation of an ectodermal cell and a nervous system cell from an embryonic cell by coculturing the embryonic cell and stromal cell include a method in which the recovered embryonic stem cell is suspended in a medium prepared in the following item 2 (e.g., a medium prepared by adding 10% KNOCKOUT™ SR (manufactured by GIBCO BRL), 2 mM glutamine, 50 U/ml penicillin, 50 U/ml streptomycin, 100 µM MEM non-essential amino acids solution, 1 mM pyruvic acid and 100 µM 2-mercaptoethanol to the Glasgow MEM medium), the suspension is inoculated at a cell density of several tens to several hundreds of cells/cm², preferably 100 cells/cm², into a culture vessel (e.g., a cell culture flask) in which the stromal cell produced in the following item 4 is cultured, and then the cells are cultured at 37°C for 5 to 20 days, preferably 7 to 10 days, in a stream of several percent, preferably 5%, of carbon dioxide in a CO₂ incubator.

Specific examples of the method for effecting differentiation induction of an ectodermal cell and an epidermal system cell from an embryonic cell by coculturing the embryonic cell and stromal cell include a method in which the recovered embryonic stem cell is suspended in a medium prepared in the following item 2 (e.g., a medium prepared by adding 10% KNOCKOUT™ SR (manufactured by GIBCO BRL), 2 mM glutamine, 50 U/ml penicillin, 50 U/ml streptomycin, 100 µM MEM non-essential amino acids solution, 1 mM pyruvic acid, 100 µM 2-mercaptoethanol and 0.1 to 100 ng/ml, preferably 1 to 50 ng/ml, BMP4 to the Glasgow MEM medium), the suspension is inoculated at a cell density of several tens to several hundreds of cells/cm², preferably 100 cells/cm², into a culture vessel (e.g., a cell culture flask) in which the stromal cell produced in the following item 4 is cultured, and then the cells are cultured at 37°C for 5 to 20 days, preferably 7 to 10 days, in a stream of several percent, preferably 5%, of carbon dioxide in a CO₂ incubator.

In addition, instead of coculturing the embryonic stem cell with stromal cell, the ectodermal cell and ectoderm-derived cell can also be differentiation-induced from the embryonic cell by using a medium prepared by adding a culture supernatant of OP9 cell (T. Nakano *et al*., *Science*, 272, 722 (1996)), NIH/3T3 cell (J.L. Jainchill *et al*., *J*. *Virol*., 4, 549 (1969)) or MC3T3-G2/PA6 cell to the embryonic stem cell culturing medium. Furthermore, instead of coculturing the embryonic stem cell with stromal cell, the ectodermal cell and ectoderm-derived cell can also be differentiation-induced from the embryonic cell by using a medium prepared by adding a factor produced by OP9 cell, NIH/3T3 cell, MC3T3-G2/PA6 cell (H. Komada *et al*., *J*. *Cell. Physiol*., 112, 89 (1982)), STO cell (G. Martin, *Proc. Natl*. *Acad*. *Sci. USA*, 78, 7634 (1981); M.J. Evans *et al*., *Nature,* 292, 154 (1981)) or fetal primary culture fibroblast *(Manipulating* *the Mouse Embryo*, *A Laboratory Manual, Gene Targeting*; *Production of Mutation Mice Using ES Cell*) to the medium.

### (9) Culturing without using retinoic acid

According to the method for inducing differentiation of the present invention, it is preferred to culture the embryonic stem cell without using retinoic acid in the culturing step of the cell under non-aggregation conditions.

The term "culturing without using retinoic acid" means that the cell is cultured without using retinoic acid at a non-physiological concentration. The non-physiological concentration means a concentration higher than the *in vivo* physiological concentration. Specifically, since it is known that retinoic acid is present in human blood generally at a concentration of about 10⁻⁸ mol/l (*Biochemistry Dictionary* (*Seikagaku Jiten*), second edition, Tokyo Kagaku Dojin (1992)), a concentration range of 10⁻⁷ to 10⁻⁶ mol/l is the non-physiological concentration. Since retinoic acid has an action as a morphogen which has influence upon the morphogenesis at the time of developmental differentiation and, depending on the cell species, also has strong toxicity, there is a possibility of causing secondary side effects when a culture system using retinoic acid at a non-physiological concentration is applied to a medical treatment. Thus, since the risk accompanied by the use of retinoic acid can be avoided, culturing without using retinoic acid is useful.

### (10) Culturing in which differentiation of mesodermal system cell is not substantially induced

According to the method for inducing differentiation of the present invention, it is preferred that differentiation of a mesodermal system cell is not substantially induced in the culturing system.

The term "differentiation of a mesodermal system cell is not substantially induced" as used herein means that the ratio of a mesodermal system cell differentiated in the culturing system is 5% or less, preferably 2% or less, based on the total number of cells in the culturing system.

In this case, the mesodermal system cell means a cell comprising organs and tissues such as muscular system, connecting tissue, skeletal system, circulatory organ system, urinary organ system, and reproductive system.

The mesodermal system cell can be identified by using, e.g., a method in which it is detected by using an antibody which specifically recognizes the mesodermal system cell, a method in which mRNA for a protein specifically expressed in the mesodermal system cell is detected or a method in which it is detected by using an antibody which specifically recognizes the protein.

### (11) Culture vessel

As the culture vessel used in the present invention, any culture vessel which can culture the embryonic stem cell can be used, but a culture vessel for cell culturing is preferred. Examples of the culture vessel for cell culturing include a flask, a tissue culture flask, a dish, a Petri dish, a tissue culture dish, a Conzar dish, a Permanox dish, a multi-dish, a microplate, a micro-well plate, a multi-plate, a multi-well plate, a separate strip well, a Terasaki plate, a tissue culture chamber slide, a schale, a cell culture schale, a tissue culture tube, a tray, a cell culture tray, a cell factory, a culture bag, a techno pot, a roller bottle, a spinner, a hollow fiber and the like. In order to control adhesiveness of the culture vessel and cells, the cell-contacting side surface of the culture vessel can be artificially treated. Examples of the artificial treatment of the culture vessel surface include collagen coating, gelatin coating, poly-L-lysine coating, fibronectin coating, laminin coating, proteoglycan coating, glycoprotein coating, matrigel coating, silicon coating and the like. In addition, the vessel can also be treated to provide a negative electric charge such as Primaria (manufactured by Falcon).

Furthermore, the culture vessel which can be used in the present invention includes the culture vessel immobilized with the agent for inducing differentiation of the present invention described in the following item 9(1). Generally, the method for immobilizing the culture vessel with the agent for inducing differentiation of the present invention includes a method in which a solution comprising the agent for inducing differentiation of the present invention is added to a culture vessel for culturing of animal cells, followed by culturing at room temperature for several minutes to several hours. Moreover, the efficiency for immobilizing the agent for inducing differentiation of the present invention can be increased by using a culture vessel which is subjected to polymeric surface treatment such as plasma treatment, corona discharge treatment and ozone treatment described in literatures (*Basis and Application of Polynieric Surface,* Kagaku Dojin (1986); H.P. Wendel & G. Ziemer, *etc. Eur. J*. *Cardiothorac. Surg*., 10, 54 (1996)).

### 2. Production of medium

With regard to the medium of the present invention used in the method for differentiation-inducing an ectodermal cell and ectoderm-derived cell from an embryonic stem cell, a medium usually used in culturing of animal cells can be prepared as a basal medium.

As the basal medium, the media described in the above item 1(6) are mentioned, and any medium available for the culturing of animal cells can be used.

Also, any of the embryo culturing media described, e.g., in *Manipulating the Mouse Embryo, A Laboratory Manual* and *Methods in Enzymology*, volume 225, Guide to Techniques in Mouse Development, Academic Press (1993); *Production of Mutation Mice Using ES Cell,* such as M2 medium, M16 medium, Whitten medium and *in vitro* fertilization medium, can be used as a basal medium, so long as it can be used in embryo culturing.

Moreover, any one of media produced by adding various growth factors as serum substitutes, or a protein-free medium capable of culturing animal cells and embryo can also be used as the basal medium. Specific examples include a serum-free medium to which commercially available KNOCKOUT™ SR is added (M.D. Goldsborough *et al*., *Focus,* 20, 8 (1998)), a serum-free medium to which insulin and transferrin are added (e.g., CHO-S-SFM II (manufactured by GIBCO BRL), Hybridoma-SFM (manufactured by GIBCO BRL), eRDF Dry Powdered Media (manufactured by GIBCO BRL), UltraCULTURE™ (manufactured by BioWhittaker), UltraDOMA™ (manufactured by BioWhittaker), UltraCHO™ (manufactured by BioWhittaker), UltraMDCK™ (manufactured by BioWhittaker), ITPSG medium (S. Hosoi *et al*., *Cytotechnology,* 5, S17 (1991)), ITSFn medium (A. Rizzino and C. Growley, *Proc. Natl*. *Acad Sci. USA*, 77, 457 (1980)) or mN3 medium (S. Okabe *et al*., *Mech*. *Dev.,* 59, 89 (1996)), a medium to which a cell-derived factor is added (e.g., a medium to which a culture supernatant of a pluripotency teratocarcinoma cell PSA1 is added (G.R. Martin, *Proc. Natl*. *Acad*. *Sci. USA,* 78, 7634 (1981)), a medium which contains a culture filtrate of the stromal cell described in the following item 4, a medium which contains a factor produced by the stromal cell described in the following item 5, a medium which contains an antigen component obtained in the following item 8, a medium which contains BMP4 and a protein-free medium (e.g., CD-CHO (manufactured by GIBCO BRL), PFHM-II (manufactured by GIBCO BRL) or UltraDOMA-PF™ (manufactured by BioWhittaker)).

The medium used in the method for inducing differentiation of an embryonic stem cell into an ectodermal cell or an ectodermal-derived cell can be prepared by adding to the basal medium several ones to several tens %, preferably 2 to 40%, and more preferably 2 to 20% of the agent for inducing differentiation of the present invention described in the following item 9(1). The preferable medium includes a medium in which 10% KNOCKOUT™ SR (manufactured by GIBCOBRL), 2 mM glutamine, 50 U/ml penicillin, 50 U/ml streptomycin, 1 mM 2-mercaptoethanol and 20% of the agent for inducing differentiation described in the following item 9(1) are added to Dulbecco MEM medium.

In the method for inducing differentiation of the present invention, the above-described medium to which the agent for inducing differentiation having SDIA activity is preferably used. When the embryonic stem cell is cultured in the culture vessel immobilized with the agent for inducing differentiation, the above-described medium comprising the agent for inducing differentiation of the present invention is not necessarily used, and a medium having the same components except for the absence of the agent for inducing differentiation of the present invention can be used.

### 3. Production of embryonic stem cell

Production methods of the embryonic stem cells (a), (b) and (c) described in the item 1 (2) are specifically described.

### (1) Production of embryonic stem cell established by culturing early stage embryo before implantation

An embryonic stem cell can be produced from an early stage embryo before implantation by culturing the early stage embryo according to the method described in a reference (*Manipulating the Mouse Embryo, A Laboratory Manual*).

Examples of the method for culturing the obtained embryonic stem cell include the embryonic stem cell culturing methods described in literatures (*Manipulating the Mouse Embryo, A Laboratory Manual*; *Methods in Enzymology*, volume 225, Guide to Techniques in Mouse Development, Academic Press (1993); *Production of Mutation Mice Using ES Cell;* and the like). Also, it is possible to carry out serum-free culturing; e.g., the cell can be subcultured while keeping its characters as an undifferentiated embryonic stem cell in a Dulbecco's MEM medium supplemented with 15 to 20% KNOCKOUT™ SR (manufactured by GIBCO BRL), 2 mM glutamine, 100 µM MEM non-essential amino acids solution, 50 U/ml penicillin, 50 U/ml streptomycin, 100 µM 2-mercaptoethanol and 1,000 U/ml LIF (M.D. Goldsborough *et al*., *Focus,* 20, 8 (1998)).

### (2) Production of embryonic stem cell nuclear-transplanted with the nucleus of somatic cell

An egg transplanted with the nucleus of a somatic cell of a mammal cell and started normal development can be produced in the following manner using a method reported by, e.g., Wilmut *et al*. (*Nature,* 385, 810 (1997)), Cibelli *et al*. (*Science,* 280, 1256 (1998)), A. Iritani *et al*. (*Protein, Nucleic Acid and Enzyme,* 44, 892 (1999)), *Baguisi et al*. (*Nature Biotechnology*, 17, 456 (1999)), Wakayama *et al*. (*Nature,* 394, 369 (1998); *Nature Genetics*, 22, 127 (1999); *Proc. Natl. Acad*. *Sci. USA,* 96, 14984 (1999)) or Rideout III *et al* (*Nature Genetics*, 24, 109 (2000)).

An egg which acquired the nucleus of other somatic cell and started normal development can be obtained by starting its development using a method in which the nucleus of a mammal cell is excised, initialized (an operation to return the nucleus to such a state that it can repeat the development again) and injected into an enucleated unfertilized egg of a mammal, and then incubating the development-started egg.

As the method for initializing the nucleus of a somatic cell, several methods are known. For example, the following methods are known.

The initialization can be carried out by changing the medium for culturing a nuclear donor side cell from a medium containing from 5 to 30%, preferably 10%, of fetal calf serum (e.g., M2 medium) to a poor nutrient medium containing from 0 to 1%, preferably 0.5%, of fetal calf serum and culturing the cell for a period of 3 to 10 days, preferably 5 days, thereby to induce the cell cycle into an interphase state (G0 phase or G1 phase). This method is suitable, for example, when the mammal is sheep, goat or cattle.

Also, the initialization can be carried out by injecting the nucleus of a nucleus donor side cell into an enucleated unfertilized egg of a mammal of the same species and incubating the egg for several hours, preferably from about 1 to 6 hours. This method is suitable, for example, when the mammal is a mouse.

The thus initialized nucleus becomes possible to start its development in an enucleated unfertilized egg. Several methods are known as the method for starting development of the initialized nucleus in an enucleated unfertilized egg. The development can be started by transplanting a nucleus initialized by inducing the cell cycle into an interphase state (G0 phase or G1 phase) into an enucleated unfertilized egg of a mammal of the same species, e.g., by electrofusion to thereby activate the egg. This method is suitable, for example, when the mammal is sheep, goat or cattle.

Development of the nucleus initialized by injecting it into an enucleated unfertilized egg of a mammal of the same species can be carried out by again transplanting it into an enucleated unfertilized egg of a mammal of the same species, for example, using a method which uses a micromanipulator, stimulating it with an egg activating factor (e.g., strontium) and then treating it with a cell division inhibitor (e.g., cytochalasin B) to inhibit release of a secondary polar body. This method is suitable, for example, when the mammal is a mouse.

Once an egg which started the development is obtained, the embryonic stem cell can be obtained by a known method described in, for example, *Manipulating the Mouse Embryo*, *A Laboratory Manual*; *Gene Targeting*; *Production of Mutation Mice Using ES Cell* and the like.

### (3) Production of embryonic stem cell in which gene on its chromosome is modified

An embryonic stem cell in which a gene on its chromosome is modified can be produced by using homologous recombination techniques.

Examples of the chromosomal gene to be modified include genes for histocompatibility antigens and genes related to diseases caused by disorders of nervous system cells or epidermal system cells.

Modification of the target gene on the chromosome can be carried out by using a method described in, for example, *Manipulating the Mouse Embryo*, *A Laboratory Manual; Gene Targeting; Production of Mutation Mice Using ES Cell* and the like.

Specifically, a genomic gene of the target gene to be modified (e.g., a histocompatibility antigen gene or a disease-related gene) is isolated, and a target vector for homologous recombination of the target gene is produced by using the isolated genomic gene. An embryonic stem cell having a modified chromosomal gene can be produced by introducing the thus produced target vector into embryonic stem cells and selecting a cell in which homologous recombination occurred between the target gene and the target vector.

Examples of the method for isolating genomic gene of the target gene include a known method described in *Molecular Cloning, A Laboratory Manual,* Second Edition, Cold Spring Harbor Laboratory Press (1989) (hereinafter referred to as *"Molecular Cloning,* Second Edition") or in *Current Protocols in Molecular Biology,* John Wiley & Sons (1987-1997) (hereinafter referred to as "*Current Protocols in Molecular Biology*") and the like. The genomic gene of the target gene can also be isolated, for example, using Genome DNA Library Screening System (manufactured by Genome Systems) or Universal GenomeWalker™ Kits (manufactured by CLONTECH).

The target vector for carrying out homologous recombination of the target gene can be produced by using the method described in, for example, *Gene Targeting; Production of Mutation Mice Using ES Cell* and the like. As the target vector, any one of its replacement type and insertion type can be used.

Examples of a method for efficiently selecting a homologous recombinant include a method such as the positive selection, promoter selection, negative selection or poly(A) selection described in, for example, *Gene Targeting*; *Production of Mutation Mice Using ES Cell* and the like.

Examples of the method for the selection of the homologous recombinant of interest from the selected cell lines include the Southern hybridization for genomic DNA *(Molecular Cloning,* Second Edition), PCR *(PCT Protocols,* Academic Press (1990)) and the like.

### 4. Method for preparing the solution having SDIA activity of the present invention

A solution having SDIA activity can be prepared by the method of the present invention from a stromal cell having the activity. Specifically, a culture is prepared by adding 0.000001 to 1% (w/v), preferably 0.00001 to 0.1% (w/v), and more preferably 0.0001 to 0.01% (w/v), of a polyanionic compound to a basal medium or a balanced salt solution used for cell culturing, a stromal cell is washed several times, preferably 2 to 3 times, with the basal medium or the balanced salt solution which does not contain the polyanionic compound and cultured in the prepared culture for several minutes to several hours, preferably from 15 minutes to 2 hours, and more preferably from 30 minutes to 1 hour, and then the culture is recovered by carrying out an operation for separating the cells and culture, which is generally carried out in culturing of animal cells, to obtain a solution having SDIA activity from the stromal cell.

In the method of the present invention for preparing a solution having SDIA activity from a stromal cell having the activity, a culture comprising a polyanionic compound is used, and in this case, a basal medium or a balanced salt solution used for culturing of animal cells can be used as the culture to which a polyanionic compound is added. The basal medium and balanced salt solution to be used in culturing of animal cells includes the basal media and the balanced salt solution described in the above item 1(6).

As the polyanionic compound, any compound having the ability to extract the SDIA activity present on the stromal cell membrane can be used, and a homopolymer or a sulfated mucopolysaccharide or non-sulfated mucopolysaccharide as a copolymer, which has a negative charge in the culture can be used. Examples include the polyanionic compounds described in the above item 1(5).

By method for inducing differentiation of the present invention which uses the solution having SDIA activity obtained from a stromal cell according to the present invention, differentiation of an embryonic stem cell into an ectodermal cell or an ectoderm-derived cell can be induced by culturing the embryonic stem cell under the above-described non-aggregation conditions.

As the stromal cell used in the present invention, any stromal cell can be used, so long as it has SDIA activity. Whether or not it has activity to induce differentiation of an embryonic stem cell can be examined according to the report of Kawasaki *et al*. (H. Kawasaki *et al*., *Neuron,* 28, 31 (2000)), and the stromal cells described in the above item 1(4) are exemplified.

The culturing of a stromal cell is preferably subcultured by the method used for its establishment. Also, useful are the methods for culturing feeder cells used in the culturing of embryonic stem cells, described in *Manipulating the Mouse Embryo, A Laboratory Manual, Methods in Enzymology*, Vol. 225, Guide to Techniques in Mouse Development, Academic Press (1993), *Gene Targeting, Production of Mutation Mice Using ES Cell* and the like. For example, the culturing can be carried out by using Dulbecco's MEM medium (manufactured by GIBCO BRL) supplemented with 10% fetal bovine serum (manufactured by GIBCO BRL), 2 mM glutamine, 50 U/ml penicillin and 50 U/ml streptomycin.

After sub-culturing of the above-described stromal cell, the SDIA activity is preferably extracted by using cells which reached almost confluent.

The amount of the basal medium or the balanced salt solution containing polyanionic compound to be added to the washed stromal cells is preferably the same amount or from 1/2 to 1/3 of the medium used for the culturing of the stromal cells. Specifically, when stromal cells were cultured by using a flask for cell culture having a culture area of 25 cm² (manufactured by FALCON), about 3 to 10 ml of the basal medium or balanced salt solution containing a polyanionic compound is preferably used

A culture of the stromal cells cultured by using the basal medium or the balanced salt solution containing a polyanionic compound can be recovered by an operation for separating the cells and the culture which is generally carried out in culturing of animal cells, for example, centrifugation separation, separation by using a filter of 0.22 to 0.45 µm in pore size, or the like.

The recovered solution can be stored at 4°C or under freezing.

When the stromal cell is used in culturing for inducing differentiation of an embryonic stem cell into an ectodermal cell or an ectoderm-derived cell, stromal cells proliferated on an appropriate support such as a culture dish may be used as living cells, or cells which lost the proliferation ability by undergoing a physicochemical treatment can be used. The cells which lost the proliferation ability by physicochemical treatment are cells from which the ability of forming next generation progenies accompanied by gene replication is completely lost, specifically, those cells which are obtained by culturing with an antitumor agent-containing medium, by a lethal dose radiation irradiation or by applying treatment for tissue fixation used in pathologic diagnosis.

The living stromal cells can be produced, for example, by washing the cells whose cell density reached almost confluent by exchanging the medium on the preceding day, several times with PBS, and then adding a medium of the present invention obtained in the above item 2 (e.g., a serum-free medium used in culturing for inducing differentiation of an embryonic stem cell into an ectodermal cell or an ectoderm-derived cell). Alternatively, they can also be produced by digesting the cells which reached almost confluent with an appropriate digestive enzyme (e.g., PBS containing 0.02% EDTA and 0.05 to 0.25% trypsin or actinase), suspending the thus recovered cells in a medium of the present invention obtained in the above item 2 (e.g., a serum-free medium used in culturing for inducing differentiation of an embryonic stem cell into an ectodermal cell or an ectoderm-derived cell), and then inoculating the suspension into a culture vessel (e.g., a tissue culture dish coated with 0 to 1%, preferably 0.1%, of gelatin) and culturing for about 1 day.

The stromal cell which lost the proliferation ability by culturing in an antitumor agent-containing medium can be produced by a method described in, for example, *Manipulating the Mouse Embryo, A Laboratory Manual*; *Gene Targeting*; *Production of Mutation Mice Using ES Cell* or the like. For example, it can be produced by culturing the cells whose cell density reached almost confluent by exchanging the medium on the preceding day, in a medium containing 1 to 100 µg/ml, preferably 10 µg/ml, of mitomycin C for several hours, preferably 2 to 3 hours, washing the resulting cells several times with PBS, digesting the cells with an appropriate digestive enzyme (e.g., PBS containing 0.02% EDTA and 0.05 to 0.25% trypsin or actinase), suspending the thus recovered cells in a medium of the present invention obtained in the above item 2 (e.g., a serum-free medium used in culturing for inducing differentiation of an embryonic stem cell into an ectodermal cell or an ectoderm-derived cell), and then inoculating the suspension into a culture vessel (e.g., a tissue culture dish coated with 0 to 1%, preferably 0.1%, of gelatin), followed by culturing for about 1 day. In addition, the stromal cell which lost the proliferation ability can also be produced by using other antitumor agent such as 5-fluorouracil, adriamycin, ara-C, or methotrexate at a concentration of 1/10 to 10 times of, preferably identical to, the concentration used in the living body described in *The Pharmacopoeia of Japan*, instead of mitomycin C.

The stromal cell which lost the proliferation ability by receiving lethal dose of a radiation irradiation can be produced by using a method described in, for example, in *Tissue Culture Techniques,* Asakura Shoten (1982); *Tissue Culture Techniques* (Second Edition), Asakura Shoten (1988); *Tissue Culture Techniques* (Third Edition), Asakura Shoten (1996); or the like. For example, it can be produced by exposing the cells whose cell density reached almost confluent by exchanging the medium on the preceding day to 200 to 5,000 rad, preferably 500 to 1,000 rad, of an X-ray or γ-ray, washing the cells several times with PBS, and then adding a medium of the present invention obtained in the above item 2 (e.g., a serum-free medium to be used in the culturing for inducing differentiation of an embryonic stem cell into an ectodermal cell or an ectoderm-derived cell). Alternatively, it can also be produced by digesting the radiation-irradiated cells with an appropriate digestive enzyme (e.g., PBS containing 0.02% EDTA and 0.05 to 0.25% trypsin or actinase), suspending the thus recovered cells in a medium of the present invention obtained in the above item 2 (e.g., a serum-free medium used in culturing for inducing differentiation of an embryonic stem cell into an ectodermal cell or an ectoderm-derived cell), and then inoculating the suspension into a culture vessel (e.g., a tissue culture dish coated with 0 to 1%, preferably 0.1%, of gelatin), followed by culturing for about 1 day.

The stromal cell which lost the proliferation ability by a tissue fixation treatment used in pathologic diagnosis can be produced by using a method described in, for example, *Histochemistry and Cytochemistry*, Gakusai Kikaku (1987-1999), edited and published every year by Japanese Society of Histochemistry and Cytochemistry, *Basic Techniques for Transmission Electron Microscopy,* Acad. Press (1986), *Electron* *Microscopy Chart Manual,* Igaku Shuppan Center (1993) or the like. Specifically, it can be produced by carrying out a microwave fixation, a rapid freeze-substitution fixation, a glutaraldehyde fixation, a paraformaldehyde fixation, a formalin fixation, an acetone fixation, a Van fixation, a periodic acid fixation, a methanol fixation or an osmic acid fixation. For example, it can be produced by soaking the cells whose cell density reached almost confluent by exchanging the medium on the preceding day, in a solution containing 0.1 to 50%, preferably 1 to 10%, and more preferably from 3 to 5%, of paraformaldehyde at 4°C for, e.g., several minutes to several hours, preferably 5 minutes to 1 hour, and more preferably 30 minutes, and then washing the cells several times with PBS.

### 5. Method for identifying a factor having SDIA activity in a solution having SDIA activity

When a basal medium or balanced salt solution which does not contain a polyanionic compound is used as the culture of the method described in the aforementioned 4, it hardly contains the SDIA activity. Accordingly, a factor which induces differentiation of an embryonic stem cell into an ectodermal cell or an ectoderm-derived cell can be identified by comparing components contained in a stromal cell culture prepared by using a basal medium or a balanced salt solution containing a polyanionic compound as the culture with those in a control stromal cell culture prepared by using a basal medium or a balanced salt solution which does not contain a polyanionic compound. The method for comparing and identifying components in the culture includes a proteome analysis and a purification means using chromatography.

Activity of the identified component can be confirmed by preparing a medium containing the identified component and inducing differentiation of an embryonic stem cell according to the method for inducing differentiation of an embryonic stem cell of the present invention described in the above item 1(7).

### 6. Method for obtaining stromal cell-derived factor having SDIA activity

A factor having SDIA activity can be obtained from the stromal cell of the present invention. Specifically, it can be carried out by using an expression cloning method described in, e.g., *Molecular Cloning,* Second Edition, *Current Protocols in Molecular Biology, Monoclonal Antibodies: principles and practice,* Third Edition, Acad. Press (1993) (hereinafter referred to as "*Monoclonal Antibodies*"), *Antibody* *Engineering, A Practical Approach,* IRL Press at Oxford University Press (1996) (hereinafter referred to as "*Antibody Engineering*"), or the like.

Specifically, for example, cDNA is produced from the stromal cell of the present invention and the cDNA is inserted into downstream of the promoter of an appropriate expression vector to prepare a recombinant vector and a cDNA library. Transformants which produce gene products produced by the stromal cell of the present invention are obtained by introducing the recombinant vector into a host cell suitable for the expression vector, and a transformant which produces a gene product having SDIA activity is selected therefrom. A factor having SDIA activity can be obtained by determining the gene sequence encoded by the cDNA introduced into the selected transformant.

This procedure is described in detail below.

As the host cell, a cell which does not have SDIA activity is preferred. Specific examples include a Chinese hamster ovary-derived CHO cell (T.T. Puck *et al*., *J*. *Exp*. *Med*., 108, 945 (1985)), a female cocker spaniel kidney-derived MDCK cell (C.R. Gaush *et al*., *Proc*. *Soc*. *Exp*. *Biol*. *Med*., 122, 931 (1966); D.S. Misfeldt *et al*., *Proc. Natl*. *Acad*. *Sci*. *USA*, 73, 1212 (1976)), a rat fibroblast 3Y1 (S. Sandineyer *et al*., *Cancer Res*., 41, 830 (1981)) and a green monkey kidney-derived COS cell (Y. Gluzman, *Cell,* 23, 175 (1981)). Among these, the COS cell which is suitable for expression cloning using an SV40 expression vector is preferred.

As the cell to be used in the production of cDNA, a stromal cell having SDIA activity is preferred. Specific examples include a fetal primary culture fibroblast *(Manipulating the Mouse Embryo*, *A Laboratory Manual*; *Production of Mutation Mice Using ES Cell*) and an SIHM mouse-derived STO cell (G. Martin, *Proc. Natl*. *Acad Sci*. *USA*, 78, 7634 (1981)); M.J. Evans *et al*., *Nature*, 292, 154 (1981), more preferably a mouse fetus-derived NIH/3T3 cell (J.L. Jainchill *et al*., *J*. *Virol*., 4, 549 (1969)), an M-CSF deficient mouse calvaria-derived OP9 cell (T. Nakano *et al*., *Science,* 272, 722 (1996)) and a mouse calvaria-derived MC3T3-G2/PA6 cell (H. Kodama *et al*., *J*. *Cell. Physiol*., 112, 89 (1982)).

Examples of the method for producing a cDNA library include methods described in *Molecular Cloning,* Second Edition, *Current Protocols in Molecular Biology, etc*.; methods using a commercially available kit, such as SuperScript Plasmid System for cDNA Synthesis and Plasmid Cloning (manufactured by Life Technologies), ZAP-cDNA Synthesis Kit (manufactured by STRATAGENE); and the like.

As the vector for producing cDNA library, any of a phage vector, plasmid vector and the like can be used, so long as it can autonomously replicate in a microorganism such as *Escherichia coli* K12 and can express cDNA which is introduced into the host cell.

When a phage is used as the host cell, a transformant to which the prepared cDNA has been introduced can be obtained, for example, by using a commercially available kit Recombinant Phage Antibody System (manufactured by Pharmacia).

When prokaryote such as a bacterium is used as the host cell, it is preferred that the recombinant vector comprising the prepared cDNA can autonomously replicate in the prokaryote and is constructed with a promoter, a ribosome binding sequence, the cDNA gene and a transcription termination sequence. A promoter-controlling gene may be contained therein.

Examples of the expression vector include pBTrp2, pBTac1 and pBTac2 (manufactured by Boehringer Mannheim), pKK233-2 (manufactured by Pharmacia), pSE280, pSE380 and pSE420 (manufactured by Invitrogen), pAX and pMEX (manufactured by MOBITEC), pGEMEX-1 (manufactured by Promega), pQE-8 (manufactured by QIAGEN), pKYP10 (Japanese Published Unexamined Patent Application No. 110600/83), pKYP200 (*Agric*. *Biol*. *Chem.,* 48, 669 (1984)), pLSA1 (*Agric*. *Biol*. *Chem.,* 53, 277 (1989)), pGEL1 (*Proc*. *Natl*. *Acad Sci. USA*, 82, 4306 (1985)), pBluescript II SK(-) (manufactured by Stratagene), pTrs30 (prepared from *Escherichia coli* JM109/pTrS30 (FERM BP-5407)), pTrs32 (prepared from *Escherichia coli* JM109/pTrS32 (PERM BP-5408), pGHA2 (prepared from *Escherichia coli* IGHA2 (PERM B-400), Japanese Published Unexamined Patent Application No. 221091/85), pGKA2 (prepared from *Escherichia coli* IGKA2 (FERM BP-6798), Japanese Published Unexamined Patent Application No. 221091/85), pTerm2 (US 4686191, US 4939094, US 5160735), pSupex, pUB110, pTP5, pC194 and pEG400 *(J. Bacteriol,* 172, 2392 (1990)), pGEX (manufactured by Amersham Pharmacia Biotech), pET system (manufactured by Novagen) and the like.

As the promoter, any promoter can be used, so long as it can function in a host cell. Examples include promoters derived from *Escherichia coli,* phage and the like, such as *trp* promoter (Pₜᵣₚ), *lac* promoter, P_{L} promoter, P_{R} promoter, and T7 promoter. Also, artificially designed and modified promoters, such as a promoter in which two Pₜᵣₚ are linked in series (Pₜᵣₚ×2), *tac* promoter, lacT7 promoter, letI promoter and the like, can be used.

A plasmid in which the space between Shine-Dalgarno sequence which is a ribosome binding sequence and the initiation codon is adjusted to a suitable distance (for example, 6 to 18 nucleotides) is preferably used.

Examples of the host cell include microorganisms belonging to the genus *Escherichia*, the genus *Serratia*, the genus *Bacillus,* the genus *Brevibacterium,* the genus *Corynebacterium*, the genus *Microbacterium,* the genus *Pseudomonas* and the like. Specific examples include *Escherichia coli* XL1-Blue, *Escherichia coli* XL2-Blue, *Escherichia coli* DH1, *Escherichia coli* MC1000, *Escherichia coli* KY3276, *Escherichia coli* W 1485, *Escherichia coli* JM109, *Escherichia coli* HB 101, *Escherichia coli* No.49, *Escherichia coli* W3110, *Escherichia coli* NY49, *Serratia ficaria*, *Serratia fonticola*, *Serratia liquefaciens*, *Serratia marcescens, Bacillus subtilis*, *Bacillus amyloliquefaciens*, *Brevibacterium immariophilum* ATCC 14068, *Brevibacterium saccharolyticum* ATCC 14066, *Brevibacterium flavum* ATCC 14067, *Brevibacterium lactofermentun* ATCC 13869, *Corynebacterium glutamicum* ATCC 13032, *Corynebacterium acetoacidophilum* ATCC 13870, *Microbacterium ammoniaphilum* ATCC 15354, *Pseudomonas* sp. D-0110 and the like.

The thus produced cDNA library may be used as such, but, in order to concentrate the target gene, a cDNA library produced by carrying out a subtraction method (*Proc*. *Natl*. *Acad Sci. USA*, 85, 5783 (1988)) using mRNA of a cell which does not have SDIA activity can also be used.

As the method for introducing the recombinant vector, any method can be used, so long as it is a method for introducing DNA into the above host cell. Examples include a method using a calcium ion (*Proc. Natl*. *Acad Sci. USA,* 69, 2110 (1972)), a protoplast method (Japanese Published Unexamined Patent Application No. 248394/88), methods described in *Gene,* 17, 107 (1982) and *Molecular & General Genetics,* 168, 111 (1979), and the like.

When yeast is used as the host cell, examples of the expression vector include YEP13 (ATCC 37115), YEp24 (ATCC 37051), YCp50 (ATCC 37419) and the like.

Any promoter can be used, so long as it can function in yeast. Examples include a promoter of a gene in the glycolysis system such as hexose kinase, PHO5 promoter, PGK promoter, GAP promoter, ADH promoter, gal 1 promoter, gal 10 promoter, a heat shock polypeptide promoter, MFα1 promoter, CUP 1 promoter and the like.

Examples of the host cell include microorganisms belonging to the genus *Saccharomyces,* the genus *Kluyveromyces*, the genus *Trichosporon*, the genus *Schwanniomyces* and the like. Specific examples include *Saccharomyces cerevisiae, Schizosaccharomyces pombe*, *Kluyveromyces lactis, Trichosporon pullulans, Schwanniomyces alluvius* and the like.

As the method for introducing the recombinant vector, any method can be used, so long as it is a method for introducing DNA into yeast. Examples include electroporation (*Methods in Enzymology,* 194, 182 (1990)), a spheroplast method *(Proc. Natl*. *Acad*. *Sci. USA,* 84, 1929 (1978)), a lithium acetate method (*J*. *Bacteriology*, 153, 163 (1983)), a method described in *Proc. Natl. Acad*. *Sci. USA,* 75, 1929 (1978) and the like.

When an animal cell is used as the host, examples of the expression vector include pcDNAI and pcDM8 (manufactured by Funakoshi), pAGE107 (Japanese Published Unexamined Patent Application No. 22979/91; *Cytotechnology,* 3, 133 (1990)), pAS3-3 (Japanese Published Unexamined Patent Application No. 227075/90), pCDM8 (*Nature*, 329, 840 (1987)), EBV Vector (manufactured by Invitrogen), pRc/CMV2 (manufactured by Invitrogen), pRc/RSV (manufactured by Invitrogen), pZeoSV Vector (manufactured by Invitrogen), pcDNAI/Amp (manufactured by Invitrogen), pDisplayp (manufactured by Invitrogen), REP4 (manufactured by Invitrogen), pcDNA3.1 Vector (manufactured by Invitrogen), pXT1 (manufactured by Invitrogen), pSG5 (manufactured by Invitrogen), pBK-CMV (manufactured by Stratagene), pBK-RSV (manufactured by Stratagene), pAGE103 (*J*. *Biochemistry*, 101, 1307 (1987)), pAGE210 and the like.

Any promoter can be used, so long as it can function in the animal cell. Examples include a promoter of IE (immediate early) gene of cytomegalovirus (CMV), an early promoter of SV40, a promoter of retrovirus, a metallothionein promoter, a heat shock promoter, SRα promoter and the like. Also, the enhancer of the IE gene of human CMV can be used together with the promoter.

Examples of the host cell include human Namalwa cell, monkey COS cell, Chinese hamster CHO cell, HBT5637 (Japanese Published Unexamined Patent Application No. 299/88) and the like.

As the method for introducing the recombinant vector, any method can be used, so long as it is a method for introducing DNA into an animal cell. Examples include electroporation (*Cytotechnology*, 3, 133 (1990)), a calcium phosphate method (Japanese Published Unexamined Patent Application No. 227075/90), a lipofection method (*Proc. Natl*. *Acad*. *Sci. USA*, 84, 7413 (1987)) and the like.

When an insect cell is used as the host, a protein can be expressed by a method described in, for example, *Current Protocols in Molecular Biology, Bacurovirus Expression Vectors*, *A Laboratory Manual,* W.H. Freeman and Company, New York (1992), *Bio*/*Technology*, 6, 47 (1988) or the like.

Specifically, a vector for recombinant gene introduction and a baculovirus are cotransfected into an insect cell to thereby obtain a recombinant virus in an insect cell culture supernatant, and then the insect cell is infected with the recombinant virus to express a protein.

Examples of the vector for gene introduction used in the method include pVL1392, pVL1393, pBlueBacIII (all manufactured by Invitrogen) and the like.

Examples of the bacurovirus include *Autographa californica* nuclear polyhedrosis virus which infects insects of the family *Barathra* and the like.

Examples of the insect cell include Sf9 and Sf21 which are *Spodoptera frugiperda* ovary cells *(Baculovirus Expression Vectors*, *A Laboratory Manual,* W.H. Freeman and Company, New York (1992)), High 5 which is *Trichoplusia ni* ovary cell and the like.

The method for cotransfecting the above vector for recombinant gene introduction and the above bacurovirus for the preparation of the recombinant virus include a calcium phosphate method (Japanese Published Unexamined Patent Application No. 227075/90), a lipofection method (*Proc*. *Natl*. *Acad*. *Sci. USA,* 84, 7413 (1987)) and the like.

When a plant cell is used as the host cell, examples of the expression vector include Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

Examples of the host cell include plant cells such as tobacco, potato, tomato, carrot, soybean, rape, alfalfa, rice, wheat, and barley.

As the method for introducing the recombinant vector, any method can be used, so long as it is a method for introducing DNA into a plant cell. Examples include a method using *Agrobacterium* (Japanese Published Unexamined Patent Application No. 140885/84, Japanese Published Unexamined Patent Application No. 70080/85, WO94/00977), electroporation (Japanese Published Unexamined Patent Application No. 251887/85), a method using a particle gun (gene gun) (Japanese Patent No. 2606856, Japanese Patent No. 2517813) and the like.

The thus-obtained transformant is cultured in a medium to express a gene product encoded by the cDNA introduced. Culturing of the transformant in a medium may be carried out according to a method generally carried out in culturing a host.

As a medium for culturing the transformant obtained by using, as the host, prokaryote such as *Escherichia coli* or eukaryote such as yeast, the medium may be either a natural medium or a synthetic medium, so long as it contains a carbon source, a nitrogen source, an inorganic salt and the like which can be assimilated by the organism and the transformant can be cultured efficiently.

Any carbon source can be used, so long as it can be assimilated by the organism. Examples include carbohydrates, such as glucose, fructose, sucrose, molasses containing them, starch, and starch hydrolysate; organic acids, such as acetic acid, and propionic acid; alcohols, such as ethanol, and propanol; and the like.

Examples of the nitrogen source include ammonia; ammonium salts of inorganic acids or organic acids, such as ammonium chloride, ammonium sulfate, ammonium acetate, and ammonium phosphate, other nitrogen-containing compounds, peptone, meat extract, yeast extract, corn steep liquor, casein hydrolysate, soybean meal, soybean meal hydrolysate, various fermented cells, hydrolysates thereof and the like.

Examples of the inorganic salt include potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is generally carried out under aerobic conditions by shaking culture, deep aeration stirring culture or the like. The culturing temperature is preferably 15 to 40°C. The culturing time is generally 16 hours to 7 days. The pH is maintained at 3.0 to 9.0 during culturing. The pH is adjusted with inorganic or organic acid, an alkali solution, urea, calcium carbonate, ammonia or the like.

Also, if necessary, antibiotics such as ampicillin and tetracycline can be added to the medium during culturing.

When a microorganism transformed with an expression vector using an inducible promoter as the promoter is cultured, an inducer may be added to the medium, if necessary. For example, when a microorganism transformed with an expression vector using *lac* promoter is cultured, isopropyl-β-D-thiogalactopyranoside or the like can be added to the medium, and when a microorganism transformed with an expression vector using *trp* promoter is cultured, indoleacrylic acid or the like can be added to the medium.

Examples of the medium for culturing a transformant obtained by using an animal cell as the host include generally used RPMI 1640 medium (*The Journal of the American Medical Association,* 199, 519 (1967)), Eagle's MEM medium (*Science,* 122, 501 (1952)), Dulbecco modified MEM medium (*Virology*, 8, 396 (1959)), 199 Medium (*Proceeding of the Society for the Biological Medicine,* 73, 1 (1950)), media obtained by adding fetal calf serum or the like to these media, and the like.

Culturing is generally carried out, for example, at pH of 6 to 8 and at 30 to 40°C for 1 to 7 days in the presence of 5% CO₂.

Furthermore, if necessary, antibiotics such as kanamycin and penicillin can be added to the medium during culturing.

Examples of the medium for culturing a transformant obtained by using an insect cell as the host include generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium (Grace, T.C.C., *Nature,* 195, 788 (1962)) and the like.

Culturing is generally carried out, for example, at pH of 6 to 7 and at 25 to 30°C for 1 to 5 days.

Furthermore, if necessary, antibiotics such as gentamicin can be added to the medium during culturing.

A transformant obtained by using a plant cell as the host can be used as the cell or after differentiating to a plant cell or organ. Examples of the medium used for culturing the transformant include generally used Murashige and Skoog (MS) medium, White medium, media to which a plant hormone such as auxin or cytokinine has been added, and the like.

Culturing is generally carried out at a pH of 5 to 9 and at 20 to 40°C for 3 to 60 days.

Also, if necessary, antibiotics such as kanamycin and hygromycin can be added to the medium during culturing.

As described above, a transformant derived from a microorganism, an animal cell or a plant cell comprising a recombinant vector to which cDNA prepared from a stromal cell used in the present invention has been inserted is cultured according to the generally used culturing method to thereby produce a transformant expressing a gene product encoded by the cDNA.

In the method for inducing differentiation of the present invention, a transformant which produces a gene product having SDIA activity can be selected by coculturing an embryonic stem cell with the transformant.

Examples include enzyme immunoassay described in *Antibodies, A Laboratory Manual; Monoclonal Antibodies*; *Antibody Engineering; Enzyme Immunoassay,* Third Edition, Igaku Shoin (1987) and the like, a method using a flow cytometer described in *Antibodies, A Laboratory Manual; Monoclonal Antibodies; Antibody Engineering*; *Int. Immunol*., 10, 275 (1998); *Exp. Hematol.,* 25, 972 (1997) and the like, the Panning method described in *Monoclonal Antibodies*; *Antibody Engineering*; *J*. *Immunol*., 141, 2797 (1988) and the like.

As a method for isolating cDNA which has been introduced into the selected transformant, when an expression vector which can autonomously replicate is used in a host cell, the method includes a generally used method which recovers a phage vector or a plasmid vector described in *Molecular Cloning,* Second Edition; *Current Protocols in Molecular Biology; Mol. Cell. Biol*., **8**, 2837 (1988) and the like, and the Hirt method. When an expression vector which is integrated into a chromosome is used, cDNA which is to be introduced into a host is classified into groups of various kinds (e.g., 100 to 1000 kinds) and pooled, the group which provides the target transformant is classified into groups having few kinds (e.g., 10 to 100 kinds) and pooled, and this classification and pooling are repeated to thereby isolate the target cDNA.

The nucleotide sequence of the isolated cDNA is analyzed from its end according to a generally used nucleotide sequence analyzing method such as the dideoxy method of *Sanger et al*. (*Proc. Natl*. *Acad*. *Sci. USA*, 74, 5463 (1997)) or by using a nucleotide sequence analyzing apparatus such as ABIPRISM377DNA sequencer (manufactured by PE Biosystems) to thereby determine the nucleotide sequence of the DNA.

As described above, the factor having SDIA activity of the present invention can be obtained by using the method for expression cloning.

In addition to the method for expressing cloning, a factor having SDIA activity derived from a stromal cell can be obtained. Specifically, the factor can be purified by using the stromal cell according to the present invention as the starting material, and by using, as the index, the acceleration effect of inducing differentiation of an embryonic stem cell into an ectoderm-derived cell when it is added to the medium.

More specifically, stromal cells according to the present invention are recovered by centrifugation and suspended in an aqueous buffer, and then the cells are disrupted with an ultrasonicator, a French press, a Manton Gaulin homogenizer, a Dynomill, surfactant treatment or the like to obtain a cell-free extract. From the supernatant obtained by centrifuging the cell-free extract, a purified product can be obtained by the general method used for isolating and purifying an enzyme, for example, solvent extraction, salting out using ammonium sulfate or the like, desalting, precipitation using an organic solvent, anion exchange chromatography using a resin, such as diethylaminoethyl (DEAE)-Sepharose or DIAION HPA-75 (manufactured by Mitsubishi Chemical), cation exchange chromatography using a resin, such as S-Sepharose FF (manufactured by Pharmacia), hydrophobic chromatography using a resin, such as butyl sepharose or phenyl sepharose, gel filtration using a molecular sieve, affinity chromatography, chromatofocusing, or electrophoresis, such as isoelectronic focusing, alone or in combination thereof.

In addition, since the stromal cell-derived factor has a property of adsorbing the above-described polyanionic compound, the factor can be obtained from the stromal cell-derived factor absorbed on a polyanionic compound, after allowing the factor in a culture system in which the stromal cell is cultured, or in a culture system in which an embryonic stem cell is cultured under non-aggregation conditions in the presence of the stromal cell, to absorb on the polyanionic compound. For example, the stromal cell-derived factor can be obtained by absorbing the factor to heparin using column chromatography with heparin as the carrier, eluting the bound factor, and using the above-described effect of inducing differentiation as an index.

### 7. Method for producing a factor having SDIA activity

The factor derived from a stromal cell having SDIA activity of the present invention includes a polypeptide comprising the amino acid sequence represented by SEQ ID NO:7 or 8, and the like. Furthermore, it includes a polypeptide which consists of an amino acid sequence in which one or more amino acid(s) is/are deleted, substituted and/or added in the amino acid sequence represented by SEQ ID NO:7 or 8 and has activity to induce differentiation of an embryonic stem cell into an ectodermal cell or an ectoderm-derived cell, and the like.

The polypeptide which consists of an amino acid sequence in which one or more amino acid(s) is/are deleted, substituted, inserted and/or added in the amino acid sequence represented by SEQ ID NO:7 or 8 and has activity to induce differentiation of an embryonic stem cell into an ectodermal cell or an ectoderm-derived cell can be obtained by using a method for introducing part-specific site-directed mutagenesis described in, for example, *Molecular Cloning,* Second Edition; *Current Protocols in Molecular Biology; Nucleic Acids. Research,* 10, 6487 (1982); *Proc. Natl*. *Acad*. *Sci. USA,* 79, 6409 (1982); *Gene,* 34, 315 (1985); *Nucleic Acids. Research,* 13, 4431 (1985); *Proc. Natl*. *Acad*. *Sci. USA,* 82, 488 (1985); and the like. For example, the polypeptide can be obtained by introducing site-directed mutation(s) to a DNA encoding a polypeptide comprising the amino acid sequence represented by SEQ ID NO:7 or 8.

The number of the amino acids which are deleted, substituted, inserted and/or added is not particularly limited; however, it is such a number that deletion, substitution, insertion or addition can be carried out by a known method such as method for introducing part-specific site-directed mutation(s). The number is 1 to several tens, preferably 1 to the order of tens, preferably 1 to 20, more preferably 1 to 10, and most preferably 1 to 5.

The deletion, substitution, insertion or addition of one or more amino acid residue(s) in the amino acid sequence represented by SEQ ID NO:7 or 8 means that one or two or more amino acid(s) is/are deleted, substituted, inserted and/or added at any position in the same sequence. The deletion, substitution, insertion or addition can be carried out in the same amino acid sequence simultaneously. Also, the amino acid residue deleted, substituted, inserted or added can be natural or non-natural. The natural amino acid residue includes L-alanine, L-asparagine, L-asparatic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like.

Herein, examples of amino acid residues which are substituted with each other are shown below. The amino acid residues in the same group can readily be substituted with each other.
Group A:
   leucine, isoleucine, norleucine, valine, norvaline, alanine, 2-aminobutanoic acid, methionine, *O*-methylserine, t-butylglycine, t-butylalanine, cyclohexylalanine;
Group B:
   asparatic acid, glutamic acid, isoasparatic acid, isoglutamic acid, 2-aminoadipic acid, 2-aminosuberic acid;
Group C:
   asparagine, glutamine;
Group D:
   lysine, arginine, ornithine, 2,4-diaminobutanoic acid, 2,3-diaminopropionic acid;
Group E:
   proline, 3-hydroxyproline, 4-hydroxyproline;
Group F:
   serine, threonine, homoserine;
Group G:
   phenylalanine, tyrosine.

Also, in order that the protein which consists of an amino acid sequence in which one or more amino acid(s) is/are deleted, substituted, inserted and/or added in the amino acid sequence represented by SEQ ID NO:7 or 8 is a polypeptide having activity to induce differentiation of an embryonic stem cell into an ectodermal cell or an ectoderm-derived cell, it has a homology of 60% or more, generally 80% or more, preferably 95% or more, and more preferably 98% or more, with the amino acid sequence represented by SEQ ID NO:7 or 8.

The homology of an amino acid sequence can be determined by using the algorithm BLAST by Karlin and Altschul [*Proc*. *Natl*. *Acad Sci. USA*, 90, 5873 (1993)] or PASTA *[Methods Enzymol*., 183, 63 (1990)]. When programs based on the algorithms are used, a default parameter of each program can be used. The specific analysis methods of this analysis are known (http://www.ncbi.nlm.nih.gov.).

The polypeptide of the present invention can be produced by using the DNA encoding the protein. The DNA can be obtained by carrying out a method such as colony hybridization or plaque hybridization (*Molecular Cloning,* Second Edition) for cDNA library prepared by cells, tissues or the like which produce the protein, by using the DNA encoding the amino acid sequence.

By using the amino acid sequence as the query, the nucleotide sequence of a DNA encoding a protein having the amino acid sequence can be obtained by searching a known data base.

The DNA obtained by the above methods include:
(i) a DNA comprising the nucleotide sequence represented by SEQ ID NO:9 or 10,
(ii) a DNA encoding a protein consisting of an amino acid sequence in which one or more amino acid residue(s) is/are deleted, substituted, inserted and/or added in the amino acid sequence represented by SEQ ID NO:7 or 8, and
(iii) a DNA encoding a protein which is hybridizable with a DNA comprising the nucleotide sequence represented by SEQ ID NO:9 or 10 under stringent conditions and has activity of inducing differentiation of an embryonic stem cell into an ectodermal cell or an ectoderm-derived cell.

The DNA which is hybridizable under stringent conditions is a DNA obtained by colony hybridization, plaque hybridization, Southern blot hybridization or the like using, as a probe, a part or a full length of the DNA comprising the nucleotide sequence represented by SEQ ID NO:9 or 10. Specifically, the DNA includes a DNA which can be identified by carrying out hybridization at 65°C in the presence of 0.7-1.0 mol/l NaCl using a filter on which a DNA derived from colonies or plaques is immobilized, and then washing the filter with 0.1× to 2× SSC solution (the composition of 1× SSC solution contains 150 mmol/l sodium chloride and 15 mmol/l sodium citrate) at 65°C. The hybridization can be carried out in accordance with a known method described in, for example, *Molecular Cloning*, Second Edition; *Current Protocols in Molecular Biology*; *DNA Cloning I*: *Core Techniques*, *A Practical Approach,* Second Edition, Oxford University (1995) or the like. Specifically, the DNA which is hybridizable includes a DNA having a homology of at least 60% or more, preferably 80% or more, and more preferably 95% or more, with the nucleotide sequence represented by SEQ ID NO:9 or 10 when calculated by using the above BLAST (*J*. *Mol*. *Biol*., 215, 403 (1990)) *FASTA (Methods in Enzymology*, 183, 63-98 (1990)) or the like.

The polypeptide used in the present invention can be produced by expressing the DNA of the present invention described in the above item 6 in a host cell according to the method described in *Molecular Cloning,* Second Edition, *Current Protocols in Molecular Biology,* or the like. Furthermore, as the method for expressing the gene, secretory production, fusion protein expression or the like can be carried out according to the method described in *Molecular Cloning*, Second Edition in addition to the direct expression.

When expressed in yeast, an animal cell, an insect cell or a plant cell, a glycosylated or sugar chain-added polypeptide can be obtained.

Thus, the polypeptide can be produced by culturing a transformant derived from a microorganism, an animal cell or a plant cell containing a recombinant vector to which a cDNA prepared from the stromal cell of the present invention or a DNA encoding the polypeptide used in the present invention is inserted according to the general culturing method to produce and accumulate the polypeptide, and recovering the polypeptide from the culture.

The method for producing the polypeptide used in the present invention includes a method of intracellular expression in a host cell, a method of extracellular secretion from a host cell, or a method of production on a host cell membrane outer envelope. The method can be selected by changing the host cell used or the structure of the polypeptide produced.

When the polypeptide used in the present invention is produced in a host cell or on a host cell membrane outer envelope, the polypeptide can be positively secreted extracellularly, for example, according to the method of Paulson *et al*. (*J*. *Biol*. *Chem*., 264, 17619 (1989)), the method of Lowe *et al*. (*Proc. Natl*. *Acad Sci. USA*, 86, 8227 (1989); *Genes Develop.,* 4, 1288 (1990)), or the method described in Japanese Published Unexamined Patent Application No. 336963/93, WO 94/23021, or the like.

Specifically, the polypeptide used in the present invention can be positively secreted extracellularly by expressing it in the form that a signal peptide has been added to the foreground of a polypeptide containing an active site of the polypeptide used in the present invention according to the genetic engineering technique.

Furthermore, the amount produced can be increased by using a gene amplification system, for example, by using a dihydrofolate reductase gene or the like according to the method described in Japanese Published Unexamined Patent Application No. 227075/90.

Moreover, by redifferentiating an animal or plant cell to which the gene is introduced, a transgenic animal individual (transgenic non-human animal) or plant individual (transgenic plant) to which the gene is introduced is developed, and the polypeptide used in the present invention can be produced by using these individuals.

When the transformant is the animal individual or plant individual, the polypeptide can be produced by breeding or cultivating it to produce and accumulate the polypeptide, and recovering the polypeptide from the animal individual or plant individual according to a general method.

Examples of the method for producing the polypeptide used in the present invention by using the animal individual include a method for producing the polypeptide used in the present invention in an animal developed by inserting a gene according to a known method (*American Journal of Clinical Nutrition*, 63, 639S (1996), *American Journal of Clinical Nutrition*, 63, 627S (1996), *Bio*/*Technology*, 9, 830 (1991)).

In the animal individual, the polypeptide can be produced by breeding a transgenic non-human animal to which the DNA encoding the polypeptide used in the present invention is inserted to produce and accumulate the polypeptide in the animal, and recovering the polypeptide from the animal. Examples of the production and accumulation place in the animal include milk (Japanese Published Unexamined Patent Application No. 309192/88), egg and the like in the animal. Any promoter can be used, so long as it can be expressed in the animal. Examples include an α-casein promoter, a β-casein promoter, a β-lactoglobulin promoter, a whey acidic protein promoter, and the like, which are specific for mammary glandular cells.

Examples of the method for producing the polypeptide used in the present invention by using the plant individual include a method for producing the polypeptide used in the present invention by cultivating a transgenic plant to which the DNA encoding the protein used in the present invention is introduced by a known method (*Tissue Culture, 20* (1994), *Tissue Culture, 21* (1995), *Trends in Biotechnology,* 15, 45 (1997)) to produce and accumulate the polypeptide in the plant, and recovering the polypeptide from the plant.

The polypeptide produced by the transformant of the present invention can be isolated and purified by using the general method for isolating and purifying an enzyme. For example, when the polypeptide of the present invention is expressed as a soluble product in the host cells, the cells are collected by centrifugation after cultivation, suspended in an aqueous buffer, and disrupted with an ultrasonicator, a French press, a Manton Gaulin homogenizer, a Dynomill, or the like to obtain a cell-free extract. From the supernatant obtained by centrifuging the cell-free extract, a purified product can be obtained by the general method used for isolating and purifying an enzyme, for example, solvent extraction, salting out using ammonium sulfate or the like, desalting, precipitation using an organic solvent, anion exchange chromatography using a resin, such as diethylaminoethyl (DEAE)-Sepharose or DIAION HPA-75 (manufactured by Mitsubishi Chemical), cation exchange chromatography using a resin, such as S-Sepharose FF (manufactured by Pharmacia), hydrophobic chromatography using a resin, such as butyl sepharose or phenyl sepharose, gel filtration using a molecular sieve, affinity chromatography, chromatofocusing, or electrophoresis, such as isoelectronic focusing, alone or in combination thereof.

When the polypeptide is expressed as an insoluble product in the host cells, the cells are collected in the same manner, disrupted and centrifuged to recover the insoluble product of the polypeptide as the precipitate fraction. The recovered insoluble product of the polypeptide is solubilized with a protein denaturing agent. The solubilized solution is diluted or dialyzed to lower the concentration of the protein denaturing agent in the solution to reconstitute the normal configuration of the polypeptide. After the procedure, a purified product of the polypeptide can be obtained by a purification and isolation method similar to the above.

When the polypeptide used in the present invention or its derivative, such as a polypeptide formed by adding a sugar chain thereto, is secreted out of cells, the polypeptide or its derivative can be collected in the culture supernatant. That is, the culture supernatant is obtained by treating the culture medium in a treatment, such as centrifugation, similar to the above. Then, a purified product can be obtained from the culture medium by using a purification and isolation method similar to the above.

Furthermore, the polypeptide used in the present invention can be produced by a chemical synthesis method, such as Fmoc (fluorenylmethyloxycarbonyl) method or tBoc (t-butyloxycarbonyl) method. It can also be synthesized by using a peptide synthesizer manufactured by Advanced ChemTech, Perkin-Elmer, Pharmacia, Protein Technology Instrument, Synthecell-Vega, PerSeptive, Shimadzu Corporation, or the like.

### 8. Medicament comprising the factor having SDIA activity of the present invention

The agent for inducing differentiation comprising a factor which induces differentiation of an embryonic stem cell into an ectodermal cell or ectoderm-derived cell or the factor described in the following item 9(1) of the present invention can be used as a therapeutic agent for diseases caused by the disorder of ectoderm-derived cells.

Examples of the disease cause by the disorder of ectoderm-derived cells include diseases caused by the disorder of nervous system cells or epidermal system cells.

Examples of the disease caused by the disorder of nervous system cells include Alzheimer disease, Huntington chorea, Parkinson disease, ischemic cerebral disease, epilepsy, brain injury, vertebral injury, motor neuron disease, neurodegeneration disease, pigmentary retinal dystrophy, cochlear hearing loss, multiple sclerosis, amyotrophic lateral sclerosis, a disease due to a neurotoxin damage and the like. Examples of the disease caused by the disorder of epidermal system cells include bum, wound, healing of wound, compression gangrene, psoriasis and the like.

The medicament comprising the factor having SDIA activity as an active ingredient can be administered by the active ingredient alone, but generally, it is preferable to provide the active ingredient as a pharmaceutical production produced by an optional method well known in the technical field of manufacturing pharmacy, by mixing it with one or more pharmaceutically acceptable carriers. Preferably, a sterile solution produced by dissolving it in an aqueous carrier such as water or an aqueous solution of sodium chloride, glycine, glucose, human albumin or the like is used. Also, pharmaceutically acceptable additives including a buffering agent and a tonicity agent for use in resembling the pharmaceutical production solution to physiological conditions, such as sodium acetate, sodium chloride, sodium lactate, potassium chloride, or sodium citrate, can be added. Also, it is possible to store the production by freeze-drying and use it by dissolving in an appropriate solvent when used.

It is preferable to use a route of administration which is most effective in carrying out a treatment. Examples include oral administration and parenteral administration such as buccal, airway, rectal, subcutaneous, intramuscular, and intravenous administration. Examples of the dosage form include sprays, capsules, tablets, granules, syrups, emulsions, suppositories, injections, ointments, tapes and the like.

Examples of the pharmaceutical production suitable for oral administration include emulsions, syrups, capsules, tablets, powders, granules and the like. For example, liquid productions such as emulsions and syrups can be produced by using, as additives, water, saccharides such as sucrose, sorbitol, and fructose; glycols such as polyethylene glycol and propylene glycol; oils such as sesame oil, olive oil, and soybean oil; antiseptics such as p-hydroxybenzoic acid esters; flavors such as strawberry flavor and peppermint flavor; and the like. Capsules, tablets, powders and granules can be produced by using, as additives, fillers such as lactose, glucose, sucrose, and mannitol; disintegrating agents such as starch sodium alginate; lubricants such as magnesium stearate and talc; binders such as polyvinyl alcohol, hydroxypropylcellulose, and gelatin; surfactants such as a fatty acid ester; plasticizers such as glycerol; and the like.

Examples of the pharmaceutical production suitable for parenteral administration include injections, suppositories, sprays and the like. For example, injections are produced by using a carrier such as a salt solution, a glucose solution, or a mixture thereof. Suppositories are produced by using a carrier such as cacao butter, hydrogenated fat, or carboxylic acid. Also, sprays are produced by using the active ingredient as such or using a carrier which does not stimulate the buccal or airway mucous membrane of the patient and can facilitate absorption of the active ingredient by dispersing it as fine particles. Specific examples of the carrier include lactose, glycerine and the like. Depending on the properties of the active ingredient and the carrier, it is possible to produce other pharmaceutical productions such as aerosols and dry powders. In addition, the components exemplified as additives for oral productions can also be added to these parenteral productions.

Although the clinical dose or the frequency of administration varies depending on various conditions such as the intended therapeutic effect, administration method, treating period, age and body weight, it is usually from 10 µg/kg to 8 mg/kg per day per adult.

### 9. Agent for inducing differentiation of the present invention and its application

### (1) Agent for inducing differentiation of the present invention

The agent for inducing differentiation of the present invention is not particularly limited, so long as it comprises, as an active ingredient, the solution obtained by the method described in the above item 4, the factor described in the above item 6 or 7, a DNA encoding a polypeptide constituting the factor, a recombinant vector comprising the DNA, a transformant obtained by introducing the vector into a stromal cell, or Wnt antagonist.

The solution obtained by the method described in the above item 4 includes a medium in which differentiation of an embryonic stem cell can be induced, a solution having SDIA activity, and the like.

The factor is not particularly limited, so long as it is a substance having SDIA activity described in the above item 6 or 7. Examples include a polypeptide comprising the amino acid sequence represented by SEQ ID NO:7 or 8, a polypeptide consisting of an amino acid sequence in which one or more amino acid residue(s) is/are deleted, substituted, inserted and/or added in the amino acid sequence represented by SEQ ID NO:7 or 8; a polypeptide consisting of an amino acid sequence having a homology of 60% or more with the amino acid sequence represented by SEQ ID NO:7 or 8.

The DNA encoding the polypeptide contained in the factor includes a DNA encoding the amino acid sequence represented by SEQ ID NO:7 or 8 and a DNA encoding the nucleotide sequence represented by SEQ NO:9 or 10 described above, and the like.

The recombinant vector comprising the DNA is not particularly limited, so long as it can be introduced into a human cell, preferably a stromal cell.

The transformant obtained by introducing the above-described recombinant vector into a stromal cell is not particularly limited, so long as it can be used for the method for inducing differentiation of the present invention.

The Wnt antagonist is not particularly limited, so long as it is a substance which inhibits the binding of Wnt to the Wnt antagonist, and examples include SFRP and the like.

The agent for inducing differentiation of an ectodermal cell to an epidermal system cell preferably contains BMP4.

### (2) Application of agent for inducing differentiation of the present invention

The agent for inducing differentiation of the present invention can be applied as a therapeutic agent for diseases caused by the disorder of ectoderm-derived cells as described in the above item 8. Further, it can be applied to the method for inducing differentiation of an embryonic stem cell into an ectodermal cell and an ectoderm-derived cell as described in the above item 1(7).

### 10. Applications of method for inducing differentiation, cell and agent for inducing differentiation of the present invention

### (l) Method for evaluating or screening substance using the method for inducing differentiation of the present invention

The culturing method of the present invention for inducing differentiation of an embryonic stem cell into an ectodermal cell or ectoderm-derived cell is useful for the pharmacological evaluation and activity evaluation of substances such as a physiologically active substance (for example, a drug) and a novel gene product whose functions are unknown, in the differentiation process of these cells or in the cell function regulation. It is also useful for the function evaluation of a gene in the step of an embryonic stem cell into an ectodermal cell or ectoderm-derived cell, by using an embryonic stem cell in which the specified gene is modified.

For example, the application method of the culturing method of the present invention are shown below.

According to the method for inducing differentiation of the present invention, influences upon the differentiation process into an ectodermal cell or ectoderm-derived cell or upon the functional regulation of an ectodermal cell or an ectoderm-derived cell of a test substance added to the medium can be evaluated. Any substance can be used as the substance to be tested, so long as it can be added to the culturing system. Examples include a low molecular weight compound, a high molecular weight compound, an organic compound, an inorganic compound, a protein, a gene, a virus, a cell and the like. The substances to be tested, excluding genes, may be added directly to the culture medium.

Examples of the method for efficiently introducing a gene into the culture system include a method in which the gene is added to the culture system by carrying it on a virus vector such as retrovirus, adenovirus, adeno-associated virus, herpes simplex virus or lentivirus and a method in which it is added to the culture system by including into an artificial vesicle structure such as liposome. Specific examples include reports on the analysis of genes using recombinant virus vectors (*Proc. Natl*. *Acad Sci. USA,* 92, 6733 (1995); *Nucleic Acids Res*., 18, 3587 (1990); *Nucleic Acids Res.,* 23, 3816 (1995)).

These substances to be tested can be added to the culture system in the differentiation induction method at any stage, for example, each substance to be tested can be evaluated by adding it at a relatively early stage of the culturing when it is necessary to evaluate its action on a differentiation step of the stem cell into an ectodermal cell, or at a relatively latter stage of culturing when it is necessary to evaluate its action on a differentiation step of an ectodermal cell into an ectoderm-derived cell. In order to judge the differentiating degree in the culture system, it can be understood by examining expression of a marker protein of respective differentiated cell formed as a result of its differentiation from the embryonic stem cell. The evaluation or screening of a substance to be tested can be carried out, e.g., by measuring qualitative or quantitative changes in the differentiation efficiency into an ectodermal cell or ectoderm-derived cell after a predetermined period of culturing. Examples of the method for measuring qualitative changes include a method in which van Inzen *et al*. have measured the action potential using a nerve cell differentiation-induced from an embryonic stem cell *(Biochim. Biophys. Acta.,* 1312, 21 (1996)).

### (2) Medicament containing the cell of the present invention

The ectodermal cell or an ectoderm-derived cell of the present invention which is obtained by carrying out differentiation induction of an embryonic cell can be used as a therapeutic agent for treating diseases caused by the disorder of ectoderm-derived cells.

Examples of the disease cause by the disorder of ectoderm-derived cells include diseases caused by the disorder of nervous system cells or epidermal system cells.

Examples of the disease caused by the disorder of nervous system cells include Alzheimer disease, Huntington chorea, Parkinson disease, ischemic cerebral disease, epilepsy, brain injury, vertebral injury, motor neuron disease, neurodegeneration disease, pigmentary retinal dystrophy, cochlear hearing loss, Down's syndrome, multiple sclerosis, amyotrophic lateral sclerosis, a disease due to a neurotoxin damage and the like. Examples of the disease caused by the disorder of epidermal system cells include burn, wound, healing of wound, compression gangrene, psoriasis and the like.

Examples of the therapeutic agent for diseases caused by the disorder of ectoderm-derived cells include a cell having the same function of the cell which caused a disorder, a precursor of the cell which caused a disorder, a cell which can compensate function of the disordered cell or a cell having a function to accelerate regeneration of the disordered cell, which can be applied to the transplantation medical treatment.

The therapeutic agent of the present invention can be produced by purifying an ectodermal cell or an ectoderm-derived cell obtained by its differentiation induction from an embryonic stem cell according to the method of the present invention.

Since the therapeutic agent of the present invention is used for the purpose of carrying out a transplantation medical treatment, it is required to avoid contamination of impurities such as sera and viruses. According to the method of the present invention, differentiation of an ectodermal cell and an ectoderm-derived cell can be induced under serum-free culture conditions and without requiring an agent for inducing differentiation such as retinoic acid at a non-physiological concentration, so that it is suitable for objects of transplantation medical treatments.

Any one of the already known methods for separating and purifying cells can be used as the method for purifying cells. Examples include a method using a flow cytometer described in, for example, *Antibodies, A Laboratory Manual,* Cold Spring Harbor Laboratory, Chapter 14 (1998), *Monoclonal Antibodies: principles and practice,* Third Edition, Acad. Press (1993), *Antibody Engineering, A Practical Approach,* IRL Press at Oxford University Press (1996), *Int. Immunol*., 10, 275 (1998), *Exp*. *Hematol*., 25, 972 (1997) or the like, a panning method described in, for example, *Monoclonal Antibodies: principles and practice,* Third Edition, Acad. Press (1993), *Antibody Engineering, A Practical Approach,* IRL Press at Oxford University Press (1996), *J*. *Immunol.,* 141, 2797 (1988) or the like, and a cell fractionation method using density difference of sucrose concentration described in, for example, *Techniques of Tissue Culture* (Third Edition), Asakura Shoten (1996).

The method for increasing purity of differentiaged cell according to the present invention, comprises a step of culturing the ectodermal cell or ectoderm-derived cell obtained by differentiation-inducing the embryonic stem cell as described above, in a medium comprising an antitumor agent. Since cells under an undifferentiated state can be removed by this step, differentiated cells can be obtained with further higher purity, so that the product becomes more suitable as a medicament. That is, by the treatment with an antitumor agent, cells other than the target differentiated cell, such as undifferentiated cells, can be removed. It is considered that such undifferentiated cells will become a cause of teratoma, but the danger can be avoided by treating with an antitumor agent.

Examples of the antitumor agent include mitomycin C, 5-fluorouracil, adriamycin, ara-C, methotrexate and the like. It is preferable to use these antitumor agents at a concentration which shows stronger cytotoxicity on undifferentiated cells than that on differentiated cells. For example, it is preferably at a concentration of 1/100 to 1 equivalent of the concentration in these antitumor agents are used in the living body described in *The Pharmacopoeia of Japan*.

The step of culturing the ectodermal cell or ectoderm-derived cell obtained by differentiation-inducing the embryonic stem cell, in a medium comprising an antitumor agent includes a method in which an antitumor agent at an appropriate concentration (for example, 1 to 100 µg/ml, preferably 10 µg/ml, mitomycin C) is added to a culturing system in which its medium was changed on the previous day, and culturing is carried out at 37°C for several hours, preferably 2 to 3 hours, in a stream of 5% carbon dioxide in a CO₂ incubator

Any medium may be used in this method, so long as it is capable of culturing differentiation-induced cells. The medium described in the above item 2 can be exemplified.

The therapeutic agent of the present invention may further contain pharmaceutically acceptable physiological saline, additives and/or a medium, in addition to the cells (including an ectodermal cell or ectoderm-derived cell), but since it is used for the purpose of carrying out a transplantation medical treatment, it is preferable to avoid contamination of impurities such as sera and viruses. According to the present invention, differentiation of an ectodermal cell and an ectoderm-derived cell can be induced from an embryonic stem cell under serum-free culture conditions and without requiring an agent for inducing differentiation such as retinoic acid at a non-physiological concentration, so that it is useful in transplantation medical treatments.

In the transplantation medical treatment, rejection due to difference in the histocompatibility antigens sometimes causes a problem, but this problem can be resolved by using the embryonic stem cell described in the above item 3(2) into which the nucleus of a somatic cell has been transplanted or the embryonic stem cell described in the above item 3(3) in which a gene on the chromosome has been modified.

Also, an ectodermal cell and an ectoderm-derived cell of a somatic cell-donated individual can be obtained by carrying out differentiation induction using the embryonic stem cell described in the above item 3(2) into which the nucleus of a somatic cell has been transplanted. Such a cell of individual person is useful not only as a transplantation medical treatment of the cell itself but also as a diagnosing material for judging whether or not an existing agent is effective for the person. Also, since sensitivities to oxidation stress and aging can be judged by culturing a differentiation-induced cell for a prolonged period of time, risk of individual person for a disease such as a nerve degeneration disease can be evaluated by comparing its function and life with those of a cell derived from other individual, and the evaluation data are useful for providing an effective method for preventing a disease which is diagnosed as high in its future morbidity rate.

As the transplantation method, any method can be used, so long as it is a method suitable for the disease to be treated, and methods suitable for respective diseases are known. For example, an embryonic stem cell is collected from a patient, and the disease can be treated by inducing differentiation of an ectodermal cell or an ectoderm-derived cell from the embryonic stem cell and then transplanting the resulting cell into the affected part of the patient. Examples of a method for transplanting a brain cell of an abortion fetus into a patient of Parkinson disease include the method described in, e.g., *Nature Neuroscience,* 2, 1137 (1999) and the like.

The present invention is described more specifically based on the following examples, but these examples show only illustrations of the present invention and do not limit the scope of the present invention.

### BRIEF DESCRIPTION OF THE DRAWINGS

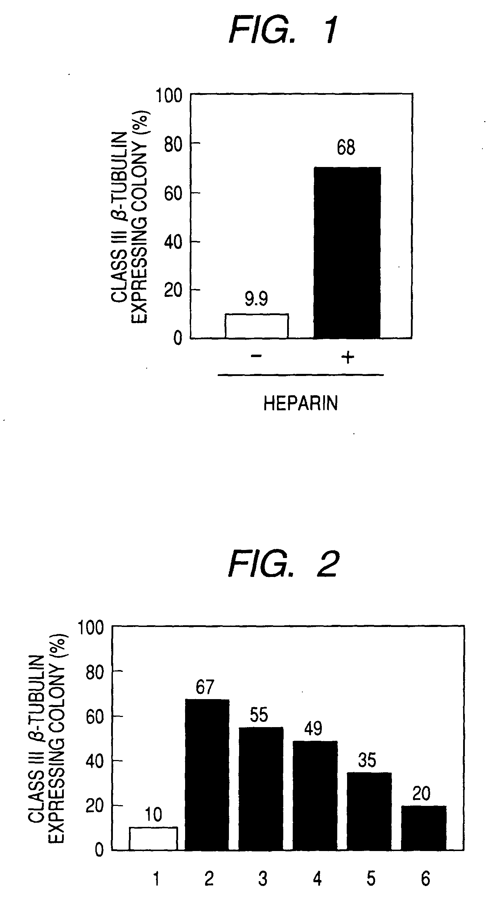

Fig. 1 is a graph showing a result of the recovery of SDIA activity by a heparin solution. The ordinate shows influence of a solution recovered from PA6 cell by Hanks' balanced salt solution containing heparin (0.001%) or Hanks' balanced salt solution which does not contain heparin upon nerve differentiation of ES cell by the ratio of colonies expressing a nerve marker class III β-tubulin. In this case, optionally selected 100 or more colonies were used in the calculation.
Fig. 2 is a graph showing a result of the recovery of SDIA activity by various poly-anion solutions. The ordinate shows influence of a solution recovered from PA6 cell using 1) Hanks' balanced salt solution which does not contain poly-anion, 2) 0.001% (w/v) dextran sulfate, 3) 0.001% (w/v) polyvinyl sulfate, 4) 0.001% (w/v) polystyrene sulfonate, 5) 0.1% (w/v) carboxymethylcellulose or 6) Hanks' balanced salt solution containing 0.1% (w/v) hyaluronic upon nerve differentiation of ES cell by the ratio of colonies expressing a nerve marker class III β-tubulin. In this case, optionally selected 100 or more colonies were used in the calculation.

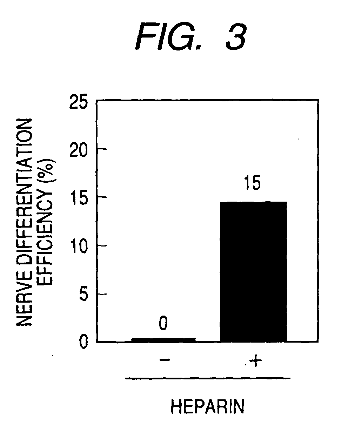

Fig. 3 is a graph showing a result of the fixation of a solution containing SDIA activity on the surface of a culture vessel. The ordinate shows nerve differentiation efficiency of ES cell on a culture vessel in which a solution recovered from PA6 cell by Hanks' balanced salt solution containing heparin (0.001%) or Hanks' balanced salt solution which does not contain heparin was fixed on the surface by the ratio of colonies having nerve processes. In this case, optionally selected 10 or more colonies were used in the calculation.

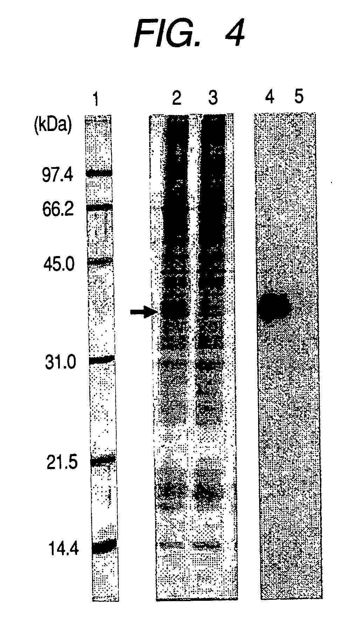

Fig. 4 is a drawing showing a result of the analysis of a solution containing SDIA activity and a solution which does not contain SDIA activity, carried out by an electrophoresis. Lane 1 is a molecular weight marker, and molecular weights of respective proteins are shown next thereto. Lane 2 and lane 3 are SYPRO-Ruby staining images of a solution containing SDIA activity and a solution which does not contain SDIA activity, respectively, and the arrow shown on the left side of lane 2 indicates the position of SFRP 1. Lane 4 and lane 5 are anti-SFRP 1 antibody Western blotting images of a solution containing SDIA activity and a solution which does not contain SDIA activity, respectively.

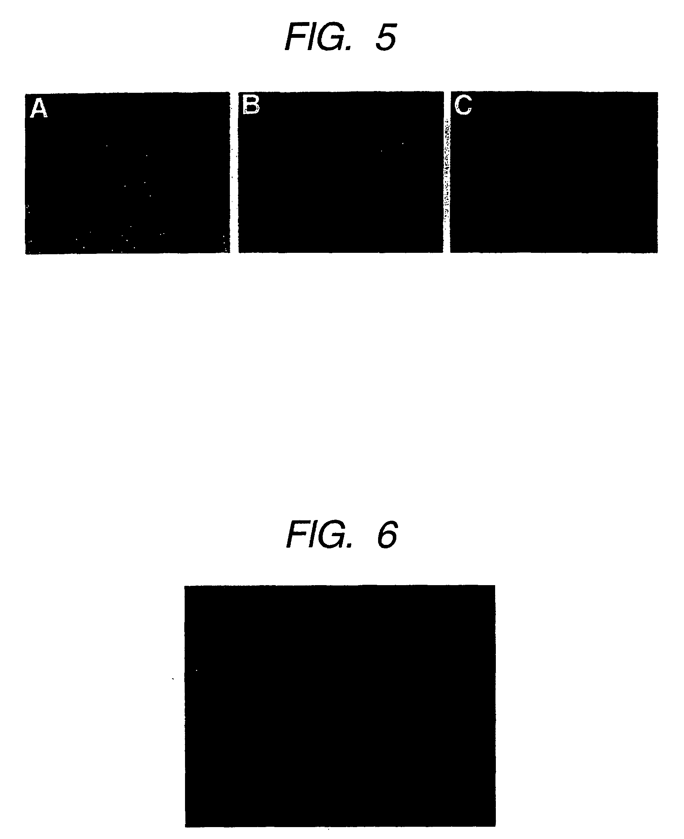

Fig. 5 is a microphotograph showing a result in which colonies formed by coculturing ES cell EB5 with PA6 cell are stained with antibodies against (A) NCAM, (B) tubulin and (C) nestin.
Fig. 6 is a microphotograph showing a result in which colonies formed by coculturing ES cell EB5 with MEF cell are stained with anti-NCAM antibody.

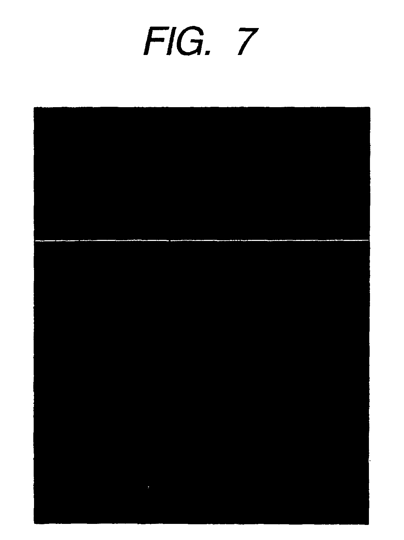

Fig. 7 is a microphotograph showing a result in which colonies formed by coculturing ES cell EB5 with PA6 cell are stained with an antibody against tyrosine hydroxylase.

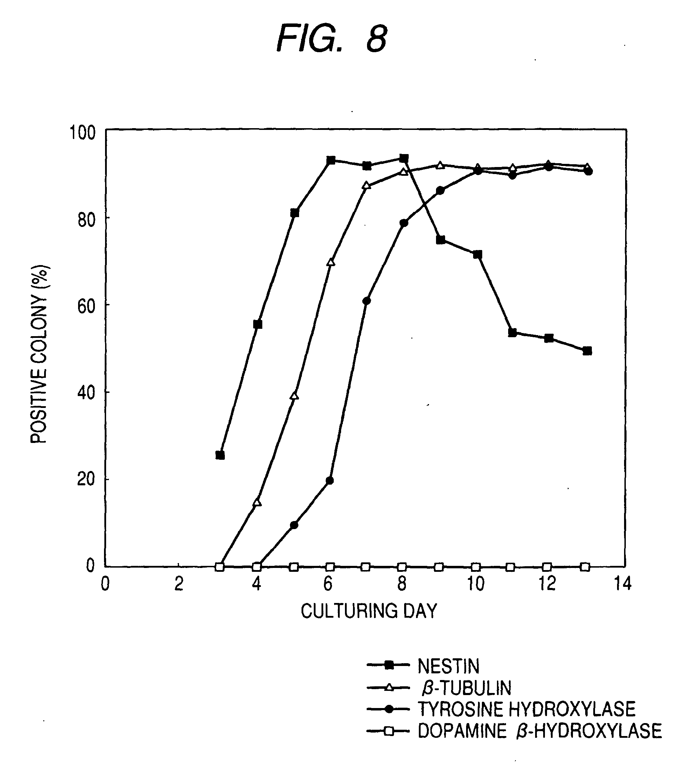

Fig. 8 is a graph showing periodical changes in the ratio of various marker positive colonies among colonies formed by coculturing ES cell EB5 with PA6 cell.

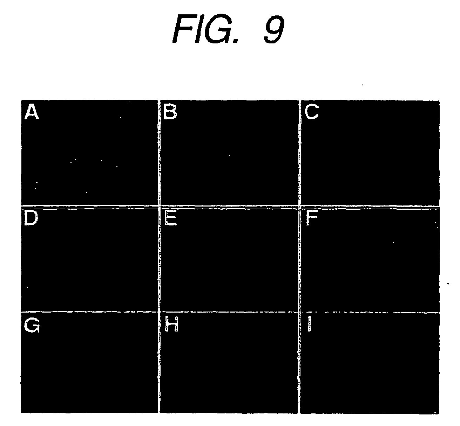

Fig. 9 is a microphotograph showing a result in which colonies formed by coculturing ES cell EB5 with PA6 cell in the absence of BMP4 are stained with (A) an antibody against NCAM, (B) an antibody against nestin, (C) an antibody against E cadherin and (G) an antibody against keratin 14, and a result in which colonies formed by coculturing ES cell with PA6 cell in the presence of BMP4 are stained with (D) an antibody against NCAM, (E) an antibody against nestin, (F) an antibody against E cadherin and (H, I) an antibody against keratin 14.

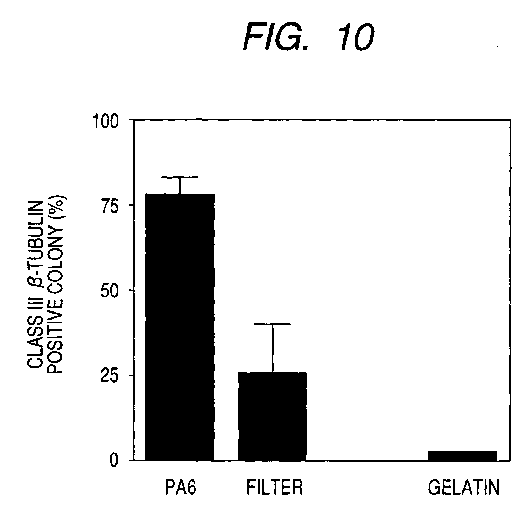

Fig. 10 is a graph showing a result in which colonies formed by coculturing ES cell EB5 with PA6 cell via a filter (filter) or not via a filter (PA6) or colonies formed by culturing ES cell EB5 on gelatin without PA6 cell (gelatin) are stained with an antibody against tubulin.

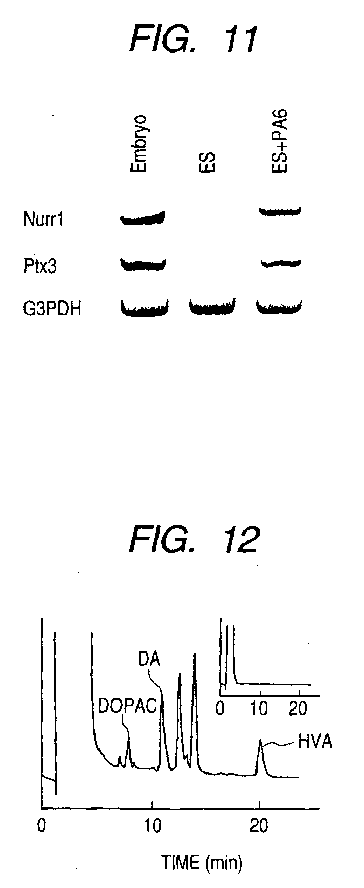

Fig. 11 is a graph showing a result of RT-PCR analysis of the expressed quantity of Nurr1, Ptx3 and G3PDH in differentiated cells formed by coculturing ES cell EB5 with PA6 cell. It shows a result of the analysis by agarose electrophoresis after carrying out RT-PCR using a cell of the head of a mouse of 12 days of fetal age (shown as Embryo in the drawing), ES cell EB5 cocultured with PA6 cell for 12 days (shown as ES + PA6 in the drawing) and a control ES cell EB5 cultured for 12 days (shown as ES in the drawing) as materials.
Fig. 12 is a chromatogram showing a result of HPLC analysis of components secreted into the medium caused by the stimulation of differentiated cells formed by coculturing ES cell EB5 with PA6 cell. As a control, a result of the analysis of components secreted by the same stimulation of PA6 cell alone used as a feeder cell is shown in the upper right-side chromatogram.

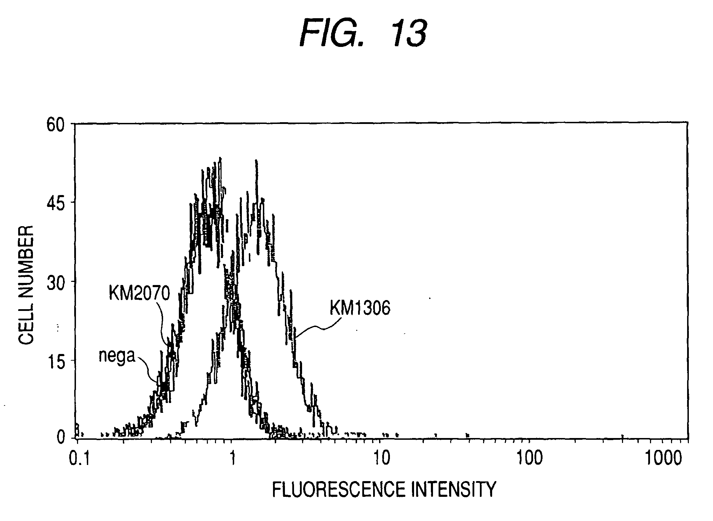

Fig. 13 is a graph showing a result on the reactivity of a monoclonal antibody KM1306 with PA6 cell, analyzed by fluorescent antibody technique using a cell sorter. As a control, a result of the same analysis carried out by using a rat IgM monoclonal antibody KM2070 whose species and subclass coincided with the antibody is shown. The number of cells is shown as the ordinate and the fluorescence intensity is shown as the abscissa.

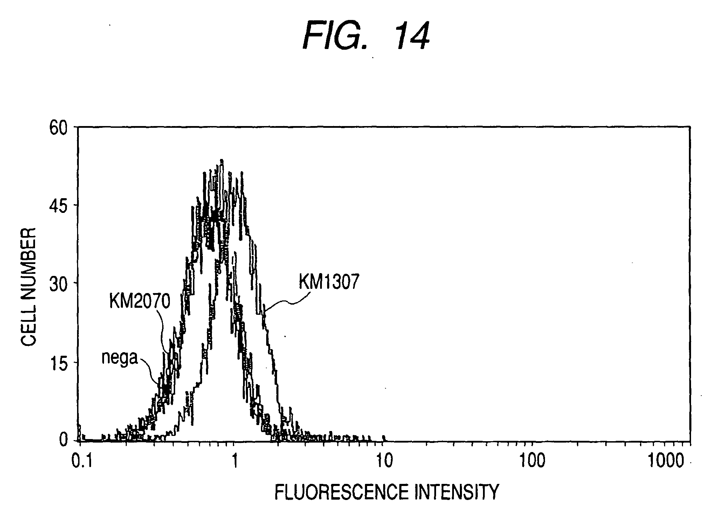

Fig. 14 is a graph showing a result on the reactivity of a monoclonal antibody KM1307 with PA6 cell, analyzed by fluorescent antibody technique using a cell sorter. As a control, a result of the same analysis carried out by using a rat IgM monoclonal antibody KM2070 whose species and subclass coincided with the antibody is shown. The number of cells is shown as the ordinate and the fluorescence intensity is shown as the abscissa.

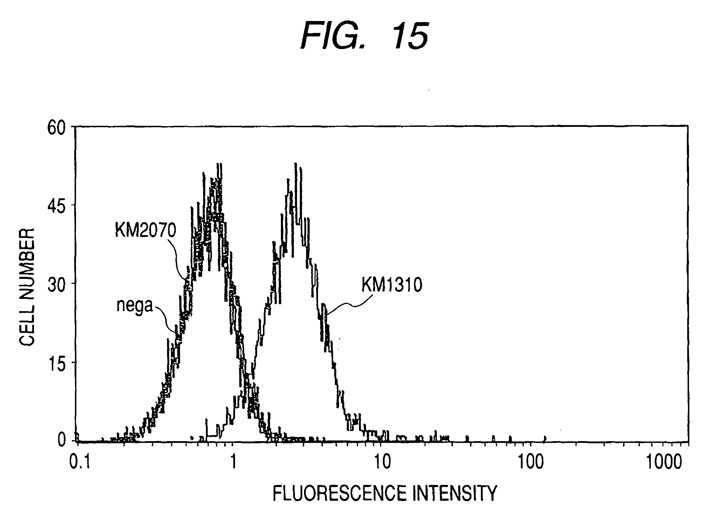

Fig. 15 is a graph showing a result on the reactivity of a monoclonal antibody KM1310 with PA6 cell, analyzed by fluorescent antibody technique using a cell sorter. As a control, a result of the same analysis carried out by using a rat IgM monoclonal antibody KM2070 whose species and subclass coincided with the antibody is shown. The number of cells is shown as the ordinate and the fluorescence intensity is shown as the abscissa.

### BEST MODE FOR CARRYING OUT THE INVENTION

### Example 1

### Recovery of SDIA activity factor from stromal cell:

Recovery of an SDIA activity factor existing in a stromal cell into a solution was tested.

ES cell EB5 (H. Niwa *et al*., *Nature Genet.,* 24, 372 (2000); obtained from Dr. Hitoshi Niwa at the Molecular Control Medical Science Course, Medical School, Osaka University) was used as the embryonic stem cell, and an MC3T3-G2/PA6 cell (H. *Kodama et al*., *J*. *Cell Physiol*., 112, 89 (1982), hereinafter referred to as "PA6 cell") as the stromal cell, and the ES cell EB5 was cultured in accordance with the method described in Reference Example 1 and used in the test.

Cells of PA6 cultured to almost confluent state on a dish of 10 cm in diameter were washed twice with 10 ml of PBS(-) (manufactured by Invitrogen) and once with 10 ml of Hanks' balanced salt solution (manufactured by Invitrogen), 3 ml of Hanks' balanced salt solution containing from 0.001% (w/v) to 0.1% (w/v) of heparin (manufactured by Nacalai Tesque) was added thereto, the mixture was allowed to stand at 37°C for 30 minutes, and the solution was recovered. The solution obtained was added to the PA6 on the 10 cm dish washed in the same manner as described above, the mixture was allowed to stand at 37°C for 30 minutes in the same manner, and then the solution was recovered. This process was carried out on PA6 on the other 10 cm dish, and effect of the recovered solution ES cell was examined. In this case, a solution recovered in the same manner by using a heparin-free Hanks' balanced salt solution was used as a negative control.

The solution obtained in the above was mixed at a ratio of 1:2 with a solution containing the ES cell EB5 which had been adjusted to a single cell state in a serum-free medium by the method described in Reference Example 1, and the mixture was inoculated into a gelatin-coated plastic culture dish at a cell density of 1,250 cells/cm² and incubated at 37°C for 14 days in a CO₂ incubator in an atmosphere of 5% carbon dioxide.

The cells after the culturing were fixed by 4% paraformaldehyde solution and subjected to immune staining in accordance with the method described in *Using Antibodies,* Cold Spring Harbor Laboratory Press (1999), using an anti-NCAM antibody (manufactured by Chemicon) as a general nerve marker, an anti-class III β-tubulin antibody (manufactured by Babco) as a nerve-specific marker and an anti-nestin antibody (manufactured by Pharmingen) as a nerve precursor cell-specific marker.

When 100 or more colonies were optionally selected from the thus obtained colonies and the ratio of expressing these nerve markers was calculated, as shown in Fig. 1, the ratio of colonies expressing class III β-tubulin was 9.9% (n = 101) in the case of the addition of the solution recovered with the negative control heparin-free Hanks' balanced salt solution, while the ratio was significantly increased to 68% (n = 256) in the case of the addition of the solution recovered with the Hanks' balanced salt solution containing 0.001% (w/v) of heparin (Fig. 1). Thus, it was found that an SDIA activity factor can be recovered in a solution by Hanks' balanced salt solution containing heparin.

### Example 2

### Selection of polyanionic compound capable of recovering SDIA activity factor:

Compounds other than heparin with which the SDIA activity factor can be recovered were searched by the method for recovering an SDIA activity factor described in Example 1. Using a Hanks' balanced salt solution containing, instead of heparin, 0.001% (w/v) dextran sulfate (manufactured by Sigma), 0.001% (w/v) polyvinyl sulfate (manufactured by Sigma), 0.001% (w/v) polystyrene sulfonate (manufactured by Tosoh), 0.1% (w/v) carboxymethylcellulose (manufactured by Wako Pure Chemical Industries) or 0.1% (w/v) hyaluronic acid (manufactured by Kibun Chemical Foods), respective solutions were recovered by the method described in Example 1 to examine their effects on the ES cell. In this case, a solution recovered in the same manner by using a heparin-free Hanks' balanced salt solution was used as a negative control.

When 100 or more colonies were optionally selected from the thus obtained colonies and the ratio of expressing a nerve marker was calculated, as shown in Fig. 2, the ratio of colonies expressing class III β-tubulin was significantly increased in comparison with the negative control (10.2%, n = 118), by the use of dextran sulfate (67.2%, n = 238), polyvinyl sulfate (54.8%, n = 155), polystyrene sulfonate (48.7%, n = 229), carboxymethylcellulose (34.6%, n = 358) and hyaluronic acid (19.8%, n = 372). Thus, it was found that the SDIA activity factor can be recovered by using polyanionic compounds such as dextran sulfate, polyvinyl sulfate, polystyrene sulfonate, carboxymethylcellulose, and hyaluronic acid.

### Example 3

### Fixation of solution containing recovered SDIA activity factor on the culture dish surface:

Fixation of the SDIA activity factor recovered in a solution, on the culture dish surface was tested.

An untreated 24 well cell culture dish made of polystyrene was put into the reactor of a PA-300AT type plasma treating apparatus (manufactured by Ohkuma Engineering), and a plasma treatment was carried out for 30 seconds in an oxygen atmosphere of 5 Pa. Immediately after the treatment, the treated dish was returned to the air, 400 µl of 2% polyethyleneimine solution (manufactured by Sigma) was added to each well and allowed to stand for 5 minutes, and then the supernatant was removed from each well by sucking and the dish was dried at 60°C for 6 hours for fixation of polyethyleneimine on the polystyrene surface. After this operation, the dish was soaked in water at 50°C for 30 minutes to remove polyethyleneimine unfixed on the surface. Fixation of the SDIA activity factor on the surface of the culture dish was carried out by adding 1.5 ml of the SDIA activity factor-containing solution recovered by using 0.001% (w/v) heparin solution according to the method described in Example 1 to each well of the polyethyleneimine-fixed 24 well cell culture dish and allowing the mixture to stand at 37°C for 24 hours. In this case, a solution recovered by using a heparin-free Hanks' balanced salt solution was fixed in the same manner and used as a negative control.

The ES cell EB5 which had been prepared to a single cell state by the method described in Reference Example 1 was inoculated onto the thus SDIA activity factor-fixed culture dish at a cell density of 1,250 cells/cm² and cultured in the same manner as the method described in Example 1.

When 10 or more of colonies were optionally selected from the thus obtained colonies and the ratio of colonies having neurite was calculated, as shown in Fig. 3, the ratio was 0% (n = 13) in the case of the negative control but significantly increased to 14.7% (n = 56) on the culture dish coated with the SDIA activity factor-containing solution recovered by heparin. Accordingly, it was found that the SDIA activity factor can be fixed on the culture dish surface by this method.

### Example 4

### Identification of protein having SDIA activity:

As shown in Example 1, a solution containing SDIA activity and a solution which does not contain SDIA activity were prepared by adding Hanks' balanced salt solution containing 0.001% heparin and a solution which does not contain heparin to the PA6 cells, respectively. To 200 µl of each of the solutions, 22 µl of 100% (w/v) trichloroacetic acid solution (manufactured by Nacalai Tesque) was added and the mixture was allowed to stand on ice for 3 hours. After centrifugation at 18,800 g for 20 minutes, the supernatant was discarded and the residue was dissolved in 62.5 mmol/liter Tris-HCl buffer (pH 6.8) containing 2% SDS and 5% 2-mercaptoethanol. Each sample was applied to 12.5% SDS-PAGE together with molecular weight markers and fluorescence-stained with SYPRO-Ruby (manufactured by Bio-Rad) in accordance with the method recommended by the manufacture. An electrophoresis image was obtained from the gel after the staining by using Molecular Imager FX (manufactured by Bio-Rad, excitation wavelength 532 nm, 555 nm long pass filter) (Fig. 4, lanes 1 to 3).

The electrophoresis patterns of lanes 2 and 3 were compared by using a one-dimensional electrophoresis analyzing soft ware Quantity One (manufactured by Bio-Rad), and it was found that a band of about 38 kDa in molecular weight shown by an arrow in Fig. 4 is present in a large amount in the solution having SDIA activity (lane 2). This band was cut out from the gel and then allowed to react together with 25 ng of Sequencing Grade Modified Trypsin (manufactured by Promega) at 37°C for 14 hours in 25 mmol/liter NH₄HCO₃. Peptide fragments formed by the trypsin digestion were separated by a linear density gradient of 5 to 60% acetonitrile containing 0.1% formic acid using reverse phase HPLC (Magic C18 Column, manufactured by Microm BioResources), and the peptide was detected by an ion trap type mass spectrometer LCQ (manufactured by Thermoquest). When values of the mass number of the detected peptide (MS) and the mass number obtained by cleaving the peptide bonding site with a collision gas (MS/MS) were referred to a protein data base using MASCOT soft ware (manufactured by Matrics Sciences), the peptide was identified as a mouse-derived SFRP 1.

Also, the above-described trichloroacetic acid precipitates of the solution containing SDIA activity and the solution which does not contain SDIA activity were subjected to 12.5% SDS-PAGE, electrically transferred on a PVDF membrane, blocked with 5% skim milk solution and then subjected to Western blotting using an anti-SFRP lpolyclonal antibody FRP-1 (H-90) (manufactured by Santacruz) and an HRP-labeled anti-rabbit IgG antibody (manufactured by Amersham Bioscience). The detection was carried out by using an ECL Western blotting detection system (manufactured by Amersham Bioscience) (lanes 4 and 5 in Fig. 4). As a result, SFRP 1 was detected only in the solution having SDIA activity (lane 4).

In addition, when the above-described solution containing SDIA activity was allowed to react at 4°C for 15 hours with a protein G Sepharose carrier to which the anti-SFRP 1polyclonal antibody FRP-1 (H-90) had been linked to obtain a sample in which SFRP 1 was removed from the solution and SDIA activity in the sample was evaluated by the method described in Example 1, decrease of SDIA activity was observed in comparison with the solution containing untreated SDIA activity. Thus, it was found that the SFRP 1 is a protein having SDIA activity.

### Reference Example 1

### Differentiation of embryonic stem cell into dopaminergic neuron:

Coculturing with PA6 cell or a mouse fetal primary culture fibroblast (MEF cell) was carried out.

Since the ES cell EB5 is gene-transferred in such a manner that a drug-resistant gene blastocidine-R is expressed in downstream region of an undifferentiation-specific promoter (Oct3 promoter; E. Pikarsky *et al*., *Mol*. *Cell. Biol.,* 14, 1026 (1994)), undifferentiated ES cell alone can be selected and maintained by culturing it by adding 20 µg/ml of blastocidine. The ES cell EB5 was used in the present invention after confirming that it survived and maintained the undifferentiated state during the testing period in a medium to which 20 µg/ml of blastocidine had been added.

The ES cell EB5 was cultured on a gelatin-coated plastic culture dish in Dulbecco's MEM medium supplemented with 10% fetal bovine serum (ES cell-qualified; manufactured by Litech Oriental), 2 mM glutamine, 100 µM MEM non-essential amino acids solution, 50 U/ml penicillin, 50 U/ml streptomycin, 100 µM 2-mercaptoethanol and 1,000 U/ml LIF (ESGRO Murine LIF; manufactured by Litech Oriental), while keeping the undifferentiated characters according to the method described in *Manipulating the Mouse Embryo, A Laboratory Manual.*

The PA6 cell was cultured according to the method of *Kodama et al*. (H. *Kodama et al*., *J*. *Cell Physiol*., 112, 89 (1982)) in α-MEM medium containing 10% fetal bovine serum (manufactured by GIBCO-BRL).

The MEF cell was prepared and cultured according to the method described in *Manipulating the Mouse Embryo, A Laboratory Manual,* in Dulbecco's MEM medium supplemented with 10% fetal bovine serum (ES cell-qualified; manufactured by Litech Oriental), 2 mM glutamine, 50 U/ml penicillin and 50 U/ml streptomycin.

Differentiation of the ES cell was induced by coculturing the ES cell in a single cell state with the PA6 cell or MEF cell.

The ES cell EB5 in a single cell state was produced in the following manner.

The ES cell EB5 was proliferated to a 30% confluent by exchanging the medium. After removing the medium, the cells were washed twice with PBS(-) and then cultured at 37°C for 20 minutes by adding PBS(-) containing 1 mM EDTA and 0.25% trypsin. The culture solution was suspended in a medium (hereinafter referred to as "serum-free medium") produced by adding 10% KNOCKOUT SR (manufactured by GIBCO BRL), 2 mM glutamine, 100 µM MEM non-essential amino acids solution, 1 mM pyruvic acid, 50 U/ml penicillin, 50 U/ml streptomycin and 100 µM 2-mercaptoethanol to the Glasgow MEM medium. The suspension was centrifuged at 4°C and at 200×g for five minutes, and the precipitated cells were suspended again in the serum-free medium to prepare the ES cell EB5 in a single cell state.

The PA6 cell or MEF cell whose cell density reached almost confluent by exchanging the medium in advance was washed twice with PBS(-) and then suspended in the serum-free medium to prepare as feeder cells.

The ES cell EB5 in a single cell state was inoculated at a cell density of 10 to 100 cells/cm² into a culture vessel in which the thus produced PA6 cell was cultured, the medium was exchanged with a fresh serum-free medium on the 4th, 6th and 7th day, and then the cells were cultured at 37°C for 8 days in a stream of 5% carbon dioxide in a CO₂ incubator. As a control, the ES cell was inoculated in the same manner into a simply gelatin-coated culture vessel and cultured in the same manner.

Eight days after coculturing, the medium in the culturing vessel was removed and the cells were fixed for 30 minutes by adding 4% paraformaldehyde solution. The thus fixed cells were immunologically stained according to the method described in *Using Antibodies*, Cold Harbor Laboratory Press (1999), using an antibody against a typical neuron marker NCAM (manufactured by Chemicon, hereinafter referred to as "anti-NCAM antibody"), an antibody against a neuron-specific marker class III β-tubulin (manufactured by Babco, hereinafter referred to as "anti-tubulin antibody") and an antibody against a neural precursor cell-specific marker nestin (manufactured by Pharmingen, hereinafter referred to as "anti-nestin antibody").

PA6 cell and ES cell EB5 were cocultured for 10 days by the above method. Resulting cells in the culture vessel were fixed and then immunologically stained by using an antibody against a dopaminergic neuron marker tyrosine hydroxylase (manufactured by Chemicon), an antibody against a cholinergic neuron marker VAchT (manufactured by Chemicon), an antibody against a GABAergic neuron marker GAD (manufactured by Chemicon), an antibody against a serotonergic neuron marker serotinin (manufactured by Dia Sorin) or an antibody against a noradrenaline neuron marker dopamine β-hydroxylase (manufactured by PROTOS Biotech).

Using a 3 cm dish for tissue culture (made of plastic, manufactured by FALCON) as a culturing vessel, 200 cells of the ES cell EB5 were cultured by inoculating them into each of 1) the dish prepared by using the PA6 cell as a feeder cell, 2) the dish prepared by using the MEF cell as a feeder cell and 3) the dish simply coated with gelatin, with the results shown in the drawings.

Cells of the ES cell EB5 inoculated in a single cell state adhered to the feeder cells or to the dish surface without causing mutual aggregation, repeated cell division and formed colonies (hereinafter referred to as "ES cell-derived colonies" or simply as "colonies").

Fig. 5 shows a result of staining of the colonies formed by the coculturing with PA6 cell, with (A) the anti-NCAM antibody, (B) the anti-tubulin antibody or (C) the anti-nestin antibody.

Fig. 6 shows a result of staining of the colonies formed by the coculturing with MEF cell, with the anti-NCAM antibody.

Fig. 7 shows a result of staining of the colonies formed by the coculturing with PA6 cell, with an antibody against tyrosine hydroxylase (hereinafter referred to as "anti-tyrosine hydroxylase antibody").

Fig. 8 shows periodical changes in the ratio of each marker-positive colonies among colonies formed by the coculturing with PA6 cell. The ratio of colonies was calculated by preparing 160 dishes cocultured under each of the above-mentioned conditions 1), 2) and 3) and observing staining intensity of all of the formed colonies under a microscope.

In the coculture system of the condition 1) in which the PA6 cell was prepared as a feeder cell, 90% (n = 160) of colonies derived from the ES cell EB5 were strongly NCAM-positive as shown in Fig. 5A. These colonies were staining-positive with both of the anti-tubulin antibody (Fig. 5B) and anti-nestin antibody (Fig. 5C). On the other hand, the appearance of significant neuron markers was not found by coculturing with MEF cell as the condition 2) (Fig. 6). The colonies cultured on the gelatin-coated culture vessel showed the same staining result of the colonies formed by coculturing with MEF cell as the condition 2). In the coculture system of the condition 1) in which the PA6 cell was prepared as a feeder cell, anti-tyrosine hydrolase antibody-positive colonies derived from the ES cell were found at a high frequency (89%) (Fig. 7). As a result of coculturing of PA6 cell with ES cell EB5, nestin-positive colonies appeared 3 days, and tubulin-positive colonies 4 days, after starting of the coculturing, periodically as shown in Fig. 8. Also, 5 days after, tyrosine hydrolase-positive colonies appeared, and 10 days after, they reached the peak. During this period, the immunological staining with the antibody against a noradrenaline neuron marker dopamine β-hydroxylase was negative. Ten days thereafter, cholinergic neuron marker VAchT-positive colonies were formed at a frequency of 5%, GABAergic neuron marker GAD-positive colonies at a frequency of 15% and serotonin-positive colonies at 4%.

Also, a result similar to the above was obtained when coculturing was carried out using a typical ES cell, 129 line mouse-derived CCE cell (M.R. Kuehn *et al*., *Nature,* 326, 295 (1987); *Production of Mutation Mice Using ES Cell*).

### Reference Example 2

### Differentiation of embryonic stem cell into non-neuroectodermal cell:

A medium was prepared by adding 0.5 nmol/l BMP4 (manufactured by R & D) to the serum-free medium described in Reference Example 1. The ES cell EB5 was cocultured with PA6 cell according to the method described in Reference Example 1, using the thus produced BMP4-added serum-free medium instead of the serum-free medium used in Reference Example 1. Eight days after culturing, immunological cell staining was carried out by using the anti-NCAM antibody, the anti-nestin antibody or an antibody against a non-neural ectoderm cell marker E cadherin (manufactured by Takara Shuzo). As a control, coculturing was carried out in the serum-free medium without BMP4. The results are shown in Figs. 9A, B, C, D, E and F.

Also, 8 days after culturing in the BMP4-added serum-free medium, the medium was changed to Glasgow MEM medium containing 10% fetal bovine serum (manufactured by GIBCO BRL), followed by culturing for 3 days. The thus cultured cells were fixed for 30 minutes by adding 4% paraformaldehyde and immunologically stained with an antibody against a skin epidermis cell marker keratin 14 (manufactured by Biomedia), and the results were compared with those in which culturing was continued for additional 3 days in the bovine serum-free medium, with the results shown in Figs. 9G, H and I.

As shown in Reference Example 1, when the medium without BMP4 was used, the ES cell-derived colonies were strongly anti-NCAM antibody-positive (Fig. 9A) and strongly anti-nestin antibody-positive (Fig. 9B), whereas the number of E cadherin-positive colonies was small (18%) (Fig. 9C). On the other hand, when the BMP4-added serum-free medium was used, the ES cell-derived colonies were anti-NCAM antibody-negative (Fig. 9D) and anti-nestin antibody-negative (Fig. 9E), whereas E cadherin-positive colonies appeared at a high frequency (98%) (Fig. 9F). Keratin 14-positive colonies were not formed when the medium without no BMP4 was used (Fig. 9G), whereas they appeared when the BMP4-added serum-free medium was used at a frequency of 34% (Fig. 9H). When culturing was carried out for 8 days in the BMP4-added serum-free medium and then for next 3 days in the Glasgow MEM medium containing 10% fetal bovine serum, both of the frequency of keratin 14-positive colonies (47%) and the colony size significantly increased (Fig. 9I).

Also, a result similar to the above was obtained when coculturing was carried out with a typical ES cell, 129 line mouse-derived CCE cell (M.R. Kuehn *et al*., *Nature*, 326, 295 (1987); *Production of Mutation Mice Using ES Cell*).

### Reference Example 3

### Selection of stromal cell having activity of inducing differentiation of embryonic cell into dopaminergic neuron:

ES cell EB5 was cocultured with PA6 cell, MEF cell, STO cell, NIH/3T3 cell, OP9 cell, CHO cell, MDCK cell, 3Yl cell or COS cell (hereinafter referred to as "respective cells").

The STO cell was cultured according to the method described by Evans *et al*. (M.J. Evans *et al*., *Nature*, 292, 154 (1981)). The NIH/3T3 cell was cultured according to the method described by Jainchill *et al*. (J.L. Jainchill *et al*., *J*. *Virol*, 4, 549 (1969)). The OP9 cell was cultured according to the method described by Nakano *et al*. (T. Nakano *et al*., *Science,* 272, 722 (1996)). The CHO cell was cultured according to the method described by Puck *et al*. (T.T. Puck *et al*., *J*. *Exp*. *Med*., 108, 945 (1985)). The MDCK cell was cultured according to the method described by Misfeldt *et al*. (D.S. Misfeldt *et al*., *Proc. Natl*. *Acad*. *Sci. USA*, 73, 1212 (1976)). The 3Y1 cell was cultured according to the method described by Sandineyer *et al*. (S. Sandineyer *et al*., *Cancer Res.,* 41, 830 (1981)). The COS cell was cultured according to the method described by Gluzman (*Cell,* 23, 175 (1981)).

According to the method described in Reference Example 1, the respective cells and ES cell EB5 were cocultured for 8 days and immunologically stained with the anti-NCAM antibody, and the ratio of positive ES cell-derived colonies was examined. As a result, the PA6 cell, OP9 cell and NIH/3T3 cell showed positive ratios of 95%, 45% and 10%, respectively, so that it was confirmed that these cells have significant nerve differentiation-inducing activity for the ES cell. On the other hand, other cells did not show significant nerve differentiation-inducing activity.

Next, the ES cell were cocultured with the respective cells fixed with paraformaldehyde.

Respective cells whose cell density reached almost confluent by exchanging the medium in advance was washed twice with PBS(-) and then fixed by adding 4% paraformaldehyde solution and incubated at 4°C for 30 minutes. Respective cells were prepared by washing the fixed cells several times with PBS(-).

The ES cell EB 5 was cocultured with each of the thus prepared respective cells as a feeder cell according to the method described in Reference Example 1. When the cells fixed with paraformaldehyde were used, differentiation of the ES cell into nerve cell was observed at a high ratio by coculturing with the PA6 cell, OP9 cell, NIH/3T3 cell, MEF cell or STO cell but was not observed by coculturing with the 3Y1 cell, COS cell, MDCK cell or CHO cell. It was found from these results that a group of stromal cells, namely PA6 cell, OP9 cell, NIH/3T3 cell, MEF cell and STO cell, have the nerve differentiation-inducing activity, and that this activity remains even when these cells are fixed with paraformaldehyde. Also, it was suggested that a mechanism for inhibiting the nerve differentiation-inducing activity is removed by the paraformaldehyde treatment in the MEF cell and STO cell.

### Reference Example 4

### Analysis of activity of stromal cell to differentiate embryonic stem cell into nerve cell:

In order to analyze the activity of stromal cell to differentiate an embryonic stem cell into a nerve cell, an ES cell and a stromal cell were cocultured via a porous filter.

As the porous filter, a 6-well cell culture insert of which pore size is 0.45 µm (product No. 3090, manufactured by FALCON) was used. The PA6 cell was cultured in the inner side of the cell culture insert, and the PA6 cell adhered on the filter was prepared as a feeder cell according to the method described in Reference Example 1.

The ES cell EB5 suspended in the serum-free medium described in Reference Example 1 was inoculated in 400 cells/well into a gelatin-coated 6-well culture dish (manufactured by FALCON), and the cell culture insert prepared by using the PA6 cell as a feeder cell was inserted into the wells, followed by culturing. That is, the ES cell EB5 inoculated onto the 6-well culture dish and the PA6 cell prepared as a feeder cell inside the cell culture insert were cocultured via the filter membrane. Forth, sixth and seventh days after starting of culturing, the medium was exchanged with a fresh serum-free medium, and the cells were cultured at 37°C for 8 days in a stream of 5% carbon dioxide in a CO₂ incubator. Eight days after coculturing, the medium was removed and the cells were fixed for 30 minutes by adding 4% paraformaldehyde solution. The thus fixed cells were immunologically stained with an antibody against a neuron-specific marker tubulin (manufactured by Babco) according to the method described in *Using Antibodies,* Cold Harbor Laboratory Press (1999). The formed ratio of tubulin-positive colonies was compared with that in culturing without using the filter, with the results shown in Fig. 10.

When the PA6 cell and ES cell EB5 were cocultured via the filter (Fig. 10, Filter), 25% of the colonies were tubulin-positive. Although this proportion was about 1/3 of the efficiency in comparison with that in culturing without using the filter (Fig. 10, PA6), the nerve differentiation was significantly higher than that in culturing on gelatin without PA6 cell (Fig. 10, Gelatin, positive ratio 3% or less).

### Reference Example 5

### Analysis of intracerebral transplantation of embryonic stem cell differentiated into dopaminergic neuron (Part 1):

According to the method described in Reference Example 1, the ES cell EB5 was cultured for 10 days in the serum-free medium without BMP4 using the PA6 cell as a feeder cell. That is, the PA6 cell proliferated to almost confluent on a 6 cm tissue culture dish was used as a feeder cell, the ES cell EB5 was inoculated onto the feeder cell at a density of 2,000 cells/dish, the medium was exchanged with a fresh serum-free medium on the 4th, 6th and 8th days, and the cells were cultured at 37°C for 10 days in a stream of 5% carbon dioxide in a CO₂ incubator.

The cells differentiation-induced as a result of culturing were fluorescence-labeled with a cell lineage tracer DiI (manufactured by Molecular Probe) according to the manufacture's instructions. After labeling, a papain-treatment was carried out at room temperature for 5 minutes by using Papain Dissociation System Kit (manufactured by Worthington) according to the manufacture's instructions, and the formed ES cell-derived colonies were separated from the feeder cell almost as a mass. In this case, in order to avoid damage to nerve cells in the colonies, each colony formed by the differentiation induction was separated from the feeder cell as a mass of colonies as much as possible and used in the transplantation.

After inactivation of the enzyme by using the papain inhibitor attached to the kit, a mass of the differentiation-induced ES cells was recovered by centrifugation at 300 rpm for 5 minutes. The mass of the differentiation-induced ES cells recovered from one 6 cm dish was suspended in 5 µl of N₂-added Glasgow MEM medium (manufactured by Gibco Lifetech) and used in the following transplantation.

The transplantation and pharmaceutical injection were carried out according to the method described in *Current Protocols in Neuroscience* (John Wiley & Sons (1999)) 3.10. Each of C57BL/6 mice anesthetized with nembutal was fixed on a stereotaxic apparatus (manufactured by Narishige), and positions of the striate bodies were identified according to the method described in *The Mouse Brain in Stereotaxic Coordinates* (Academic Press (1997)). In order to destroy the topical dopamine nerve, 6-hydroxydopamine (2,4,5-trihydroxyphenethylamine)hydrobromide (hereinafter referred to as "6-OHDA") was dissolved in PBS at a concentration of 8 mg/ml and, using a micro-glass tube, injected into a position on the mouth side and a position on the tail side of either of the striate bodies, 4 µl of the resulting solution for each of the two positions. Three days thereafter, extrapyramidal signs in the injected side of several of the mice were confirmed and then 2 µl of the suspension ofES cell mass differentiation-induced into nerve cells was injected by the above method into central region of the same side striate body, spending 4 minutes by using a blunted 26G Hamilton syringe. Eight days after the 6-OHDA treatment, tissue samples were produced by perfusion-fixing the brain of each mouse and immunologically stained with an antibody against a dopaminergic neuron marker tyrosine hydroxylase (manufactured by Chemicon) and an antibody against dopamine transporter (manufactured by Chemicon).

In the group in which cell transplantation was not carried out by treating with 6-OHDA for destroying the dopamine nerve, nerve tissues expressing the tyrosine hydroxylase and dopamine transporter in the same side striate body were 40% or less of the normal tissues (n = 6). On the other hand, in the group in which transplantation of the differentiation-induced ES cell was carried out, the tyrosine hydroxylase- and dopamine transporter-expressing regions in the same side striate body were significantly recovered and became about 75% as a total (n = 6), mainly in the DiI-labeled grafts, so that recovery of the dopaminergic neuron by the transplantation was observed.

### Reference Example 6

### Analysis of intracerebral transplantation of embryonic stem cell differentiated into dopaminergic neuron (Part 2):

According to the method described in Reference Example 1, the ES cell EB5 was cultured for 8 days in the serum-free medium without BMP4 by using the PA6 cell as a feeder cell. That is, the PA6 cell proliferated to almost confluent on a 6 cm tissue culture dish was used as a feeder cell, the ES cell EB5 was inoculated onto the feeder cell at a density of 2,000 cells/dish, the medium was exchanged with a fresh serum-free medium on the 4th and 6th days, and the cells were cultured at 37°C for 8 days in a stream of 5% carbon dioxide in a CO₂ incubator.

Culturing was further carried out for 4 days in Glasgow MEM medium to which 2 mmol/l glutamine, 1 mmol/l pyruvic acid, 0.1 mmol/l MEM non-essential amino acids solution, 0.1 mmol/l 2-mercaptoethanol and N₂ (manufactured by GIBCO BRL, 1/100 of the 100-fold stock solution was added) had been added (hereinafter referred to as "N₂-added Glasgow MEM medium").

After culturing, the cells were cultured for 2 hours in a medium (Glasgow MEM medium) containing 10 µg/ml mitomycin C (MMC) according to the method described in *Manipulating the Mouse Embryo*, *A Laboratory Manual*.

The cells differentiation-induced as a result of culturing were subjected to a papain-treatment at room temperature for 5 minutes by using Papain Dissociation System Kit (manufactured by Worthington) according to the manufacture's instructions, and the formed ES cell-derived colonies were separated from the feeder cell almost as a mass (in this case, in order to avoid damage to nerve cells in the colonies, each colony formed by the differentiation induction was separated from the feeder cell as a mass of colonies as much as possible). After separation of the colonies, the colony-forming cells were fluorescence-labeled by using a cell lineage tracer DiI (manufactured by Molecular Probe) according to the manufacture's instructions, by allowing the cells to react at room temperature for 20 minutes in a PBS solution containing 5 µg/ml CM-DiI and 4 mg/ml glucose. After labeling, the cells were washed with the N₂-added Glasgow MEM medium, made into a suspension of such a density that about 4×10⁵ cells were contained in 1 µl of the N₂-added Glasgow MEM medium, and used in the following transplantation.

The transplantation and pharmaceutical injection were carried out according to the method described in *Current Protocols in Neuroscience* (John Wiley & Sons (1999) 3.10. Each of C57BL/6 mice anesthetized with nembutal was fixed on a stereotaxic apparatus (manufactured by Narishige), and positions of the striate bodies were identified according to *The Mouse Brain in Stereotaxic Coordinates* (Academic Press, 1997). In order to destroy the topical dopamine nerve, 6-OHDA was dissolved in PBS at a concentration of 8 µg/µl and, using a micro-glass tube, injected into three positions in either of the striate bodies, 0.5 µl for each of the three positions (A+0.5, L+2.0, V+3.0), (A+1.2, L+2.0, V+3.0) and (A+0.9, L+1.4, V+3.0), according to the method as described in *The Mouse Brain in Stereotaxic Coordinates* (Academic Press, 1997). Three days thereafter, extrapyramidal signs in the injected side of part of the mice were confirmed and then 1 µl of the suspension of ES cell mass differentiation-induced into nerve cell by the above method was injected into central region (A+0.9, L+2.0, V+3.0) of the same side of the striate body, spending a period of 3 minutes by use of a blunted 26G Hamilton syringe. Into a control group, Glasgow MEM medium supplemented with 1 µl of N₂ was injected. Fourteen days after the 6-OHDA treatment, the brain of each mouse was perfusion-fixed and immunologically stained with an antibody against a dopaminergic neuron marker tyrosine hydroxylase (manufactured by Chemicon) and an antibody against dopamine transporter (manufactured by Chemicon).

In the group in which cell transplantation was not carried out and treatment with 6-OHDA for destroying the dopamine nerve was carried out, nerve tissues expressing the tyrosine hydroxylase and dopamine transporter in the same side of the striate body were 15% or less of the normal tissues (n = 5). On the other hand, in the group in which the cell transplantation was carried out, the tyrosine hydroxylase- and dopamine transporter-expressing regions in the same side of the striate body were significantly recovered and became about 50% as a total (n = 5), mainly in the DiI-labeled grafts. Also, formation of teratoma was not observed even 2 weeks after the transplantation.

### Reference Example 7

### Analysis of differentiation process of embryonic stem cell into nervous ectodermal cell:

According to the method described in Reference Example 1, the ES cell EB5 was cultured for 8 days in the serum-free medium without BMP4 by using the PA6 cell as a feeder cell. That is, the PA6 cell proliferated to almost confluent on a 3 cm tissue culture dish was used as a feeder cell, the ES cell EB5 was inoculated onto the feeder cell at a density of 200 cells/dish, the medium was exchanged with a fresh serum-free medium on the 4th, 6th and 7th days, and the cells were cultured at 37°C for 8 days in a stream of 5% carbon dioxide in a CO₂ incubator.

Eight days after coculturing, the cells were fixed according to the method described in Reference Example 1, and colonies formed as a result of coculturing of the ES cell EB5 and PA6 cell were immunologically stained with the anti-NCAM antibody, the anti-class III β-tubulin antibody, the anti-nestin antibody, an antibody against a presynapse-specific marker synaptophysin (manufactured by Sigma), an RC2 antibody which recognizes neuroepitheliums (manufactured by Developmental Studies Hybridoma Bank), an MF20 antibody which recognizes mesodermal cells (manufactured by Developmental Studies Hybridoma Bank) and an antibody against PDGF receptor α or Flk1 whose expression are observed in mesodermal cells (S.I. Nishikawa *et al*., *Development*, 125, 1747 (1998)).

Similar to the results shown in Reference Example 1, most of colonies formed as a result of coculturing of the ES cell EB5 and PA6 cell were stained with the anti-NCAM antibody. Also, as a result of double antibody staining, anti-tubulin antibody-positive colonies were stained with the anti-synaptophysin antibody and nestin-positive colonies were stained with the anti-RC2 antibody. On the other hand, colonies stained with the mesodermal cell markers PDGF receptor α and Flk1 and with various antibodies against MF20 were hardly observed, which were 2% or less of the total colonies. Thus, it was suggested that the induction process of the ES cell into the nerve cell by coculturing with PA6 cell does not substantially accompany induction of mesodermal cells.

Also, a result similar to the above was obtained when coculturing was carried out by using a typical ES cell, 129 line mouse-derived CCE cell (M.R. Kuehn *et al*., *Nature,* 326, 295 (1987); *Production of Mutation Mice Using ES Cell*).

### Reference Example 8

### Analysis of differentiation process of embryonic stem cell into non-neuroectodermal cell:

A medium was produced by adding 0.5 nmol/l of BMP4 (manufactured by R & D) to the serum-free medium described in Reference Example 1. Using the thus produced BMP4-added serum-free medium instead of the serum-free medium used in Reference Example 1, the ES cell EB5 and PA6 cell were cocultured according to the method described in Reference Example 1. That is, the PA6 cell proliferated to almost confluent on a 3 cm tissue culture dish was used as a feeder cell, the ES cell was inoculated onto the feeder cell at a density of 200 cells/dish, medium exchange was carried out with a fresh medium on the 4th, 6th and 7th days, and the cells were cultured at 37°C for 8 days in a stream of 5% carbon dioxide in a CO₂ incubator.

Eight days after coculturing, the cells were fixed according to the method described in Reference Example 1, and colonies formed as a result of coculturing of the ES cell and PA6 cell were immunologically stained with the anti-NCAM antibody, the anti-E cadherin antibody, an MF20 antibody which recognizes mesodermal cells (manufactured by Developmental Studies Hybridoma Bank) and an antibody against PDGF receptor α or Flk1 whose expression are observed in mesodermal cells (S.I. Nishikawa *et al*., *Development*, 125, 1747 (1998)).

Similar to the results shown in Reference Example 2, NCAM-negative Ecadherin-positive colonies were formed at a high frequency by coculturing the ES cell and PA6 cell in the BMP4-added serum-free medium. On the other hand, colonies stained with various antibodies against the mesodermal cell markers PDGF receptor α, Flk1 and MF20 were hardly observed, which were 5% or less of the total colonies. Thus, it was suggested that the induction process of the ES cell into the non-neural cell by coculturing with PA6 cell in the presence of BMP4 does not substantially accompany induction of mesodermal cells.

Also, a result similar to the above was obtained when coculturing was carried out by using a typical ES cell, 129 line mouse-derived CCE cell (M.R. Kuehn *et al*., *Nature*, 326, 295 (1987); *Production of Mutation Mice Using ES Cell*).

### Reference Example 9

### Analysis of nerve cell colonies differentiation-induced from embryonic stem cell:

According to the method described in Reference Example 1, the ES cell EB5 was cultured for 12 days in the serum-free medium without BMP4 by using the PA6 cell as a feeder cell. That is, the PA6 cell proliferated to almost confluent on a 3 cm tissue culture dish was used as a feeder, the ES cell was inoculated onto the feeder cell at a density of 200 cells/dish, the medium was exchange with a fresh serum-free medium on the 4th, 6th, 8th and 10th days, and the cells were cultured at 37°C for 12 days in a stream of 5% carbon dioxide in a CO₂ incubator.

Ten days after coculturing, the cells on some of the dishes were fixed according to the method described in Reference Example 1, and the colonies formed as a result of coculturing of the ES cell EB5 with PA6 cell were immunologically stained with antibodies against tyrosine hydroxylase, VachT, GAD and serotonin (n = 200).

Among the colonies formed as a result of coculturing of the ES cell EB5 and PA6 cell, 92% were dopaminergic neuron marker tyrosine hydroxylase-positive, 43% were GABAergic neuron marker GAD-positive, 28% were cholinergic neuron marker VachT-positive and 7% were serotonin-positive.

Next, culturing of the remaining dishes was continued and, 12 days after culturing, the cells were fixed according to the method described in Reference Example 1, and the colonies formed as a result of coculturing of the ES cell EB5 with PA6 cell were immunologically stained with antibodies against class III β-tubulin, nestin and tyrosine hydroxylase. Also, in order to measure the number of total cells comprising colonies, nuclear staining was carried out by using a kit YOYO-1 manufactured by Molecular Probe. After nuclear staining, colonies formed as a result of the coculturing of the ES cell EB5 with PA6 cell (n = 20) were randomly selected, and the number of stained cells was counted by observing them under a confocal microscope (n = 5,050).

Among the total cells counted, ratios of the class III β-tubulin-positive cells, nestin-positive cells and tyrosine hydroxylase-positive cells were 52±9%, 47±10% and 30±4%, respectively.

Also, a result similar to the above was obtained when coculturing was carried out by using a typical ES cell, 129 line mouse-derived CCE cell (M.R. Kuehn *et al*., *Nature,* 326, 295 (1987); *Production of Mutation Mice Using ES Cell*).

### Reference Example 10

### Analysis of dopaminergic neuron differentiation-induced from embryonic stem cell (Part 1):

In order to more minutely analyze properties of the nerve cell differentiation-induced from the embryonic stem cell by the method described in Reference Example 1, changes in the expression accompanied by the differentiation induction of Nurr1 (R.H. Zetterstrom *et al*., *Science,* 276, 248 (1997)) and Ptx3 (M.P. *Smidt et al*., *Proc. Natl. Acad*. *Sci. USA,* 94, 13305 (1997)), as markers of dopaminergic neurons in the midbrain, were examined by RT-PCR method.

The cells were prepared as follows.

According to the method described in Reference Example 1, the ES cell EB5 was cultured for 12 days in the serum-free medium without BMP4 by using the PA6 cell as a feeder cell. That is, the PA6 cell proliferated to almost confluent on a 9 cm tissue culture dish was used as a feeder, the ES cell EB5 was inoculated onto the feeder cell at a density of 5×10⁴ cells/dish, medium exchange was carried out with a fresh serum-free medium on the 4th, 6th, 8th and 10th days, and the cells were cultured at 37°C for 12 days in a stream of 5% carbon dioxide in a CO₂ incubator.

Also, using the ES cell culturing medium shown in Reference Example 1, the ES cell EB5 was inoculated into a 9 cm tissue culture dish at a density of 5×10⁴ cells/dish, medium exchange was carried out with a fresh medium on the 4th, 6th, 8th and 10th days, and the cells were cultured at 37°C for 12 days in a stream of 5% carbon dioxide in a CO₂ incubator.

In order to detect expression of Nurr1 and Ptx3 at mRNA level in the thus prepared differentiation-induced cell and control ES cell, RT-PCR was carried out by using the head of a mouse of 12 days of fetal age as the positive control according to the method reported by Sasai *et al*. (Y. Sasai *et al*., *Nature*, 376, 333 (1995)). That is, total RNA was prepared from each of the cell-produced dishes and the head of a mouse of 12 days of fetal age, and cDNA was synthesized therefrom by using SUPER SCRIPT Preamplification System for First Strand cDNA Synthesis (manufactured by GIBCO BRL). A reaction solution (10 mmol/l Tris-HCl (pH 8.3), 50 mmol/l KCl, 1.5 mmol/l MgCl₂, 0.2 mmol/l dNTP, 0.2 µmol/l of each gene-specific primer (shown in Sequence Listing) and 1 unit of recombinant Ex Taq polymerase (manufactured by Takara Shuzo)) was produced by using a solution of the thus synthesized cDNA diluted 50 times with sterile water as a material according to the usual method, and PCR was carried out under conditions in which the reaction was carried out by incubating the reaction solution at 94°C for 3 minutes, repeating 30 cycles of a cycle of 94°C for 30 seconds, 55°C for 30 second and 72°C for 1 minute and finally incubating it at 72°C for 7 minutes, and the reaction solution was stored overnight at 4°C. Semi-quantitative comparison of the expressed amounts of respective factors was carried out by subjecting the reaction solution to an agarose gel electrophoresis and comparing the density of DNA bands specific for the used primers.

In this case, oligonucleotides having the nucleotide sequences represented by SEQ ID NOs:1 and 2 were used as the Nurr1-specific primers, and oligonucleotides having the nucleotide sequences represented by SEQ ID NOs:3 and 4 as the Ptx3-specific primers and oligonucleotides having the nucleotide sequences represented by SEQ ID NOs:5 and 6 were used as the G3PDH-specific primers. When PCR was carried out by using the Ptx3-specific primers, DMSO was added to the reaction solution to give a final concentration of 5%.

As a result of the 12 days of coculturing of ES cell with PA6 cell, nerve cell-like colonies were formed similar to the case of the result shown in Reference Example 1. Also, similar to the positive control, significant expression of Nurr1 and Ptx3 was observed in a cell group containing the differentiation-induced colony (Fig. 11: ES+PA6). On the other hand, expression of Nurr1 and Ptx3 was not detected in the control ES cell (Fig. 11: ES). Also, expression of Nurr1 and Ptx3 was not detected when RT-PCR was carried out under the above conditions using the PA6 cell proliferated to almost confluent on a 9 cm tissue culture dish as a control. Thus, it was found that expression of Nurr1 and Ptx3, as markers of dopaminergic neurons in the midbrain, increases, as embryonic stem cells are differentiation-induced into nerve cells by coculturing with PA6 cell.

Also, a result similar to the above was obtained when coculturing was carried out by using a typical ES cell, 129 line mouse-derived CCE cell (M.R. Kuehn *et al*., *Nature*, 326, 295 (1987); *Production of Mutation Mice Using ES Cell*).

### Reference Example 11

### Analysis of dopaminergic neuron differentiation-induced from embryonic stem cell (Part 2):

In order to more minutely analyze properties of the nerve cell differentiation-induced from the embryonic stem cell by the method described in Reference Example 1, the produced amount of dopamine was determined by using HPLC according to the usual method (K. Inoue, J.G. Kenimer *et al*., *J. Biol*. *Chem.,* 263, 8157 (1988); M. Imaizumi and K. Kumakura, *Experimental Medicine Supplement, Nerve Biochemistry Manual,* pp. 191-200 (1990)).

The measuring samples from cells was produced as follows.

According to the method described in Reference Example 1, the ES cell EB5 was cultured for 8 days in the serum-free medium without BMP4 by using the PA6 cell as a feeder cell. That is, the PA6 cell proliferated to almost confluent on a 9 cm tissue culture dish was used as a feeder, the ES cell was inoculated onto the feeder cell at a density of 5×10⁴ cells/dish, the medium was exchanged with a fresh serum-free medium on the 4th and 6th days, and the cells were cultured at 37°C for 8 days in a stream of 5% carbon dioxide in a CO₂ incubator. Thereafter, culturing was further carried out for 6 days in the Glasgow MEM medium to which 2 mmol/l glutamine, 1 mmol/l pyruvic acid, 0.1 mmol/l MEM non-essential amino acids solution, 0.1 mmol/l 2-mercaptoethanol, 0.2 mmol/l ascorbic acid, 0.1 mmol/l tetrahydrobiopterin and N₂ had been added. After culturing, the cells were washed twice with a buffer HBSS (manufactured by GIBCO BRL), and the washed cells were cultured for 15 minutes in the HBSS solution containing 56 mmol/l KCl. Fifteen minutes thereafter, the cultured medium was recovered, mixed with 0.4 mol/l perchloric acid and 5 mmol/l EDTA in final concentrations and then preserved at -80°C as the measuring sample.

About one million cells were formed as ES cell-derived differentiated cells by coculturing ES cell with PA6 cell. Also, the amount of dopamine in measuring samples produced by using the formed cells was determined using a reverse phase HPLC-aided Monoamine Analysis System (Eicom Corp., Kyoto, Japan). The results are shown in Fig. 12.

It was found that the nerve cells differentiated from embryonic stem cells by coculturing with PA6 cell released a significant amount of dopamine by stimulation with 56 mmol/l of KCl (7.7 pmol/10⁶ cells (ES cell-derived differentiated cells)). Dopamine derivatives DOPAC (3,4-dihydroxyphenylacetic acid) and HVA (homovanillic acid) were also detected in significant amounts (2.5 pmol/10⁶ cells (ES cell-derived differentiated cells) and 4.0 pmol/10⁶ cells (ES cell-derived differentiated cells), respectively).

Thus, it was also shown *in vitro* that the dopaminergic neuron differentiation-induced from embryonic stem cells by coculturing with PA6 cell has ability of functioning as a functional nerve by producing dopamine.

### Reference Example 12

### Differentiation of primitive ectoderm-constituting cell into dopaminergic neuron:

A cell isolated from a primitive ectoderm (pre-streak epiblast) of a mouse of 6 days of fetal age was used instead of the ES cell EB5, and cocultured with PA6 cell according to the method described in Reference Example 1 or 2.

Cells constituting the pre-streak epiblast of a mouse of 6 days of fetal age were isolated and cultured according to the method described in *Manipulating the Mouse Embryo, A Laboratory Manual.*

According to the method described in Reference Example 1, the isolated embryonic cell was cultured for 8 days in the serum-free medium without BMP4 by using the PA6 cell as a feeder cell. That is, the PA6 cell proliferated to almost confluent on a 3 cm tissue culture dish was used as a feeder cell, the isolated pre-streak epiblast cell was inoculated onto the feeder cell at a density of 200 cells/dish, the medium was exchanged with a fresh serum-free medium on the 4th, 6th and 7th days, and the cells were cultured at 37°C for 8 days in a stream of 5% carbon dioxide in a CO₂ incubator.

Also, the isolated embryonic cell was cocultured with PA6 cell according to the method described in Reference Example 1, in a medium prepared by supplementing the serum-free medium used in Reference Example 1 with 0.5 nmol/l BMP4 (manufactured by R & D). That is, the PA6 cell proliferated to almost confluent on a 3 cm tissue culture dish was used as a feeder cell, the ES cell was inoculated onto the feeder cell at a density of 200 cells/dish, the medium was exchanged with a fresh medium on the 4th, 6th and 7th days, and the cells were cultured at 37°C for 8 days in a stream of 5% carbon dioxide in a CO₂ incubator.

Eight days after coculturing, the cells were fixed according to the method described in Reference Example 1, and the colonies formed as a result of coculturing of the isolated embryonic cell with PA6 cell were immunologically stained with the anti-NCAM antibody, anti-tubulin antibody, anti-nestin antibody and anti-E cadherin antibody.

A result similar to Reference Examples 1 and 2 carried out by using the ES cell EB5 was obtained also in the use of the embryonic cell of the isolated pre-streak epiblast, and appearance of a nervous system cell and an epidermal system cell was observed.

### Reference Example 13

### Recovery of factor, in stromal cell, capable of inducing differentiation of embryonic stem cell into ectodermal cell:

According to the method described in Reference Example 3, the ES cell EB5 was cultured for 8 days in the serum-free medium without BMP4 by using the paraformaldehyde-fixed PA6 cell as a feeder cell. Also, the case when the PA6 cell was cultured in a medium containing heparin (GIBCO BRL) (hereinafter referred to as "heparin treatment") was compared with the case when culturing in a heparin-free medium. That is, dishes in which the PA6 cell proliferated to almost confluent on a 3 cm tissue culture dish was cultured for 2 days in a medium containing 200 ng/ml of heparin and dishes in which it was cultured for 2 days in a heparin-free medium were prepared, washed twice with PBS(-) and subjected to paraformaldehyde fixation, and the ES cell EB5 was inoculated onto the paraformaldehyde-fixed PA6 cell at a density of 200 cells/dish, the medium was exchanged with a fresh medium on the 4th, 6th and 7th days, and then the cells were cultured at 37°C for 8 days in a stream of 5% carbon dioxide in a CO₂ incubator.

Eight days after coculturing, the cells were fixed according to the method described in Reference Example 1, and the colonies formed as a result of the coculturing of the ES cell EB5 with PA6 cell were immunologically stained with the anti-NCAM antibody, anti-tubulin antibody and anti-nestin antibody.

When the PA6 cell having no heparin treatment was used as a feeder cell, close to 90% of the ES cell-derived colonies were NCAM-positive similar to the results shown in Reference Example 3, so that differentiation of ES cell into nerve cell was observed at a high frequency. On the other hand, significant differentiation of ES cell into nerve cell was not observed when the heparin-treated PA6 cell was used as a feeder cell. Accordingly, it was suggested that the SDIA activity of a stromal cell can be recovered from a culture supernatant by culturing the stromal cell in a heparin-containing medium, similar to the phenomenon observed by Wnts molecule (R.S. Bradley & A.M.C. Brown, *EMBO J*., 9, 1569 (1990)).

### Reference Example 14

### Differentiation induction of embryonic stem cell into various neural cells along the dorso-ventral axis:

In order to examine effects of shh and BMP4 as factors which determine diversity of nerves along the dorso-ventral axis in the generation of central nervous system, these factors were allowed to act upon an ES cell which started its differentiation on a stromal cell and their influences were examined in the following manner.

According to the method described in Reference Example 1, the ES cell EB5 was cultured for 10 days in the serum-free medium without BMP4 by using the PA6 cell as a feeder cell. That is, the PA6 cell proliferated to almost confluent on a 3 cm tissue culture dish was used as a feeder cell, the ES cell was inoculated onto the feeder cell at a density of 200 cells/dish, the medium was exchanged with a fresh serum-free medium on the 4th, 6th and 8th days, and the cells were cultured at 37°C for 10 days in a 5% CO₂ incubator.

Effects of shh were evaluated according to the similar method described above, by using a serum-free medium to which 300 nmol/l of shh (manufactured by R & D) had been added at the time of the medium exchange on the 4th, 6th and 8th days.

Effects of BMP4 were evaluated according to the similar method described above, by using a serum-free medium to which 0.5 nmol/l of BMP4 (manufactured by R & D) had been added at the time of the medium exchange on the 4th, 6th and 8th days.

Ten days after coculturing, the cells cultured by respective culturing methods were fixed according to the method described in Reference Example 1, and the colonies formed as a result of coculturing of the ES cell with PA6 cell were immunologically stained with the anti-NCAM antibody, an antibody against HNF-3β which is a marker of the basal plate existing on the most ventral side of the central nervous system primordium (neural tube) (purchased from Developmental Studies Hybridoma Bank), an antibody against Nkx2.2 as a marker existing secondary to the HNF-3β from the ventral side (purchased from Developmental Studies Hybridoma Bank), an antibody against Pax-7 as a marker of the neural tube dorsal side (purchased from Developmental Studies Hybridoma Bank), an antibody against AP-2 as a marker of the neural crest cell (purchased from Developmental Studies Hybridoma Bank), an antibody against islet 1 as a marker of motor neuron (purchased from Developmental Studies Hybridoma Bank) and an antibody against VAchT which is a marker of cholinergic neuron (manufactured by Chemicon).

Regardless of the addition of shh or BMP4, most of the colonies formed as a result of coculturing of the ES cell EB5 with PA6 cell were stained with the anti-NCAM antibody similar to the results shown in Reference Example 1, and 90% of the ES cell-derived colonies were positive in both cases.

The result is shown in Table 1, together with the ratio of ES cell-derived colonies stained with antibodies against other markers.

**Table 1**

| Antibodies | Control | shh added | BMP4 added |
|---|---|---|---|
| Anti-NCAM antibody | 90% | 90% | 90% |
| Anti-HNF-3β antibody | 70% | 81% | 9% |
| Anti-Nkx2.2 antibody | 44% | 85% | 19% |
| Anti-Pax-7 antibody | 30% | 0% | 72% |
| Anti-AP-2 antibody | 16% | 0% | 24% |
| Anti-islet 1 antibody | 82% | 82% | 36% |
| Anti-VAchT antibody | 36% | 58% | 42% |

It was shown from the above results that nervous system cells expressing not only the NCAM as a neuron marker but also various types of neuron-specific markers are formed by the nerve cell induction of the ES cell by its coculturing with the PA6 cell. That is, when the ES cell was differentiation-induced by coculturing with the PA6 cell, it is differentiation-induced into a nervous system cell which is positioned on the basal plate of the most ventral side of the central nervous system primordium (neural tube) and expresses HNF-3β, a nervous system cell which is positioned secondary to the HNF-3β from the ventral side of the central nervous system primordium (neural tube) and expresses Nkx2.2, a nerve cell of the neural tube dorsal side expressing Pax-7, a neural crest cell expressing AP-2 and a motor neuron expressing islet 1.

Also, since shh and BMP4, whose relation to the determination of dorso-ventral axis during the embryo neurogenesis has been revealed, showed a differentiation potency similar to the *in vivo* differentiation potency of embryonic neural precursor cell, a cell of neural tube before the step of determining dorso-ventral axis is induced by coculturing ES cell with the PA6 cell. That is, in this neural tube cell, expression induction of the ventral side markers HNF-3β and Nkx2.2 and expression inhibition of the dorsal side markers Pax-7 and AP-2 are observed by the action of shh as a neural tube dorso-ventral factor. On the other hand, when the BMP4 as a neural tube dorsal side factor is allowed to act, expression inhibition of the ventral side markers HNF-3β and Nkx2.2 and expression induction of the dorsal side markers Pax-7 and AP-2 are observed.

Also, a result similar to the above was obtained when a typical ES cell, 129 line mouse-derived CCE cell (M.R. Kuehn *et al*., *Nature*, 326, 295 (1987); *Production of Mutation Mice Using ES Cell*), was used.

### Reference Example 15

### Production of monoclonal antibody capable of recognizing stromal cell PA6: (1) Preparation of immunogen

The PA6 cell was used as the antigen. The PA6 cell was cultured according to the method described in Reference Example 1. The PA6 cell whose cell density reached almost confluent was washed twice with PBS(-), a PBS(-) solution containing 10 µg/ml of actinase (manufactured by Kaken Pharmaceutical) and 0.02% of EDTA was added thereto, followed by culturing at 37°C for 30 minutes, the action of actinase was stopped by adding α-MEM medium containing 10% fetal bovine serum (GIBCO-BRL), and then the cells were recovered by 5 minutes of centrifugation at 4°C and at 1,000×g. The thus recovered cells were re-suspended in PBS(-) and washed by centrifugation at 4°C and at 1,000×g for 5 minutes. A total of 10⁷ of the cells washed twice with PBS(-) were suspended in 1 ml of PBS(-) and used as the antigen.

### (2) Immunization of animal and production of antibody producing cell

The 10⁷ cells produced in the above (1) were administered to each of 3 female SD rats of 6 to 8 weeks of age, together with 2 mg of an aluminum hydroxide adjuvant (*Antibody, A Laboratory Manual,* p. 99) and 1 × 10⁹ cells of pertussis vaccine (manufactured by Chiba Serum Institute). Two weeks after the administration, the 10⁷ cells produced in the above (1) were administered once a week for a total of 4 times. Blood samples were collected from the carotid artery of the rats, their serum antibody titers were examined by an enzyme immunoassay shown in the following, and the spleen was excised 3 days after the final immunization from a mouse which showed a sufficient antibody titer.

The thus excised spleen was cut to pieces in MEM (minimum essential medium) medium (manufactured by Nissui Pharmaceutical), the cells were unbound by using a pair of forceps and centrifuged (250×g, 5 minutes). The thus obtained precipitation fraction was treated with Tris-ammonium chloride buffer (pH 7.65) for 1 to 2 minutes to eliminate erythrocytes. The thus obtained precipitation fraction (cell fraction) was washed three times with MEM medium and used in cell fusion.

### (3) Enzyme immunoassay (binding ELISA)

The PA6 cell was inoculated into each well of a 96 well EIA plates (manufactured by Greiner), and a plate in which the cells were proliferated into confluent was used as an antigen plate. An immunized rat antiserum or a monoclonal antibody culture supernatant was dispensed in 50 µl/well into the plate and incubated at 37°C for 1 hour. One hour after standing, the added antiserum or culture supernatant was discarded, and PBS(-) containing 0.25% glutaraldehyde was added to the remaining cells and incubated at room temperature for 30 minutes. The plate was washed with 0.05% polyoxyethylene (20) sorbitan monolaurate (equivalent to Tween 20, trademark of ICI: manufactured by Wako Pure Chemical Industries)/PBS (hereinafter referred to as "Tween-PBS"), and a peroxidase-labeled rabbit anti-rat immunoglobulin (manufactured by DAKO) was dispensed in 50 µl/well into the plate and incubated at room temperature for 1 hour. After washing the plate with Tween-PBS, an ABTS substrate solution (2,2-azinobis(3-ethylbenzothiazole-6-sulfonic acid) ammonium salt, 1 mmol/l ABTS/0.1 mol/l citrate buffer (pH 4.2)) was added thereto, and the absorbance at 415 nm was measured by using a plate reader (Emax; manufactured by Molecular Devices).

### (4) Preparation of mouse myeloma cells

An 8-azaguanine-resistant mouse myeloma cell line P3X63Ag8U.1 (P3-U1: purchased from ATCC) was cultured in a normal medium (RPMI medium supplemented with 10% fetal calf serum), and 2×10⁷ or more of the cells are secured for cell fusion and used as the parent cell line in the cell fusion.

### (5) Preparation of hybridoma

The mouse spleen cells obtained in Reference Example 15(2) and the myeloma cells obtained in Reference Example 15(4) were mixed in a proportion of 10:1, followed by centrifugation (250×g, 5 minutes). The cells of the thus obtained precipitation fraction were thoroughly disintegrated, a mixed solution of 2 g of polyethylene glycol-1,000 (PEG-1,000), 2 ml of MEM medium and 0.7 ml of dimethyl sulfoxide was added to the cells with stirring at 37°C, in an amount of 0.5 ml per 10⁸ mouse spleen cells, 1 ml of MEM medium was added several times at 1 to 2-minute intervals and then the total volume was adjusted to 50 ml by adding MEM medium.

After centrifugation of the suspension (900 rpm, 5 minutes), the cells of the thus obtained precipitation fraction were loosened gently and then suspended in 100 ml of HAT medium (produced by adding HAT Media Supplement (manufactured by Boehringer Mannheim) to the RPMI medium supplemented with 10% fetal calf serum) by repeated drawing up into and discharging from a measuring pipette. This suspension was dispensed in 200 µl/well into a 96-well culture plates, followed by culturing at 37°C for 7 to 14 days in a 5% CO₂ incubator.

After culturing, the culture supernatant was examined by the enzyme immunoassay described in Reference Example 15(3) to select wells which reacted with the PA6 cell but did not react with a control plate coated with 1% BSA-containing PBS(-) (hereinafter referred to as "1% BSA-PBS(-)"), and cloning was repeated twice by limiting dilution to establish anti-PA6 monoclonal antibody producing hybridomas from cells contained therein. As a result, three types of anti-human PA6 cell antibodies KM1306, KM1307 and KM1310 were obtained by using the PA6 cell as the antigen.

The KM1310 producing hybridoma cell line has been deposited on April 27, 2001, as FERM BP-7573 in International Patent Organism Depositary, National Instituted of Advanced Industrial Science and Technology (Tsukuba Central 6, 1-1, Higashi 1-Chome, Tsukuba-shi, Ibaraki-ken, 305-8566, Japan).

### (6) Preparation of monoclonal antibody

Each of the hybridoma cell lines obtained in Reference Example 15(5) was intraperitoneally injected into pristane-treated 8 weeks old female nude mice (BALB/c) at a dose of 5×10⁶ to 20×10⁶ cells per animal. Ten to twenty-one days thereafter, the ascitic fluid was collected from the ascites tumor mice caused by the hybridoma (1 to 8 ml/animal).

The ascitic fluid was centrifuged (1,200×g, 5 minutes) to remove solid matter. Purified IgM monoclonal antibodies were obtained by purifying them according to an ammonium sulfate precipitation method (*Antibody, A Laboratory Manual*). The subclass of all of the monoclonal antibodies KM1306, KM1307 and KM1310 was determined to be IgM by ELISA using a subclass typing kit.

### (7) Analysis of the reactivity with PA6 cell by fluorescent antibody technique (cell sorter analysis)

The PA6 cell was cultured according to the method described in Reference Example 1. The PA6 cell whose cell density reached almost confluent was washed twice with PBS(-), a PBS(-) solution containing 10 µg/ml of actinase (manufactured by Kaken Pharmaceutical) and 0.02% of EDTA was added thereto, followed by culturing at 37°C for 30 minutes, the action of actinase was stopped by adding α-MEM medium containing 10% fetal bovine serum (manufactured by GIBCO-BRL), and then the cells were recovered by centrifugation at 4°C and at 1,000×g for 5 minutes. The thus recovered cells were re-suspended in α-MEM medium containing 10% fetal bovine serum (manufactured by GIBCO-BRL) and dispensed in 1×10⁶ cell portions into 1.5 ml tubes. The dispensed cells were washed twice by suspending them in 1% BSA-PBS(-) and centrifuging at 1,000×g for 5 minutes. The washed cells were suspended in 1% BSA-PBS(-) solution containing 10 µg/ml of a purified antibody (or 50 µg/ml of an ammonium sulfate precipitation antibody fraction) and cultured at 37°C for 30 minutes to carry out the reaction with the antibody. The cells reacted with the antibody were allowed to react with a fluorescence-labeled secondary antibody in the usual way and analyzed by using a cell sorter (*Antibody, A Laboratory Manual*). That is, the cells reacted with the antibody were recovered by centrifugation at 1,000×g for 5 minutes, suspended in 1% BSA-PBS(-) solution containing the secondary antibody, cultured at 37°C for 30 minutes, washed twice with 1% BSA-PBS(-), suspended in 2 ml of 1% BSA-PBS(-) solution and analyzed by using a cell analyzer (EPICS XLsystem II, manufactured by Coulter). As the secondary antibody, an FITC-labeled anti-rat immunoglobulin antibody (FITC-labeled goat anti-rat immunoglobulin (H+L); manufactured by CALTAG) was diluted 30 times with 1% BSA-PBS(-), and the solution was used in 100 µl/tube portions. As a control antibody, KM2070 as a monoclonal rat IgM antibody which recognizes a *Klotho* protein was allowed to react at a concentration of 10 µg/ml and detected in the same manner. Also, KM2070 is an antibody produced by a hybridoma KM2070 (FERM BP-6196; WO 98/29544). Also, KM2070 was used as the control antibody after confirming in advance that expression of the antigen molecule recognized by KM2070 is not due to the PA6 cell.

As respectively shown in Figs. 9, 10 and 11, KM1306, KM1307 and KM1310 obtained by immunizing the PA6 cell recognized the PA6 cell. The ordinate shows the number of cells, and the abscissa shows fluorescence intensity. In these drawings, "nega" indicates a result when the antibody was not added.

### INDUSTRIAL APPLICABILITY

The present invention provides an agent for inducing differentiation of an embryonic cell into an ectodermal cell or ectoderm-derived cell selectively and efficiently, a method for inducing differentiation by using the agent for inducing differentiation, a differentiation-induced cell, and use of them.

### Free Text of Sequence Listing

SEQ ID NO:1-Description of Artificial Sequence: Synthetic DNA
SEQ ID NO:2-Description of Artificial Sequence: Synthetic DNA
SEQ ID NO:3-Description of Artificial Sequence: Synthetic DNA
SEQ ID NO:4-Description of Artificial Sequence: Synthetic DNA
SEQ ID NO:5-Description of Artificial Sequence: Synthetic DNA
SEQ ID NO:6-Description of Artificial Sequence: Synthetic DNA

## Claims

1. A method for obtaining a solution having activity to induce differentiation of an embryonic stem cell into an ectodermal cell or ectoderm-derived cell, which comprises culturing a stromal cell in a culture comprising a polyanionic compound, and recovering the culture.

2. The method according to claim 1, wherein the polyanionic compound is a copolymer or homopolymer which has a negative charge in the culture.

3. The method according to claim 2, wherein the copolymer which has a negative charge in the culture is a mucopolysaccharide.

4. The method according to claim 3, wherein the mucopolysaccharide is a compound selected from the group consisting of the following (a), (b), (c), (d), (e), (f), (g), (h), (i) and (j):
(a) chondroitin 4-sulfate;
(b) chondroitin 5-sulfate;
(c) chondroitin 6-sulfate;
(d) dermatan sulfate;
(e) heparan sulfate;
(f) heparin;
(g) keratan sulfate I;
(h) keratan sulfate II;
(i) hyaluronic acid; and
(j) chondroitin.

5. The method according to claim 2, wherein the homopolymer which has a negative charge in the culture is a compound selected from the group consisting of the following (a), (b), (c), (d), (e), (f), (g), (h), (i), (j) and (k):
(a) dextran sulfate;
(b) carboxymethyldextran;
(c) sulfated polyvinyl;
(d) polyvinyl sulfite;
(e) sulfonated polystyrene;
(f) polyacrylic acid;
(g) carboxymethylcellulose;
(h) cellulose sulfate;
(i) polyglutamic acid;
(j) polymaleic acid; and
(k) polymethacrylic acid.

6. The method according to any one of claims 1 to 5, wherein the culture is a basal medium or a balanced salt solution used for cell culturing.

7. The method according to any one of claims 1 to 6, wherein the stromal cell is recognized by a monoclonal antibody produced by a hybridoma FERM BP-7573.

8. The method according to any one of claims 1 to 6, wherein the stromal cell is selected from the group consisting of the following (a), (b), (c), (d), (e), (f) and (g):
(a) a fetal primary culture fibroblast;
(b) an SIHM mouse-derived STO cell;
(c) a mouse fetus-derived NIH/3T3 cell;
(d) a macrophage colony-stimulating factor (M-CSF) deficient mouse calvaria-derived OP9 cell;
(e) a mouse calvaria-derived MC3T3-G2/PA6 cell;
(f) an embryonic stem cell-derived stromal cell; and
(g) a bone marrow mesenchymal stem cell-derived stromal cell.

9. A solution having activity to induce differentiation of an embryonic stem cell into an ectodermal cell or ectoderm-derived cell, which is obtainable by the method according to any one of claims 1 to 8.

10. An agent for inducing differentiation of an embryonic stem cell into an ectodermal cell or ectoderm-derived cell, which comprises, as an active ingredient, the solution according to claim 9.

11. A factor which induces differentiation of an embryonic stem cell into an ectodermal cell or ectoderm-derived cell contained in the solution according to claim 9.

12. An agent for inducing differentiation of an embryonic stem cell into an ectodermal cell or ectoderm-derived cell, which comprises, as an active ingredient, a polypeptide consisting of the amino acid sequence represented by SEQ ID NO:7.

13. An agent for inducing differentiation of an embryonic stem cell into an ectodermal cell or ectoderm-derived cell, which comprises, as an active ingredient, a polypeptide consisting of an amino acid sequence in which one or more amino acid residue(s) is/are deleted, substituted, inserted and/or added in the amino acid sequence represented by SEQ ID NO:7.

14. An agent for inducing differentiation of an embryonic stem cell into an ectodermal cell or ectoderm-derived cell, which comprises, as an active ingredient, a polypeptide consisting of an amino acid sequence having a homology of 60% or more with the amino acid sequence represented by SEQ ID NO:7.

15. An agent for inducing differentiation of an embryonic stem cell into an ectodermal cell or ectoderm-derived cell, which comprises, as an active ingredient, a recombinant vector comprising a DNA encoding the amino acid sequence represented by SEQ ID NO:7.

16. An agent for inducing differentiation of an embryonic stem cell into an ectodermal cell or ectoderm-derived cell, which comprises, as an active ingredient, a recombinant vector comprising a DNA having the nucleotide sequence represented by SEQ ID NO:9.

17. An agent for inducing differentiation of an embryonic stem cell into an ectodermal cell or ectoderm-derived cell, which comprises, as an active ingredient, a transformant obtainable by introducing a recombinant vector comprising a DNA encoding the amino acid sequence represented by SEQ ID NO:7 into a stromal cell.

18. An agent for inducing differentiation of an embryonic stem cell into an ectodermal cell or ectoderm-derived cell, which comprises, as an active ingredient, a transformant obtained by introducing a recombinant vector comprising a DNA having the nucleotide sequence represented by SEQ ID NO:9 into a stromal cell.

19. An agent for inducing differentiation of an embryonic stem cell into an ectodermal cell or ectoderm-derived cell, which comprises, as an active ingredient, a polypeptide consisting of the amino acid sequence represented by SEQ ID NO:8.

20. An agent for inducing differentiation of an embryonic stem cell into an ectodermal cell or ectoderm-derived cell, which comprises, as an active ingredient, a polypeptide consisting of an amino acid sequence in which one or more amino acid residue(s) is/are deleted, substituted, inserted and/or added in the amino acid sequence represented by SEQ ID NO:8.

21. An agent for inducing differentiation of an embryonic stem cell into an ectodermal cell or ectoderm-derived cell, which comprises, as an active ingredient, a polypeptide consisting of an amino acid sequence having a homology of 60% or more with the amino acid sequence represented by SEQ ID NO:8.

22. An agent for inducing differentiation of an embryonic stem cell into an ectodermal cell or ectoderm-derived cell, which comprises, as an active ingredient, a recombinant vector comprising a DNA encoding the amino acid sequence represented by SEQ ID NO:8.

23. An agent for inducing differentiation of an embryonic stem cell into an ectodermal cell or ectoderm-derived cell, which comprises, as an active ingredient, a recombinant vector comprising a DNA having the nucleotide sequence represented by SEQ ID NO:10.

24. An agent for inducing differentiation of an embryonic stem cell into an ectodermal cell or ectoderm-derived cell, which comprises, as an active ingredient, a transformant obtained by introducing a recombinant vector comprising a DNA encoding the amino acid sequence represented by SEQ ID NO:7 into a stromal cell.

25. An agent for inducing differentiation of an embryonic stem cell into an ectodermal cell or ectoderm-derived cell, which comprises, as an active ingredient, a transformant obtained by introducing a recombinant vector comprising a DNA having the nucleotide sequence represented by SEQ ID NO:10 into a stromal cell.

26. An agent for inducing differentiation of an embryonic stem cell into an ectodermal cell or ectoderm-derived cell, which comprises, as an active ingredient, an Wnt antagonist.

27. A method for inducing differentiation of an embryonic stem cell into an ectodermal cell or ectoderm-derived cell, which comprises a step of culturing the embryonic stem cell under non-aggregation conditions by using the solution according to claim 9 or the agent for inducing differentiation according to any one of claims 12 to 26.

28. The method according to claim 27, wherein a culture vessel immobilized with the solution according to claim 9 or the agent for inducing differentiation according to any one of claims 12 to 26 is used.

29. The method according to any one of claims 1 to 8, 27 and 28, wherein the ectodermal cell is a cell capable of differentiating into a nervous system cell or an epidermal system cell.

30. The method according to any one of claims 1 to 8, 27 and 28, wherein the ectoderm-derived cell is a nervous system cell or an epidermal system cell.

31. The method according to claim 29 or 30, wherein the epidermal system cell is an epidermal cell.

32. The method according to claim 29 or 30, wherein the nervous system cell is a cell selected from the group consisting of the following (a), (b), (c), (d) and (e):
(a) a neural stem cell;
(b) a nerve cell;
(c) a cell of neural tube;
(d) a cell of neural crest; and
(e) a retinal pigment cell.

33. The method according to claim 32, wherein the neural stem cell is a neural stem cell expressing nestin.

34. The method according to claim 32, wherein the nerve cell is a nerve cell selected from the group consisting of the following (a), (b), (c) and (d):
(a) a dopaminergic neuron;
(b) an acetylcholinergic neuron;
(c) a γ-aminobutyratergic neuron; and
(d) a serotonergic neuron.

35. The method according to claim 34, wherein the acetylcholinergic neuron is a motor nerve cell expressing islet 1.

36. The method according to claim 32, wherein the cell of neural tube is a cell selected from the group consisting of the following (a), (b), (c) and (d):
(a) a cell of neural tube before determination of dorso-ventral axis, which is capable of differentiating into a cell positioned at the ventral side by reacting with sonic hedgehog as a ventral factor of neural tube and of differentiating into a cell positioned at the dorsal side by reacting with bone morphogenetic protein 4 as a dorsal factor of neural tube;
(b) a cell of the neural tube ventral side, expressing HNF-3β (hepatocyte nuclear factor-3β) positioned on the basal plate of the most ventral side of neural tube;
(c) a cell of the neural tube ventral side, expressing a marker Nkx2.2 existing secondary to the HNF-3β (hepatocyte nuclear factor-3β) from the ventral side of neural tube; and
(d) a cell of the neural tube dorsal side, expressing Pax-7.

37. The method according to claim 32, wherein the cell of neural crest is a cell expressing AP-2 (activator protein 2).

38. The method according to any one of claims 27 to 37, wherein said culturing is carried out in the presence of bone morphogenetic protein 4.

39. The method according to any one of claims 27 to 38, wherein said culturing is carried out in the presence of sonic hedgehog.

40. The method according to any one of claims 27 to 39, wherein the non-aggregation conditions are conditions not mediating an embryoid body.

41. The method according to any one of claims 27 to 40, which comprises a step of culturing under serum-free culture conditions.

42. The method according to any one of claims 27 to 41, wherein said culturing is carried out in the absence of retinoic acid.

43. The method according to any one of claims 27 to 42, wherein the embryonic stem cell is selected from the group consisting of the following (a), (b) and (c):
(a) an embryonic stem cell established by culturing an early embryo before implantation;
(b) an embryonic stem cell established by culturing an early embryo produced by nuclear transplantation of the nucleus of a somatic cell; and
(c) an embryonic stem cell in which a gene on the chromosome of the embryonic stem cell of (a) or (b) is modified by using a genetic engineering technique.

44. The method according to any one of claims 1 to 8 and 27 to 43, wherein the embryonic stem cell is differentiated into an ectodermal cell or an ectoderm-derived cell at an efficiency of 5% or more.

45. The method according to any one of claims 27 to 44, which does not substantially accompany differentiation induction of a mesodermal system cell.

46. An ectodermal cell or an ectoderm-derived cell, which is induced by using the method of any one of claims 27 to 45.

47. A method for increasing purity of a cell which is differentiation-induced from an embryonic stem cell, which comprises a step of culturing the cell according to claim 46 in a medium comprising an antitumor agent.

48. The method according to claim 47, wherein the antitumor agent is selected from the group consisting of mitomycin C, 5-fluorouracil, adriamycin, methotrexate and ara-C.

49. A cell which is obtainable by using the method according to claim 46 or 47.

50. A medicament comprising the cell according to claim 46 or 49.

51. A medicament which comprises, as an active ingredient, at least one selected from the group consisting of the following (a) to (o):
(a) a polypeptide consisting of the amino acid sequence represented by SEQ ID NO:7;
(b) a polypeptide consisting of an amino acid sequence in which one or more amino acid residue(s) is/are deleted, substituted, inserted and/or added in the amino acid sequence represented by SEQ ID NO:7;
(c) a polypeptide consisting of an amino acid sequence having a homology of 60% or more with the amino acid sequence represented by SEQ ID NO:7;
(d) a recombinant vector comprising a DNA encoding the amino acid sequence represented by SEQ ID NO:7;
(e) a recombinant vector comprising a DNA having the nucleotide sequence represented by SEQ ID NO:9;
(f) a transformant obtainable by introducing a recombinant vector comprising a DNA encoding the amino acid sequence represented by SEQ ID NO:7 into a stromal cell;
(g) a transformant obtained by introducing a recombinant vector comprising a DNA having the nucleotide sequence represented by SEQ ID NO:9 into a stromal cell;
(h) a polypeptide consisting of the amino acid sequence represented by SEQ ID NO:8;
(i) a polypeptide consisting of an amino acid sequence in which one or more amino acid residue(s) is/are deleted, substituted, inserted and/or added in the amino acid sequence represented by SEQ ID NO:8;
(j) a polypeptide consisting of an amino acid sequence having a homology of 60% or more with the amino acid sequence represented by SEQ ID NO:8;
(k) a recombinant vector comprising a DNA encoding the amino acid sequence represented by SEQ ID NO:8;
(l) a recombinant vector comprising a DNA having the nucleotide sequence represented by SEQ ID NO:10;
(m) a transformant obtained by introducing a recombinant vector comprising a DNA encoding the amino acid sequence represented by SEQ ID NO:7 into a stromal cell;
(n) a transformant obtained by introducing a recombinant vector comprising a DNA having the nucleotide sequence represented by SEQ ID NO:10 into a stromal cell; and
(o) an agent for inducing differentiation of an embryonic stem cell into an ectodermal cell or ectoderm-derived cell, which comprises, as an active ingredient, an Wnt antagonist.

52. The medicament according to claim 50 or 51, which is a medicament for diagnosing, preventing and/or treating diseases caused by the disorder of an ectoderm-derived cell.

53. The medicament according to claim 52, wherein the diseases caused by the disorder of an ectoderm-derived cell are diseases caused by the disorder of a nervous system cell or an epidermal system cell.

54. The medicament according to claim 53,
wherein the diseases caused by the disorder of a nervous system cell are Alzheimer disease, Huntington chorea, Parkinson disease, ischemic cerebral disease, epilepsy, brain injury, vertebral injury, motor neuron disease, neurodegeneration disease, pigmentary retinal dystrophy, cochlear hearing loss, multiple sclerosis, amyotrophic lateral sclerosis or diseases due to a neurotoxin damage; and
the diseases caused by the disorder of an epidermal system cell are burn, wound, healing of wound, compression gangrene or psoriasis.

55. A method for evaluating a substance relating to the regulation in a differentiation step from an embryonic stem cell into an ectodermal cell or an ectoderm-derived cell, which comprises:
carrying out the method according to any one of claims 27 to 45 in the presence of a substance to be tested and the method in the absence of the substance to be tested; and
comparing the differentiation step from an embryonic stem cell into an ectodermal cell or an ectoderm-derived cell in the presence of the substance to be tested with that in the absence of the substance to be tested.

56. A method for screening a substance relating to the regulation in a differentiation step from an embryonic stem cell into an ectodermal cell or an ectoderm-derived cell, which comprises:
carrying out the method according to any one of claims 27 to 45 in the presence of a substance to be tested and the method in the absence of the substance to be tested; and
comparing the differentiation step from an embryonic stem cell into an ectodermal cell or an ectoderm-derived cell in the presence of a substance to be tested with that in the absence of the substance to be tested.

57. A method for evaluating a substance relating to the regulation of the function of an ectodermal cell or an ectoderm-derived cell, which comprises:
culturing the cell according to claim 46 in the presence of a substance to be tested and the cell in the absence of the substance to be tested; and
comparing the function of an ectodermal cell or an ectoderm-derived cell in the presence of the substance to be tested with that in the absence of the substance to be tested.

58. A method for screening a substance relating to the regulation of the function of an ectodermal cell or an ectoderm-derived cell, which comprises:
culturing the cell according to claim 46 in the presence of a substance to be tested and that in the absence of the substance to be tested; and
comparing the function of the ectodermal cell or the ectoderm-derived cell in the presence of the substance to be tested with that in the absence of the substance to be tested.
